# EUROPEAN PATENT APPLICATION

(11) **EP 1 862 466 A2**
(43) Date of publication of application: **05.12.2007**
(21) Application number: 07008749.9
(22) Date of filing: 24.06.2005
(51) Int. Cl.: C07H 3/06, G01N 33/574, A61K 31/702

(54) **Arrays with cleavable linkers**

(30) Priority: 24.06.2004 US 582713 P
(62) Divisional of application: 05789058.4
(71) Applicant: THE SCRIPPS RESEARCH INSTITUTE, La Jolla, CA 92037 (US)
(72) Inventor: Wong, Chi-Huey, Rancho Santa Fe, CA 92067 (US)
(74) Representative: Harding, Charles Thomas

(57) **Abstract**

The invention provides arrays of molecules where the molecules (e.g., glycans) are attached to the arrays by cleavable linkers. The invention also provides methods for using these arrays, methods for identifying the structural elements of molecules bound to these arrays by using the cleavable linkers, especially the structural elements that are important for binding to test samples. The invention further provides methods for evaluating whether test samples and test molecules can bind to distinct glycans on the arrays and useful glycans identified using the methods and arrays provided herein.

## Description

This application claims benefit of the filing date of U.S. Provisional Ser. No. 60/582713, filed June 24, 2004, the contents of which are incorporated herein by reference.

### Government Funding

The invention described herein was made with United States Government support under Grant Numbers GM58439 and GM44154 awarded by the National Institutes of Health. The United States Government has certain rights in this invention.

### Field of the Invention

The invention relates to cleavable linkers and methods for generating arrays with cleavable linkers. The invention also relates to methods for identifying agents that bind to various types of molecules on the arrays and to defining the structural elements of the molecules on the arrays that bind to those agents. The arrays and methods provided herein can be used for epitope identification, drug discovery and as analytical tools. For example, the invention provides useful glycans that van be used in compositions for treating and preventing cancer and/or viral infection.

### Background of the Invention

Glycans are typically the first and potentially the most important interface between cells and their environment. As vital constituents of all living systems, glycans are involved in recognition, adherence, motility and signaling processes. There are at least three reasons why glycans should be studied: (1) all cells in living organisms, and viruses, are coated with diverse types of glycans; (2) glycosylation is a form of post- or co-translational modification occurring in all living organisms; and (3) altered glycosylation is an indication of an early and possibly critical point in development of human pathologies. Jun Hirabayashi, Oligosaccharide microarrays for glycomics; 2003, Trends in Biotechnology.21(4): 141-143; Sen-Itiroh Hakomori, Tumor-associated carbohydrate antigens defining tumor malignancy: Basis for development of and-cancer vaccines; in The Molecular Immunology of Complex Carbohydrates-2 (Albert M Wu, ed., Kluwer Academic/Plenum, 2001). These cell-identifying glycosylated molecules include glycoproteins and glycolipids and are specifically recognized by various glycan-recognition proteins, called 'lectins.' However, the enormous complexity of these interactions, and the lack of well-defined glycan libraries and analytical methods have been major obstacles in the development of glycomics.

The development of nucleotide and protein microarrays has revolutionized genomic, gene expression and proteomic research. While the pace of innovation of these arrays has been explosive, the development of glycan microarrays has been relatively slow. One reason for this is that it has been difficult to reliably immobilize populations of chemically and structurally diverse glycans. Moreover, glycans are not readily amenable to analysis by many of the currently available molecular techniques (such as rapid sequencing and in vitro synthesis) that are routinely applied to nucleic acids and proteins.

Therefore, new tools are needed for understanding the structure and functional significance of interactions between glycans and other types of molecules. Moreover, pharmaceutical companies and research institutions would greatly benefit from glycan arrays for various screening and drug discovery applications, including arrays that facilitate analysis of the structural elements of glycans that contribute to binding to antibodies, receptors and other biomolecules.

### Summary of the Invention

The invention provides cleavable linkers that can be used in a variety of applications. For example, the cleavable linkers of the invention can be used to attach molecules to solid surfaces or arrays. The cleavable linker can have cleavable unit that is a photocleavable, enzyme-cleavable or chemically-cleavable unit. For example, the cleavable linker can have a cleavable unit such as a disulfide (chemically cleavable), nitrobenzo (a photocleavable unit), or amine, amide or ester (enzyme-sensitive cleavable units).

The invention also provides glycan arrays (or microarrays) with cleavable linkers. In addition, the invention provides methods for making such glycan arrays or microarrays. In other embodiments, the invention provides methods for using such arrays to identify and analyze the interactions that various types of glycans have with other molecules. These glycan arrays and screening methods are useful for identifying which protein, receptor, antibody, nucleic acid or other molecule or substance will bind to which glycan. Thus, the glycan libraries and glycan arrays of the invention can be used for receptor ligand characterization, identification of carbohydrates on cell membranes and within subcellular components, antibody epitope identification, enzyme characterization and phage display library screening. In one embodiment, the invention provides an array of glycans where the glycans attached to the array by a cleavable linker.

The glycans used on the arrays of the invention include 2 or more sugar units. The glycans of the invention include straight chain and branched oligosaccharides as well as naturally occurring and synthetic glycans. Any type of sugar unit can be present in the glycans of the invention, including allose, altrose, arabinose, glucose, galactose, gulose, fucose, fructose, idose, lyxose, mannose, ribose, talose, xylose, neuraminic acid or other sugar units. Such sugar units can have a variety of substituents. For example, substituents that can be present instead of, or in addition to, the substituents typically present on the sugar units include amino, carboxy, thiol, azide, N-acetyl, N-acetylneuraminic acid, oxy (=O), sialic acid, sulfate (-SO₄⁻), phosphate (-PO₄⁻), lower alkoxy, lower alkanoyloxy, lower acyl, and/or lower alkanoylaminoalkyl. Fatty acids, lipids, amino acids, peptides and proteins can also be attached to the glycans of the invention.

In another embodiment, the invention provides a microarray that includes a solid support and a multitude of defined glycan probe locations on the solid support, each glycan probe location defining a region of the solid support that has multiple copies of one type of glycan molecule attached thereto and wherein the glycans are attached to the microarray by a cleavable linker. These microarrays can have, for example, between about 2 to about 100,000 different glycan probe locations, or between about 2 to about 10,000 different glycan probe locations.

In another embodiment, the invention provides, a method of identifying whether a test molecule or test substance can bind to a glycan present on an array or microarray of the invention. The method involves contacting the array with the test molecule or test substance and observing whether the test molecule or test substance binds to a glycan in the library or on the array.

In another embodiment, the invention provides a method of identifying to which glycan a test molecule or test substance can bind, wherein the glycan is present on an array of the invention. The method involves contacting the array with the test molecule or test substance and observing to which glycan the array the test molecule or test substance can bind.

In another embodiment, the invention provides a library of glycans that includes a series of separate, glycan preparations wherein substantially all glycans in each glycan preparation of the library has an azido linking group that may be used for attachment of the glycan onto a solid support for formation of an array of the invention.

In another embodiment, the invention provides a method making the arrays of the invention that involves derivatizing the solid support surface of the array with trialkoxysilane bearing reactive moieties such as N-hydroxysuccinimide (NHS), amino (--NH₂), thiol (-SH), carboxyl (COOH), isothiocyanate (--NCS), or hydroxyl (--OH) to generate at least one derivatized glycan probe location on the array, and contacting the derivatized probe location with a linker precursor of formula **I** or **II**:

NH₂-(CH₂)n-S-S-(CH₂)n-NH-(C=O)-L₂ **I**

L₁-NH-(C=S)-NH-(CH₂)ₙ-S-S-(CH₂)ₙ-NH-(C=O)-L₂ **II**

wherein L₁ and L₂ are separately each a leaving group, and each n is separately an integer of 1 to 10. The derivatized probe location and the linker precursor are contacted with each other for a time and under conditions sufficient to form a covalent linkage between an amine on the linker and the reactive moieties of the array, thereby generating at least one linker-probe location. For example, when a linker precursor of formula I is used the terminal amine forms a covalent bond with one of the reactive moieties of the array. When a linker precursor of formula II is used, the L₁ leaving group is lost and the amine adjacent to the L₁ group forms a covalent bond with one of the reactive moieties of the array. In many embodiments, the linker precursor is attached to all probe locations on the array and then separate, distinct glycan preparations are linked to separate and distinct probe locations on the array. To attach a glycan preparation to a probe location, a glycan preparation is used that consists of glycans, where each glycan possesses a linking moiety, for example, an azido linking moiety. Thus, after attachment of the linker precursor, a linker-probe location on the array can be contacted with a glycan preparation under conditions sufficient for formation of a covalent bond between a linking moiety on the glycan and a carbonyl of the linker precursor attached ti the array. The L₂ leaving group is lost during this reaction.

The density of glycans at each glycan probe location can be modulated by variyng the concentration of the glycan solution applied to the derivatized glycan probe location.

Another aspect of the invention is array of molecules comprising a library of molecules attached to an array through a cleavable linker, wherein the cleavable linker has the following structure :

X-Cv-Z

wherein :
Cv is a cleavage site ;
X is a solid surface, a spacer group attached to the solid surface or a spacer group with a reactive group for attachment of the linker to a solid surface ; and
Z is a reactive moiety for attachment of a molecule, a spacer group with a reactive moiety for attachment of a molecule, a spacer group with a molecule, or a molecule attached to the linker via a linking moiety.

In some embodiments, the linker is a photocleavable linker comprising either formula **IVa** or **IVb** :

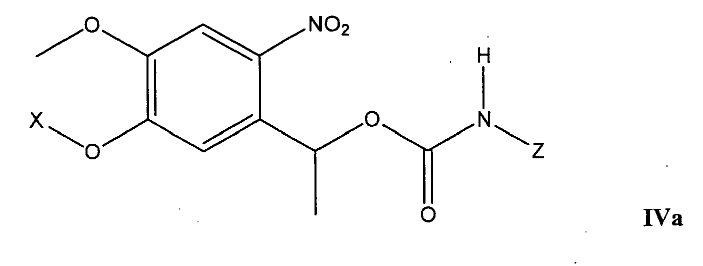

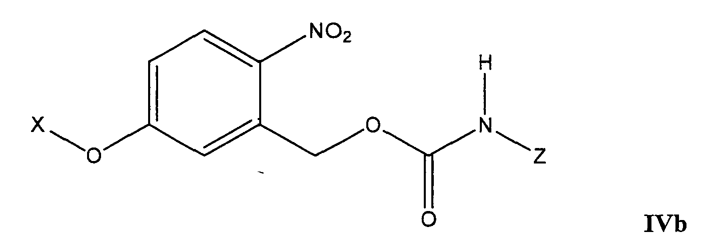

In other embodiments, the linker is a disulfide linker that has the following structure:

### X-S-S-Z

In other embodiments, the linker is a disulfide linker that has the following structure:

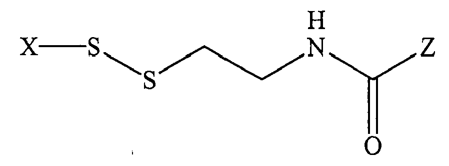

In some embodiments, the solid surface is a glass surface or a plastic surface. For example, the solid surface of the array can be a glass slide or a microtiter plate.

In some embodiments, the linker is cleaved by reduction of a bond. In other embodiments, the linker is cleaved by light. The molecules can include, for example, glycans, nucleic acids or proteins. In some embodiments, the array includes a solid support and a multitude of defined glycan probe locations on the solid support, each glycan probe location defining a region of the solid support that has multiple copies of one type of similar glycan molecules attached thereto. In some embodiments, the multitude of defined glycan probe locations are about 5 to about 200 glycan probe locations.

Another aspect of the invention is a method of testing whether a molecule in a test sample can bind to the present array of molecules comprising, (a) contacting the array with the test sample and (b) observing whether a molecule in the test sample binds to a molecule attached to the array.

Another aspect of the invention is a method of determining which molecular structures bind to biomolecule in a test sample comprising contacting an array of molecules of the invention with a test sample, washing the array and cleaving the cleavable linker to permit structural or functional analysis of molecular structures of the molecules attached to an array. For example, the biomolecule can be an antibody, a receptor or a protein complex.

Another aspect of the invention is a method of detecting breast cancer in a test sample comprising (a) contacting a test sample with glycans comprising glycans **250** or **251**, or a combination thereof: 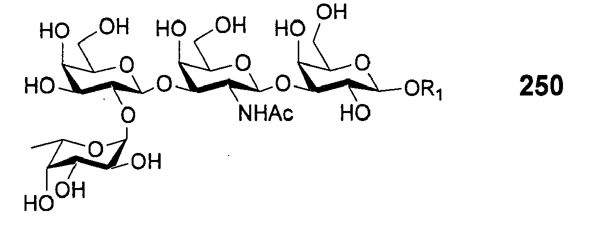 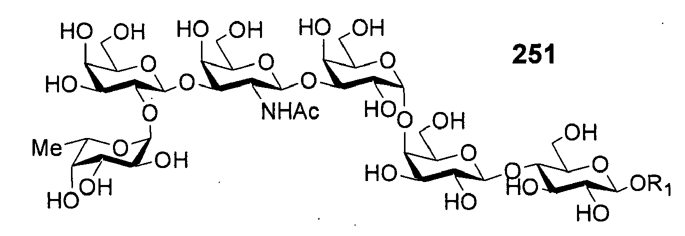 wherein R₁ is hydrogen, a glycan, a linker or a linker attached to a solid support; and
(b) determining whether antibodies in the test sample bind to molecules comprising **250** or **251**.

Another aspect of the invention is a method of detecting HIV infection in a subject comprising (a) contacting a test sample from the subject with an array of mannose containing glycans; and (b) determining whether antibodies in the test sample bind to a glycan comprising Manα1-2Man on a first (α1-3) arm of the glycan or a glycan comprising Manα1-2Man on a (α1-6) third arm of a glycan, or a combination thereof. In some embodiments the antibodies have less affinity for mannose containing glycans that have a second arm from a (α1-3) branch.

Another aspect of the invention is an isolated glycan comprising any one of the following glycans, or a combination thereof: 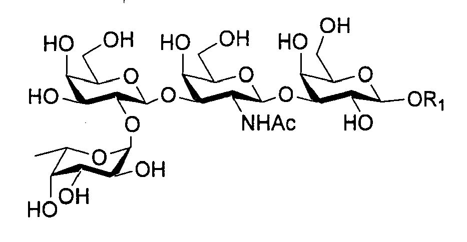 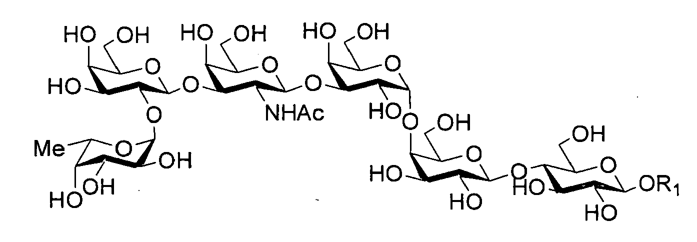 wherein: wherein R₁ is hydrogen, a glycan, a linker. In some embodiments, the linker is or can be attached to a solid support.

Another aspect of the invention is an isolated glycan comprising Manα1-2Man on a first (α1-3) arm of a glycan or Manα1-2Man on a (α1-6) third arm of a glycan, or a combination thereof. In some embodiments, the glycan does not have a second (α1-3) arm.

Another aspect of the invention is an isolated glycan comprising any one of the following oligomannose glycans, or a combination thereof: 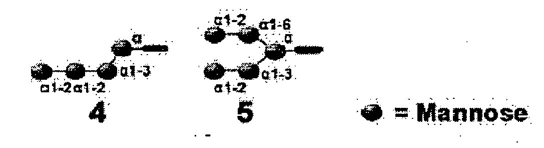 wherein the dash (-) is a covalent bond to another sugar moiety, a covalent bond to a gp20 or gp43 peptide, a covalent bond to a hydrogen, a covalent bond to a linker or a covalent bond to solid support. When the oligomannose glycans are used in pharmaceutical compositions and methods of treating disease the dash (-) is preferably a covalent bond to another sugar moiety, or a covalent bond to a hydrogen or a covalent bond to a linker. The linker can be attached to an anti-viral agent, an anti-bacterial agent or anti-cancer agent.

Another aspect of the invention is a pharmaceutical composition comprising a pharmaceutically acceptable carrier and an effective amount of a glycan comprising any one of the following glycans, or a combination thereof: 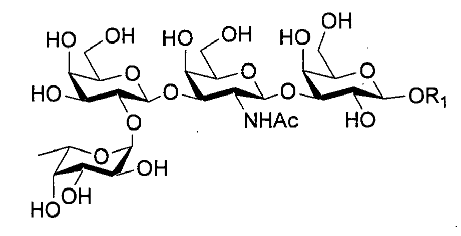 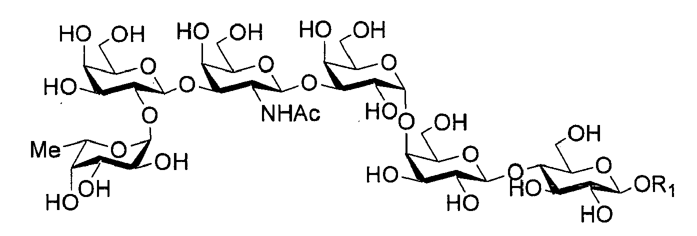 wherein: R₁ is hydrogen, a glycan or a linker. In some embodiments, the linker is or can be attached to a solid support.

Another aspect of the invention is a pharmaceutical composition comprising a pharmaceutically acceptable carrier and an effective amount of a glycan comprising Manα1-2Man on a first (α1-3) arm of a glycan or Manα1-2Man on a (α1-6) third arm of a glycan, or a combination thereof. In some embodiments, the glycan does not have a second (α1-3) arm.

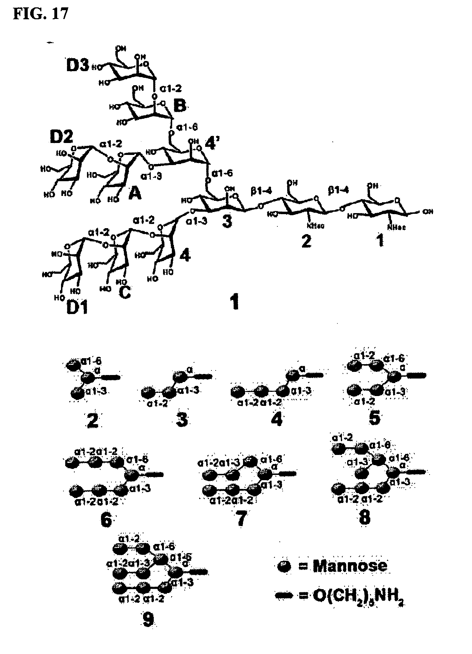

Another aspect of the invention is a pharmaceutical composition comprising a pharmaceutically acceptable carrier and an effective amount of a glycan comprising any one of the following oligomannose glycans, or a combination thereof: 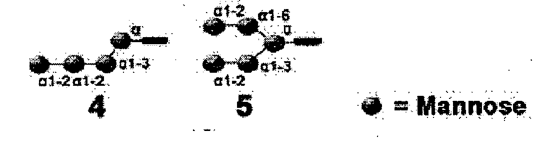 wherein the dash (-) is a covalent bond to another sugar moiety, a covalent bond to a gp20 or gp43 peptide, a covalent bond to a hydrogen, a covalent bond to a linker or a covalent bond to solid support. Other mannose-containing glycans can be included in the compositions of the invention (e.g., mannose-containing glycans having any of the structures shown in FIG. 17 can also be included). When the oligomannose glycans are used in pharmaceutical compositions and methods of treating disease the dash (-) is preferably a covalent bond to another sugar moiety, or a covalent bond to a hydrogen or a covalent bond to a linker. The linker can be attached to an anti-viral agent, an anti-bacterial agent or anti-cancer agent.

Another aspect of the invention is a method of treating or preventing breast cancer in a subject comprising administering a pharmaceutical composition comprising a pharmaceutically acceptable carrier and an effective amount of a glycan comprising any one of the following glycans, or a combination thereof: 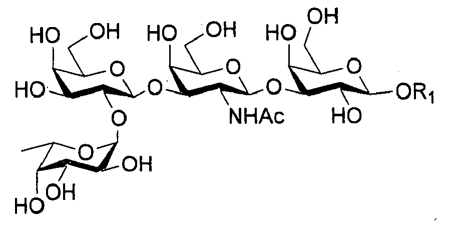 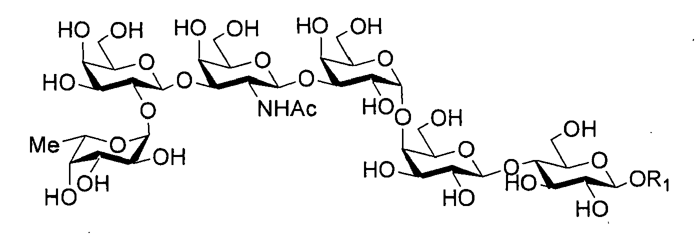 wherein: R₁ is hydrogen, a glycan or a linker. In some embodiments, the linker is or can be attached to a solid support.

Another aspect of the invention is a method for treating or preventing HIV infection in a subject comprising administering to the subject a pharmaceutical composition comprising a pharmaceutically acceptable carrier and an effective amount of a glycan comprising Manα1-2Man on a first (α1-3) arm of a glycan or Manα1-2Man on a (α1-6) third arm of a glycan, or a combination thereof. In some embodiments, the glycan does not have a second (α1-3) arm.

Another aspect of the invention is a method for treating or preventing HIV infection in a subject comprising administering to the subject a pharmaceutical composition comprising a pharmaceutical composition comprising a pharmaceutically acceptable carrier and an effective amount of a glycan comprising any one of the following oligomannose glycans, or a combination thereof: 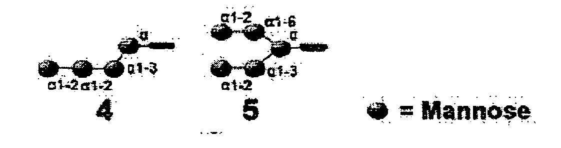 wherein the dash (-) is a covalent bond to another sugar moiety, a covalent bond to a gp20 or gp43 peptide, a covalent bond to a hydrogen, a covalent bond to a linker or a covalent bond to solid support. Other mannose-containing glycans can be included in the compositions used for treating or preventing HIV. When the oligomannose glycans are used in pharmaceutical compositions and methods of treating disease the dash (-) is preferably a covalent bond to another sugar moiety, or a covalent bond to a hydrogen or a covalent bond to a linker. The linker can be attached to an anti-viral agent, an anti-bacterial agent or anti-cancer agent.

### Description of the Figures

FIG. 1 illustrates the covalent attachment of an amino-functionalized glycan library to an N-hydroxysuccinimide (NHS) derivatized surface of a glass microarray.
FIG. 2 graphically illustrates the results of a series of experiments for optimizing the density of glycans on the microarray by varying the glycan concentration and glycan printing time.
FIG. 3A-B illustrate that the plant lectin ConA binds to high-mannose glycans on the printed glycan array. FIG. 3A provides the results for ligands (glycans) 1-104 while FIG. 3B provides the results for ligands (glycans) 105-200. This experiment was performed as a control that helped establish the concentration or density of high-mannose glycans on the printed glycan array was as expected and antibody binding to selected glycans (e.g., the eight residue mannose, or Man8, glycans) was not due to aberrant loading of the Man8 glycan.
FIG. 4A-B illustrates binding of fluorescently labeled plant lectin, *Erythrina cristagalli* (ECA) lectin to a glycan array. FIG. 4A provides the results for ligands (glycans) 1-104 while FIG. 4B provides the results for ligands (glycans) 105-200.
FIG. 5A illustrates binding of E-selectin-Fc chimera to a glycan array with detection by a fluorescently labeled anti-IgG secondary antibody.
FIG. 5B illustrates binding of human CD22-Fc chimera to a glycan array with detection by a fluorescently labeled anti-IgG secondary antibody.
FIG. 6A-B illustrates binding of fluorescently labeled human anti-glycan antibody CD 15 to a glycan array. FIG. 6A provides the results for ligands (glycans) 1-104 while FIG. 6B provides the results for ligands (glycans) 105-200.
FIG. 7A-B illustrates binding of hemaglutinin H1 (1918) of the influenza virus to a glycan array. FIG. 7A provides the results for ligands (glycans) 1-104 while FIG. 7B provides the results for ligands (glycans) 105-200.
FIG. 8 illustrates synthesis of some amine and azide cleavable linkers of the invention.
FIG. 9 illustrates synthesis of some amine and azide cleavable linkers of the invention.
FIG. 10 schematically illustrates attachment of cleavable linkers 1 and 2 to either NHS or amine-coated surfaces, for example, microtiter plates, to provide an array with alkyne=functionalized surface.
FIG. 11A schematically illustrates attachment of a glycan-azide to an alkyne-functionalized solid surface (e.g. a microtiter well) to form an immobilized glycan. The triazole formed upon reaction of the azide and the alkyne can be cleaved by DTT to permit analysis of the glycan structure, for example, by mass spectroscopy.
FIG. 11B illustrates attachment of a mannose-containing glycans to an alkyne-functionalized solid surface (e.g. a microtiter well) to form an immobilized oligomannose. The structures for oligomannoses 4, 5, 6, 7, 8 and 9 are provided in FIG. 17. The triazole formed upon reaction of the azide and the alkyne can be cleaved by DTT to permit analysis of the glycan structure, for example, by mass spectroscopy.
   Reagents and conditions used for step a: TfN₃, CuSO₄, Et₃N, H₂O/CH₂Cl₂/MeOH (1:1:1, v/v), room temperature, 48h; and for step b: CuI, 5% DIEA/ MeOH, room temperature, 12h.
FIG. 12 illustrates how oligosaccharides **201-204b** can be immobilized on a glass slide.
FIG. 13 provides and image of scan of a slide illustrating fluorescence levels following antibody incubation assay. The dots contain sugars **201-204a** printed in the top row from left to right and **201-204b** in the bottom row.
FIG. 14 provides carbohydrate-antibody binding curves for Globo-H analogs **201a, 202a, 203a** and**-204a** (identified as 1a, 2a, 3a and 4a, respectively).
FIG. 15A-B illustrates Globo H structural confirmation by analytical sequence analysis. FIG. 15A is a table showing the glycans obtained by exoglycosidase cleavage with the indicated enzymes along with the glucose unit (GU) value relative to fluorescently labeled dextran standard. FIG. 15B is a sample chromatograms from normal-phase HPLC with fluorescence detection (ex = 330 nm, em = 420 nm) highlighting glycans obtained during sequence analysis.
FIG. 16 graphically illustrates binding of increasing amounts of labeled Manα1,2Manα1,3Manα1,2 Manα1,6Man glycan to a constant amount of 2G12 antibody. This study permitted determination of the *K*_{d} value for oligomannose binding to the anti-HIV 2G12 neutralizing antibody.
FIG. 17 provides chemical structures for Man₉GlcNAc₂ **1** and oligomannoses **2-9.** The mannose residues of Man9GlcNAc2 were labeled in red in the original. To facilitate structural description and reference to branches, arms and mannose residues, all mannose residues of oligomannoses **2-9** are labeled to correspond with their structural equivalent on Man₉GlcNAc₂ and arms D1, D2 and D3 are identified on the Man₉GlcNAc₂ 1 glycan.
FIG. 18 illustrates oligomannose inhibition (%) of 2G12 binding to gp120. Black and grey bars represent the level of inhibition at oligomannose concentrations of 0.5 and 2.0 mM, respectively.

### Detailed Description of the Invention

The invention provides libraries and arrays of glycans that can be used for identifying which types of proteins, receptors, antibodies, lipids, nucleic acids, carbohydrates and other molecules and substances can bind to a given glycan structure.

The inventive libraries, arrays and methods have several advantages. One particular advantage of the arrays of the invention is that the glycans on the arrays are attached by a cleavable linker. For example, the cleavable linkers of the invention can have a disulfide bond that is stable for the types of binding interactions that typically occur between glycans and other biological molecules. However, the cleavable linker can be severed if one of skill in the art chooses so that the linker with the attached glycan can be further analyzed or utilized for other purposes.

The arrays and methods of the invention also provide highly reproducible results. The libraries and arrays of the invention provide large numbers and varieties of glycans. For example, the libraries and arrays of the invention have at least two, at least three, at least ten, or at least 100 glycans. In some embodiments, the libraries and arrays of the invention have about 2 to about 100,000, or about 2 to about 10,000, or about 2 to about 1,000, different glycans per array. Such large numbers of glycans permit simultaneous assay of a multitude of glycan types. As described herein, the present arrays have been used for successfully screening a variety of glycan binding proteins. Such experiments demonstrate that little degradation of the glycan occurs and only small amounts of glycan binding proteins are consumed during a screening assay. Hence, the arrays of the invention can be used for more than one assay. The arrays and methods of the invention provide high signal to noise ratios. The screening methods provided by the invention are fast and easy because they involve only one or a few steps. No surface modifications or blocking procedures are typically required during the assay procedures of the invention. The composition of glycans on the arrays of the invention can be varied as needed by one of skill in the art. Many different glycoconjugates can be incorporated into the arrays of the invention including, for example, naturally occurring or synthetic glycans, glycoproteins, glycopeptides, glycolipids, bacterial and plant cell wall glycans and the like. Immobilization procedures for attaching different glycans to the arrays of the invention are readily controlled to easily permit array construction.

### Definitions

The following abbreviations may be used: α₁-AGP means alpha-acid glycoprotein; AF488 means AlexaFluour-488; CFG means Consortium for Functional Glycomics; Con A means *Concanavalin* A; CVN means Cyanovirin-N; DC-SIGN means dendritic cell-specific ICAM-grabbing nonintegrin; ECA means *Erythrina cristagalli;* ELISA means enzyme-linked immunosorbent assay; FITC means Fluorescinisothiocyanate; GBP means Glycan Binding Protein; HIV means human immunodeficiency virus; HA means influenza hemagglutinin; NHS means N-hydroxysuccinimide; PBS means phosphate buffered saline; SDS means sodium dodecyl sulfate; SEM means standard error of mean; and Siglec means sialic acid immunoglobulin superfamily lectins.

A "defined glycan probe location" as used herein is a predefined region of a solid support to which a density of glycan molecules, all having similar glycan structures, is attached. The terms "glycan region," or "selected region", or simply "region" are used interchangeably herein for the term defined glycan probe location. The defined glycan probe location may have any convenient shape, for example, circular, rectangular, elliptical, wedge-shaped, and the like. In some embodiments, a defined glycan probe location and, therefore, the area upon which each distinct glycan type or a distinct group of structurally related glycans is attached is smaller than about 1 cm², or less than 1 mm², or less than 0.5 mm². In some embodiments the glycan probe locations have an area less than about 10,000 µm² or less than 100 µm². The glycan molecules attached within each defined glycan probe location are substantially identical. Additionally, multiple copies of each glycan type are present within each defined glycan probe location. The number of copies of each glycan types within each defined glycan probe location can be in the thousands to the millions.

As used herein, the arrays of the invention have defined glycan probe locations, each with "one type of glycan molecule." The "one type of glycan molecule" employed can be a group of substantially structurally identical glycan molecules or a group of structurally similar glycan molecules. There is no need for every glycan molecule within a defined glycan probe location to have an identical structure. In some embodiments, the glycans within a single defined glycan probe location are structural isomers, have variable numbers of sugar units or are branched in somewhat different ways. However, in general, the glycans within a defined glycan probe location have substantially the same type of sugar units and/or approximately the same proportion of each type of sugar unit. The types of substituents on the sugar units of the glycans within a defined glycan probe location are also substantially the same.

The term lectin refers to a molecule that interacts with, binds, or crosslinks carbohydrates. The term galectin is an animal lectin. Galectins generally bind galactose-containing glycan.

As used herein a "subject" is a mammal or a bird. Such mammals and birds include domesticated animals, farm animals, animals used in experiments, zoo animals and the like. For example, the subject can be a dog, cat, monkey, horse, rat, mouse, rabbit, goat, ape or human mammal. In other embodiments, the animal is a bird such as a chicken, duck, goose or a turkey. In many embodiments, the subject is a human.

Some of the structural elements of the glycans described herein are referenced in abbreviated form. Many of the abbreviations used are provided in the Table 1. Moreover the glycans of the invention can have any of the sugar units, monosaccharides or core structures provided in Table 1.

**Table 1**

| **Trivial Name** | **Monosaccharide / Core** | **Long Code** | **Short Code** |
|---|---|---|---|
| D-Glcp | D-Glucopyranose | Glc | G |
| D-Galp | D-Galactopyranose | Gal | A |
| D-GlcpNAc | N-Acetylglucopyranose | GlcNAc | GN |
| D-GlcpN | D-Glucosamine | GlcN | GQ |
| D-GalpNAc | N-Acetylgalactopyranose | GalNAc | AN |
| D-GalpN | D-Galactosamine | GalN | AQ |
| D-Manp | D-Mannopyranose | Man | M |
| D-ManpNAc | D-NJ-Acetylmannopyranose | ManNAc | MN |
| D-Neup5Ac | N-Acetylneuraminic acid | NeuAc | NN |
| D-Neu5G | D-N-Glycolylneuraminic acid | NeuGc | NJ |
| D-Neup | Neuraminic acid | Neu | N |
| KDN* | 2-Keto-3-deoxynananic acid | KDN | K |
| Kdo | 3-deoxy-D-manno-2 octulopyranosylono | Kdo | W |
| D-GalpA | D-Galactoronic acid | GalA | L |
| D-Idop | D-Iodoronic acid | Ido | I |
| L-Rhap | L-Rhamnopyranose | Rha | H |
| L-Fucp | L-Fucopyranose | Fuc | F |
| D-Xylp | D-Xylopyranose | Xyl | X |
| D-Ribp | D-Ribopyranose | Rib | B |
| L-Araf | L-Arabinofuranose | Ara | R |
| D-G1cpA | D-Glucoronic acid | GlcA | U |
| D-Allp | D-Allopyranose | All | O |
| D-Apip | D-Apiopyranose | Api | P |
| D-Tagp | D-Tagopyranose | Tag | T |
| D-Abep | D-Abequopyranose | Abe | Q |
| D-Xulp | D-Xylulopyranose | Xul | D |
| D-Fruf | D-Fructofuranose | Fru | E |

| | | | |
|---|---|---|---|
| * Another name for KDN is: 3-deoxy-D-glycero-K-galacto-nonulosonic acid. | | | |

The sugar units or other saccharide structures present in the glycans of the invention can be chemically modified in a variety of ways. A listing of some of the types of modifications and substituents that the sugar units in the glycans of the invention can possess, along with the abbreviations for these modifications/substituents is provided below in Table 2.

**Table 2**

| **Modification type** | **Symbol** | | **Modification type** | **Symbol** |
|---|---|---|---|---|
| Acid | A | | Acid | A |
| N-Methylcarbamoyl | ECO | | deacetylated N-Acetyl (amine) Q | Q |
| pentyl | EE | | Deoxy | Y |
| octyl | EH | | Ethyl | ET |
| ethyl | ET | | Hydroxyl | OH |
| inositol | IN | | Inositol | IN |
| N-Glycolyl | J | | Methyl | ME |
| methyl | ME | | N-Acetyl | N |
| N-Acetyl | N | | N-Glycolyl | J |
| hydroxyl | OH | | N-Methylcarbamoyl | ECO |
| phosphate | P | | N-Sulfate | QS |
| phosphocholine | PC | | O-Acetyl | T |
| Phosphoethanolamine (2-aminoethylphosphate) | PE | | Octyl | EH |
| Pyrovat acetal | PYR* | | Pentyl | EE |
| Deacetylated N-Acetyl (amine) | Q | | Phosphate | P |
| N-Sulfate | QS | | Phosphocholine | PC |
| sulfate | S or Su | | Phosphoethanolamine (2-aminoethylphosphate) | PE |
| O-Acetyl | T | | Pyrovat acetal | PYR* |
| deoxy | Y | | | |

| | | | | |
|---|---|---|---|---|
| * when 3 is present, it means 3,4, when 4 is present it means 4,6. | | | | |

Bonds between sugar units are alpha (α) or beta (β) linkages, meaning that relative to the plane of the sugar ring; an alpha bond goes down whereas a beta bond goes up. In the shorthand notation sometimes used herein, the letter "a" is used to designate an alpha bond and the letter "b" is used to designate a beta bond.

### Cleavable Linkers

The invention provides cleavable linkers that can be attached to a solid support or an array to permit release of a molecule or complex bound to the solid support or array through the cleavable linker. These cleavable linkers can be used to attach a variety of molecules to solid supports and arrays. For example, the cleavable linkers can be used to attach molecules such as glycans, nucleic acids or proteins to solid supports or arrays. In some embodiments, the cleavable linkers are used to attach glycans to a solid support or array.

In one embodiment, the invention a cleavable linker, wherein the cleavable linker has the following structure:

X-Cv-Z I

wherein:
Cv is a cleavage site;
X is a solid surface or a spacer group attached to the solid surface or a spacer group with a reactive group for attachment of the linker to a solid surface; and
Z is a reactive moiety for attachment of a molecule, a spacer group with a reactive moiety for attachment of a molecule, a spacer group with a molecule or a molecule attached to the cleavable linker via a linking moiety.

In another embodiment, the invention provides a disulfide linker, wherein the disulfide linker has the following structure:

X-S-S-Z II

wherein:
X is a solid surface or a spacer group attached to the solid surface or a spacer group with a reactive group for attachment of the linker to a solid surface; and
Z is a reactive moiety for attachment of a molecule, a spacer group with a reactive moiety for attachment of a molecule, a spacer group with a molecule, or a molecule attached to the linker via a linking moiety.

In further embodiments, the cleavable linker is a disulfide linker that has the following structure: 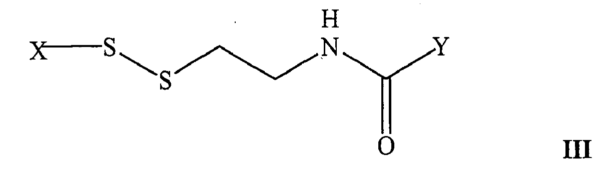 wherein:
X is a solid surface or a spacer group attached to the solid surface; and
Y is a leaving group or a glycan attached to the disulfide linker via a triazole moiety.

In another embodiment, the invention provides photocleavable linkers having either of the following structures **IVa** or **IVb:** 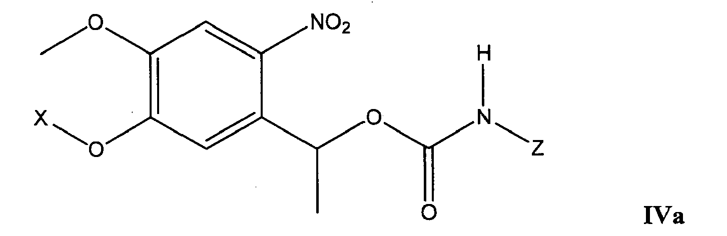 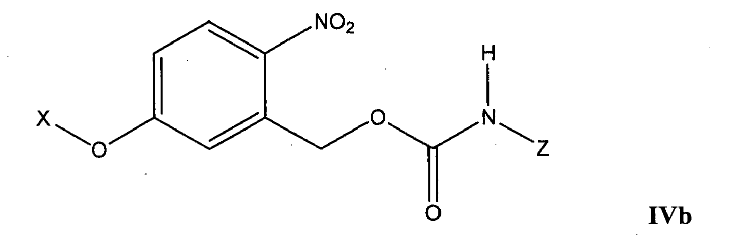 wherein:
X is a solid surface or a spacer group attached to the solid surface or a spacer group with a reactive group for attachment of the linker to a solid surface; and
Z is a reactive moiety for attachment of a molecule, a spacer group with a reactive moiety for attachment of a molecule, a spacer group with a molecule, or a molecule attached to the linker via a linking moiety.

The molecules attached to the photocleavable linkers of formula **IVa** and **IVb** can be cleaved from an attached solid support using light form a laser, for example, ultraviolet light from a laser. In some embodiments, the laser provides light of about 340-400 nm, or about 360 nm. The molecule is released from the solid support by photocleavage of the linker to facilitate functional or structural characterization of the molecule.

Spacer molecules or groups include fairly stable (e.g. substantially chemically inert) chains or polymers. For example, the spacer molecules or groups can be alkylene groups. One example of an alkylene group is -(CH₂)ₙ-, where n is an integer of from 1 to 10.

Suitable leaving groups are well known in the art, for example, but not limited to alkynes, such as-C≡CH; halides, such as chloride, bromide, and iodide; aryl- or alkylsulfonyloxy, substituted arylsulfonyloxy (e.g., tosyloxy or mesyloxy); substituted alkylsulfonyloxy (e.g., haloalkylsulfonyloxy); phenoxy or substitute phenoxy; and acyloxy groups.

In another embodiment, the invention provides a method making the arrays of the invention that involves derivatizing the solid support surface of the array with trialkoxysilane bearing reactive moieties such as N-hydroxysuccinimide (NHS), amino (--NH₂), isothiocyanate (--NCS) or hydroxyl (--OH) to generate at least one derivatized glycan probe location on the array, and contacting the derivatized probe location with a linker precursor of formula **V** or **VI:**

NH₂-(CH₂)ₙ-S-S-(CH₂)ₙ -NH-(C=O)-L₂ **V**

L₁-NH-(C=S)-NH₄CH₂)ₙ-S-S-(CH₂)ₙ -NH-(C=O)-L₂ **VI**

wherein L₁ and L₂ are separately each a leaving group, and each n is separately an integer of 1 to 10.

Thus the derivatized probe location and the linker precursor can be contacted with each other for a time and under conditions sufficient to form a covalent linkage between an amine on the linker and the reactive moieties of the array, thereby generating at least one linker-probe location. For example, when a linker precursor of formula V is used the terminal amine forms a covalent bond with one of the reactive moieties of the array. When a linker precursor of formula VI is used, the L₁ leaving group is lost and the amine adjacent to the L₁ group forms a covalent bond with one of the reactive moieties of the array. In many embodiments, the linker precursor is attached to all probe locations on the array and then separate, distinct glycan preparations are linked to separate and distinct probe locations on the array. To attach a glycan preparation to a probe location, a glycan preparation is used that consists of glycans, where each glycan possesses a linking moiety, for example, an azido linking moiety. Thus, after attachment of the linker precursor, a linker-probe location on the array can be contacted with a glycan preparation under conditions sufficient for formation of a covalent bond between a linking moiety on the glycan and a carbonyl of the linker precursor attached to the array. The L₂ leaving group is lost during this reaction.

Such methods can be adapted for use with any convenient solid support.

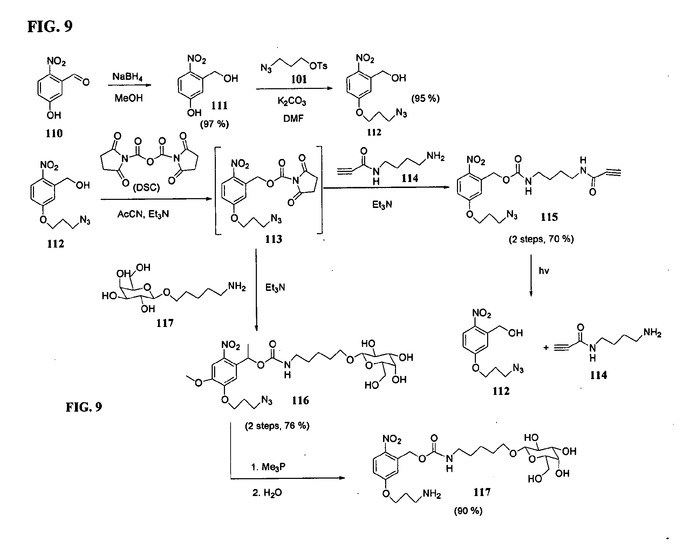

As illustrated herein, linkers **1** and **2** were synthesized for the covalent attachment of azide-containing saccharides to a solid support (see FIG. 9-11 and Example 7). The thioisocyanate (**2**) was generated from amine **1** for use with amine-coated solid supports and arrays.

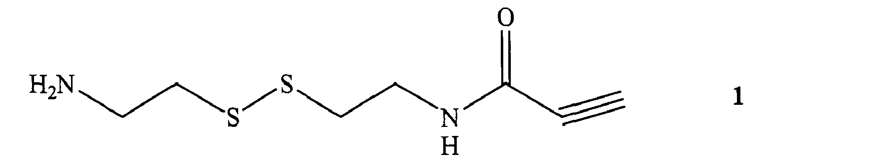

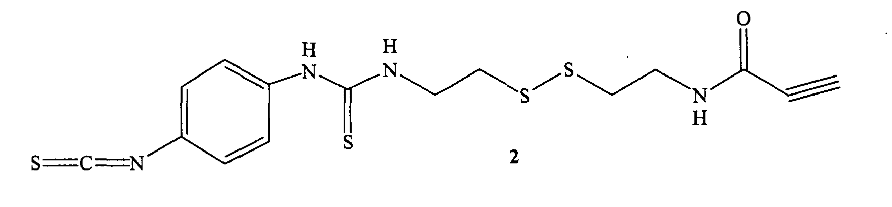

Such cleavable linkers can be attached to a solid support or array as described above. In one embodiment, linker **1** was attached to the NHS-coated surface under basic conditions to give the alkyne-functionalized surface. Attachment of the linker was verified via mass spectrometry (MS).

After incubation of linkers **1** and **2**, surfaces were repeatedly washed with water. Reaction of linkers **1** and **2** with dithiothreitol (DTT) will reduce the disulfide bonds and release any entities (e.g. glycans) linked thereto. See Lack et al. Helv. Chim. Acta 2002, 85, 495-501; Lindroos et al. Nucleic Acids. Res. 2001, 29, E69; Rogers et al. Anal. Biochem. 1999, 266, 23-30; Guillier et al. Chem. Rev. 2000, 100, 2091-2158. Cleavage was monitored directly by sonic spray ionization (SSI) and electrospray ionization (ESI) MS, which not only verified the presence of the linker but also showed low background upon DTT treatment.

Capture of azide-containing glycans onto alkyne derivatized solid supports was then accomplished by contacting probe locations or functionalized solid support surfaces displaying the activated alkyne leaving groups with the azide-containing sugars in the presence of CuI. See FIG. 9-11 and Example 7. The efficiency of this attachment method was then monitored over time using DTT or light-induced cleavage. The liberated cleavage product was directly analyzed by mass spectrometry to confirm the identity of the product's structure. This attachment strategy was successfully used to attach submicromolar concentrations to solid support surfaces and was successfully applied to the covalent attachment of numerous glycans.

### Glycans

The invention provides compositions and libraries of glycans that include numerous different types of carbohydrates and oligosaccharides. In general, the major structural attributes and composition of the separate glycans within the libraries have been identified. In some embodiments, the libraries consist of separate, substantially pure pools of glycans, carbohydrates and/or oligosaccharides. The libraries of the invention can have an attached cleavable linker of the invention.

The glycans of the invention include straight chain and branched oligosaccharides as well as naturally occurring and synthetic glycans. For example, the glycan can be a glycoaminoacid, a glycopeptide, a glycolipid, a glycoaminoglycan (GAG), a glycoprotein, a whole cell, a cellular component, a glycoconjugate, a glycomimetic, a glycophospholipid anchor (GPI), glycosyl phosphatidylinositol (GPI)-linked glycoconjugates, bacterial lipopolysaccharides and endotoxins.

The glycans of the invention include 2 or more sugar units. Any type of sugar unit can be present in the glycans of the invention, including, for example, allose, altrose, arabinose, glucose, galactose, gulose, fucose, fructose, idose, lyxose, mannose, ribose, talose, xylose, or other sugar units. The tables provided herein list other examples of sugar units that can be used in the glycans of the invention. Such sugar units can have a variety of modifications and substituents. Some examples of the types of modifications and substituents contemplated are provided in the tables herein. For example, sugar units can have a variety of substituents in place of the hydroxy (-OH), carboxylate (-COO⁻), and methylenehydroxy (-CH₂-OH) substituents. Thus, lower alkyl moieties can replace any of the hydrogen atoms from the hydroxy (-OH), carboxylic acid (-COOH) and methylenehydroxy (-CH₂-OH) substituents of the sugar units in the glycans of the invention. For example, amino acetyl (-NH-CO-CH₃) can replace any of the hydrogen atoms from the hydroxy (-OH), carboxylic acid (-COOH) and methylenehydroxy (-CH₂-OH) substituents of the sugar units in the glycans of the invention. N-acetylneuraminic acid can replace any of the hydrogen atoms from the hydroxy (-OH), carboxylic acid (-COOH) and methylenehydroxy (-CH₂-OH) substituents of the sugar units in the glycans of the invention. Sialic acid can replace any of the hydrogen atoms from the hydroxy (-OH), carboxylic acid (-COOH) and methylenehydroxy (-CH₂-OH) substituents of the sugar units in the glycans of the invention. Amino or lower alkyl amino groups can replace any of the OH groups on the hydroxy (-OH), carboxylic acid (-COOH) and methylenehydroxy (-CH₂-OH) substituents of the sugar units in the glycans of the invention. Sulfate (-SO₄⁻) or phosphate (-PO₄⁻) can replace any of the OH groups on the hydroxy (-OH), carboxylic acid (-COOH) and methylenehydroxy (-CH₂-OH) substituents of the sugar units in the glycans of the invention. Hence, substituents that can be present instead of, or in addition to, the substituents typically present on the sugar units include N-acetyl, N-acetylneuraminic acid, oxy (=O), sialic acid, sulfate (-SO₄⁻), phosphate (-PO₄⁻), lower alkoxy, lower alkanoyloxy, lower acyl, and/or lower alkanoylaminoalkyl.

The following definitions are used, unless otherwise described: Alkyl, alkoxy, alkenyl, alkynyl, etc. denote both straight and branched groups; but reference to an individual radical such as Apropyl@ embraces only the straight chain radical, when a branched chain isomer such as Aisopropyl@ has been specifically referred to. Halo is fluoro, chloro, bromo, or iodo.

Specifically, lower alkyl refers to (C₁-C₆)alkyl, which can be methyl, ethyl, propyl, isopropyl, butyl, iso-butyl, sec-butyl, pentyl, 3-pentyl, or hexyl; (C₃-C₆)cycloalkyl can be cyclopropyl, cyclobutyl, cyclopentyl, or cyclohexyl; (C₃-C₆)cycloalkyl(C₁-C₆)alkyl can be cyclopropylmethyl, cyclobutylmethyl, cyclopentylmethyl, cyclohexylmethyl, 2-cyclopropylethyl, 2-cyclobutylethyl, 2-cyclopentylethyl, or 2-cyclohexylethyl; (C₁-C₆)alkoxy can be methoxy, ethoxy, propoxy, isopropoxy, butoxy, iso-butoxy, sec-butoxy, pentoxy, 3-pentoxy, or hexyloxy.

It will be appreciated by those skilled in the art that the glycans of the invention having one or more chiral centers may exist in and be isolated in optically active and racemic forms. Some compounds may exhibit polymorphism. It is to be understood that the present invention encompasses any racemic, optically-active, polymorphic, or stereoisomeric form, or mixtures thereof, of a glycan of the invention, it being well known in the art how to prepare optically active forms (for example, by resolution of the racemic form by recrystallization techniques, by synthesis from optically-active starting materials, by-chiral synthesis, or by chromatographic separation using a chiral stationary phase).

Specific and preferred values listed below for substituents and ranges, are for illustration only; they do not exclude other defined values or other values within defined ranges or for the substituents.

The libraries of the invention are particularly useful because diverse glycan structures are difficult to make and substantially pure solutions of a single glycan type are hard to generate. For example, because the sugar units typically present in glycans have several hydroxyl (-OH) groups and each of those hydroxyl groups is substantially of equal chemical reactivity, manipulation of a single selected hydroxyl group is difficult. Blocking one hydroxyl group and leaving one free is not trivial and requires a carefully designed series of reactions to obtain the desired regioselectivity and stereoselectivity. Moreover, the number of manipulations required increases with the size of the oligosaccharide. Hence, while synthesis of a disaccharide may require 5 to 12 steps, as many as 40 chemical steps can be involved in synthesis of a typical tetrasaccharide. In the past, chemical synthesis of oligosaccharides was therefore fraught with purification problems, low yields and high costs. However the invention has solved these problems by providing libraries and arrays of numerous structurally distinct glycans.

The glycans of the invention have been obtained by a variety of procedures. For example, some of the chemical approaches developed to prepare *N*-acetyllactosamines by glycosylation between derivatives of galactose and *N*-acetylglucosamine are described in Aly, M. R. E.;Ibrahim, E.-S. I.;El-Ashry, E.-S. H. E. and Schmidt, R. R., Carbohydr. Res. 1999, 316, 121-132; Ding, Y.;Fukuda, M. and Hindsgaul, O., Bioorg. Med. Chem. Lett. 1998, 8, 1903-1908; Kretzschmar, G. and Stahl, W., Tetrahedr. 1998, 54, 6341-6358*.* These procedures can be used to make the glycans of the present libraries, but because there are multiple tedious protection/deprotection steps involved in such chemical syntheses, the amounts of products obtained in these methods can be low, for example, in milligram quantities.

One way to avoid protection-deprotection steps typically required during glycan synthesis is to mimic nature's way of synthesizing oligosaccharides by using regiospecific and stereospecific enzymes, called glycosyltransferases, for coupling reactions between the monosaccharides. These enzymes catalyze the transfer of a monosaccharide from a glycosyl donor (usually a sugar nucleotide) to a glycosyl acceptor with high efficiency. Most enzymes operate at room temperature in aqueous solutions (pH 6-8), which makes it possible to combine several enzymes in one pot for multi-step reactions. The high regioselectivity, stereoselectivity and catalytic efficiency make enzymes especially useful for practical synthesis of oligosaccharides and glycoconjugates. See Koeller, K. M. and Wong, C.-H., Nature 2001, 409, 232-240; Wymer, N. and Toone, E. J., Curr. Opin. Chem. Biol. 2000, 4, 110-119; Gijsen, H. J. M.;Qiao, L.;Fitz, W. and Wong, C.-H., Chem. Rev. 1996, 96, 443-473.

Recent advances in isolating and cloning glycosyltransferases from mammalian and non-mammalian sources such as bacteria facilitate production of various oligosaccharides. DeAngelis, P. L., Glycobiol. 2002, 12, 9R-16R; Endo, T. and Koizumi, S., Curr. Opin. Struct. Biol. 2000, 10, 536-541; Johnson, K. F., Glycoconj. J. 1999, 16, 141-146. In general, bacterial glycosyltransferases are more relaxed regarding donor and acceptor specificities than mammalian glycosyltransferases. Moreover, bacterial enzymes are well expressed in bacterial expression systems such as *E. coli* that can easily be scaled up for over expression of the enzymes. Bacterial expression systems lack the post-translational modification machinery that is required for correct folding and activity of the mammalian enzymes whereas the enzymes from the bacterial sources are compatible with this system. Thus, in many embodiments, bacterial enzymes are used as synthetic tools for generating glycans, rather than enzymes from the mammalian sources.

For example, the repeating Galβ(1-4)GlcNAc- unit can be enzymatically synthesized by the concerted action of β4-galactosyltransferase (β4GalT) and β3-*N*-acetyllactosamninyltransferase (β3GlcNAcT). Fukuda, M., Biochim. Biophys. Acta. 1984, 780:2, 119-150; Van den Eijnden, D. H.;Koenderman, A. H. L. and Schiphorst, W. E. C. M., J. Biol. Chem. 1988, 263, 12461-12471. The inventors have previously cloned and characterized the bacterial *N. meningitidis* enzymes β4GalT-GalE and β3GlcNAcT and demonstrated their utility in preparative synthesis of various galactosides. Blixt, O.; Brown, J.;Schur, M.;Wakarchuk, W. and Paulson, J. C., J Org. Chem. 2001, 66, 2442-2448; Blixt, O.;van Die, I.;Norberg, T. and van den Eijnden, D. H., Glycobiol. 1999, 9, 1061-1071. β4GalT-GalE is a fusion protein constructed from β4GalT and the uridine-5'-diphospho-galactose-4'-epimerase (GalE) for *in situ* conversion of inexpensive UDP-glucose to UDP-galactose providing a cost efficient strategy. Further examples of procedures used to generate the glycans, libraries and arrays of the invention are provided in the Examples.

While any glycans can be used with the linkers, arrays and methods of the invention, some examples of glycans are provided in Table 3. Abbreviated names as well as complete names are provided.

**Table 3**

| No. | **Glycan** |
|---|---|
| 1. | AGP α-**acid glycoprotein** |
| 2. | AGPAα-**acid glycoprotein glycoformA** |
| 3. | AGPBα-**acid glycoprotein glycoformB** |
| 4. | Ceruloplasmine |
| 5. | Fibrinogen |
| 6. | Transferrin |
| 7. | (Ab4[Fa3]GNb)2#sp1 **LeX** |
| 8. | (Ab4[Fa3]GNb)3#sp1 **LeX** |
| 9. | (Ab4GNb)3#sp1 **Tri-LacNAc** |
| 10. | [3OSO3]Ab#sp2 **3SuGal** |
| 11. | [3OSO3]Ab3ANa#sp2 **3'SuGalβ3GalNAc** |
| 12. | [3OSO3]Ab3GNb#sp2 **3'SuGalβ3GalNAc** |
| 13. | [3OSO3]Ab4[6OSO3]Gb#sp1 **3'6DiSuLac** |
| 14. | [3OSO3]Ab4[6OSO3]Gb#sp2 **3'6DiSuLac** |
| 15. | [3OSO3]Ab4Gb#sp2 **3'SuLac** |
| 16. | [3OSO3]Ab4GNb#sp2 **3'SuLacNAc** |
| 17. | [4OSO3]Ab4GNb#sp2 **4'SuLacNAc** |
| 18. | [6OPO3]Ma#sp2 **6PMan** |
| 19. | [60SO3]Ab4[6OSO3]Gb#sp2 **6'6DiSuLac** |
| 20. | [6OSO3]Ab4Gb#sp1 **6'SuLac** |
| 21. | [6OSO3]Ab4Gb#sp2 **6'SuLac** |
| 22. | [6OSO3]GNb#sp2 **6SuGlcNAc** |
| 23. | [GNb3[GNb6]GNb4]ANa#sp2 |
| 24. | [NNa3Ab]2GNb#sp2 **(Sia)2GlcNAc** |
| 25. | 3OSO3Ab3[Fa4]GNb#sp2 **3'SuLe a** |
| 26. | 3OSO3Ab4[Fa3]GNb#sp2 **3'SuLe X** |
| 27. | 9NAcNNa#sp2 **9NAc-Neu5Ac** |
| 28. | 9NAcNNa6Ab4GNb#sp2 **9NAc-Neu5Ac2,6LacNAc** |
| 29. | Aa#sp2 **Galα** |
| 30. | Aa2Ab#sp2 **Galα2Gal** |
| 31. | Aa3[Aa4]Ab4GNb#sp2 **Galα3[Galα4]LacNAc** |
| 32. | Aa3[Fa2]Ab#sp2 **Galα3[Fuc]Gal**β |
| 33. | Aa3Ab#sp2 **Galα3Gal** |
| 34. | Aa3Ab4[Fa3]GN#sp2 **Galα3Le X** |
| 35. | Aa3Ab4Gb#sp1 **Galα3Lac** |
| 36. | Aa3Ab4GN#sp2 **Galα3LacNAc** |
| 37. | Aa3Ab4GNb#sp2 **Galα3LacNAc** |
| 38. | Aa3ANa#sp2 **Galα3GalNAc** |
| 39. | Aa3ANb#sp2 **Galα-3GalNAc** |
| 40. | Aa4[Fa2]Ab4GNb#sp2 **Galα4[Fucα2]LacNAc** |
| 41. | Aa4Ab4Gb#sp1 **Galα4Lac** |
| 42. | Aa4Ab4GNb#sp1 **Galα4LacNAc** |
| 43. | Aa4Ab4GNb#sp2 **Galα4LacNAc** |
| 44. | Aa4GNb#sp2 **Galα4GlcNAc** |
| 45. | Aa6Gb#sp2 **Galα6Gal** |
| 46. | Ab#sp2 **Gal** |
| 47. | Ab[NNa6]ANa#sp2 **6Sialyl-T** |
| 48. | Ab2Ab#sp2 **Galβ2Gal** |
| 49. | Ab3[Ab4GNb6]ANa#sp2 **6LacNAc-Core2** |
| 50. | Ab3[Fa4]GNb#sp1 **Le a** |
| 51. | Ab3[Fa4]GNb#sp2 **Le a** |
| 52. | Ab3[GNb6]ANa#sp2 **Core-2** |
| 53. | Ab3[NNa6]GNb4Ab4Gb#sp4 **LSTc** |
| 54. | Ab3[NNb6]ANa#sp2 **β6Sialyl-T** |
| 55. | Ab3Ab#sp2 **Galβ3Gal** |
| 56. | Ab3ANa#sp2 **Galβ3GalNAc**α |
| 57. | Ab3ANb#sp2 **Galβ3GalNAc**β |
| 58. | Ab3ANb4[NNa3]Ab4Gb#sp1 **GM1** |
| 59. | Ab3ANb4Ab4Gb#sp2 **a-sialo-GM1** |
| 60. | Ab3GNb#sp1 **LeC** |
| 61. | Ab3GNb#sp2 **LeC** |
| 62. | Ab3GNb3Ab4Gb4b#sp4 **LNT** |
| 63. | Ab4[6OSO3]Gb#sp **16SuLac** |
| 64. | Ab4[6OSO3]Gb#sp2 **6SuLac** |
| 65. | Ab4[Fa3]GNb#sp1 **LeX** |
| 66. | Ab4[Fa3]GNb#sp2 **LeX** |
| 67. | Ab4ANa3[Fa2]Ab4GNb#sp2 |
| 68. | Ab4Gb#sp1 **Lac** |
| 69. | Ab4Gb#sp2 **Lac** |
| 70. | Ab4GNb#sp1 **LacNAc** |
| 71. | Ab4GNb#sp2 **LacNAc** |
| 72. | Ab4GNb3 [Ab4GNb6]ANa#sp2 **(LacNAc)2-Core2** |
| 73. | Ab4GNb3Ab4[Fa3]GNb3Ab4[Fa3]GNb#sp1 **LacNAc-LeX-LeX** |
| 74. | Ab4GNb3Ab4Gb#sp1 **LNnT** |
| 75. | Ab4GNb3Ab4Gb#sp2 **LNnT** |
| 76. | Ab4GNb3Ab4GNb#sp1 **LacNAc-LacNAc** |
| 77. | Ab4GNb3ANa#sp2a **3LacANTcα-Core-2** |
| 78. | Ab4GNb3ANa#sp2b **3LacNAcβ-Core-2** |
| 79. | Ab4GNb6ANa#sp2 **6LacANcα-Core-2** |
| 80. | ANa#sp2 **Tn** |
| 81. | ANa3[Fa2]Ab#sp2 **A-tri** |
| 82. | ANa3Ab#sp2 **GalNAcα3Gal** |
| 83. | ANa3Ab4GNb#sp2 **GalNAcα3LacNAc** |
| 84. | ANa3ANb#sp2 **GalNAcα3GalNAc** |
| 85. | ANa4[Fa2]Ab4GNb#sp2 **GalNAcα4[Fucα2]LacNAc** |
| 86. | ANb#sp2 **GalNAcβ** |
| 87. | ANb3[Fa2]Ab#sp2 **GalNAcβ[Fucα2]Gal** |
| 88. | ANb3Ana#sp2 **GAlNAcβ3GalNAc** |
| 89. | ANb4GNb#sp1 **LacDiNAc** |
| 90. | ANb4GNb#sp2 **LacDiNAc** |
| 91. | Fa#sp2 **Fuc** |
| 92. | Fa#sp3 **Fuc** |
| 93. | Fa2Ab#sp2 **Fucα2Gal** |
| 94. | Fa2Ab3[Fa4]GNb#sp2 **Le b** |
| 95. | Fa2Ab3ANa#sp2 **H-type 3** |
| 96. | Fa2Ab3ANb3Aa#sp3 **H-type3β3Gal** |
| 97. | Fa2Ab3ANb3Aa4Ab4G#sp3 **Globo-H** |
| 98. | Fa2Ab3ANb4[NNa3]Ab4Gb#sp1 **Fucosyl-GM1** |
| 99. | Fa2Ab3GNb#sp1 **H-type 1** |
| 100. | Fa2Ab3GNb#sp2 **H type 1** |
| 101. | Fa2Ab4[Fa3]GNb#sp1 **Le Y** |
| 102. | Fa2Ab4[Fa3]GNb#sp2 **LeY** |
| 103. | Fa2Ab4Gb#sp1 **2'FLac** |
| 104. | Fa2Ab4GNb#sp1 **H-type 2** |
| 105. | Fa2Ab4GNb#sp2 **H-type 2** |
| 106. | Fa2Ab4GNb3Ab4GNb#sp1 **H-type-2-LacNAc** |
| 107. | Fa2Ab4GNb3Ab4GNb3Ab4GNb#sp1 **H-type2-LacNAc-LacNAc** |
| 108. | Fa2GNb#sp2 **Fuα2GlcNAc** |
| 109. | Fa3GNb#sp2 **Fucα3GlcNAc** |
| 110. | Fb3GNb#sp2 **Fucβ3GlcNAc** |
| 111. | Fa2Ab3ANb4[NNa31Ab4Gb#sp3 **Fucosyl-GM1** |
| 112. | Ga#sp2 **Galα** |
| 113.. | Ga4Gb#sp2 **Galα4Gal** |
| 114. | Gb#sp2 **Galβ** |
| 115. | Gb4Gb#sp2 **Galβ4Gal** |
| 116. | Gb6Gb#sp2 **Galβ6Gal** |
| 117. | GNb#sp1 **GlcNAc** |
| 118. | GNb#sp2 **GlcNAc** |
| 119. | GNb2Ab3ANa#sp2 **GlcNAcβ2-Core-1** |
| 120. | GNb3[GNb6]ANa#sp2 **GlcNAcβ3[GlcNAcβ6GalNAc** |
| 121. | GNb3Ab#sp2 **GlcNAcβ3Gal** |
| 122. | GNb3Ab3ANa#sp2 **GlcNAcβ3-Core1** |
| 123. | GNb3Ab4Gb#sp1 **LNT-2** |
| 124. | GNb3Ab4GNb#sp1 **GlcNAcβ3LacNAc** |
| 125. | GNb4[GNb6]ANa#sp2 **GlcNAcβ4[GlcNAcβ6]GalNAc** |
| 126. | GNb4GNb4GNb4b#sp2 **Chitotriose** |
| 127. | GNb4MDPLys |
| 128. | GNb6ANs#sp2 **GlcANcβ6GalNAc** |
| 129. | G-ol-amine **glucitolamine** |
| 130. | GUa#sp2 **Glucurinic acidα** |
| 131. | GUb#sp2 **Glucuronic acid**β |
| 132. | Ka3Ab3GNb#sp1 **KDNα2,3-type1** |
| 133. | Ka3Ab4GNb#sp **KDBα2,3-LacNAc** |
| 134. | Ma#sp2 **Mannose α** |
| 135. | Ma2Ma2Ma3Ma#sp3 |
| 136. | Ma2Ma3[Ma2Ma6]Ma#sp3 |
| 137. | Ma2Ma3Ma#sp3 |
| 138. | Ma3[Ma2Ma2Ma6]Ma#sp3 |
| 139. | Ma3[Ma6]Ma#sp3 **Man-3** |
| 140. | Man-5#aa **Man5-aminoacid** |
| 141. | Man5-9 pool **Man5-9-aminoacid** |
| 142. | Man-6#aa **Man6-aminoacid** |
| 143. | Man-7#aa **Man7-aminoacid** |
| 144. | Man-8#aa **Man8-aminoacid** |
| 145. | Man-9#aa **Man9-aminoacid** |
| 146. | Na8Na#sp2 **Neu5Acα2,8Neu5Ac** |
| 147. | Na8Na8Na#sp2 **Neu5Acα2,8Neu5Acα2,5Neu5Ac** |
| 148. | NJa#sp2 **Neu5Gc** |
| 149. | NJa3Ab3[Fa4]GNb#sp1 **Neu5GcLe a** |
| 150. | NJa3Ab3GbN#sp1 **Neu5Gc-type1** |
| 151. | NJa3Ab4[Fa3]GNb#sp1 **Neu5Gc-LeX** |
| 152. | NJa3Ab4Gb#sp1 **Neu5Gcα3Lactose** |
| 153. | NJa3Ab4GNb#spl **Neu5Gcα3LacNAc** |
| 154. | NJa6Ab4GNb#sp1 **Neu5Gcα6LacNAc** |
| 155. | NJa6ANa#sp2 **Neu5Gc6GalNAc (STn)** |
| 156. | NNa#sp2 **Neu5Ac** |
| 157. | NNa3[6OSO3]Ab4GNb#sp2 **3'Sia[6'Su]LacNAc** |
| 158. | NNa3[ANb4]Ab4Gb#sp1 **GM2** |
| 159. | NNa3[ANb4]Ab4GNb#sp1 **GM2(NAc)/CT/Sda** |
| 160. | NNa3[ANb4]Ab4GNb2#sp1 sp1 **GM2(NAc)/CT/Sda** |
| 161. | NNa3{Ab4[Fa3]GN}3b#sp1 **Sia3-TriLeX** |
| 162. | NNa3Ab#sp2 **Neu5Acα2,3Gal** |
| 163. | NNa3Ab3[6OSO3]ANa#sp2 **Neu5Acα3[6Su]-T** |
| 164. | NNa3Ab3[Fa4]GNb#sp2 **SLe a** |
| 165. | NNa3Ab3[NNa6]ANa#sp2 **Di-Sia-T** |
| 166. | NNa3Ab3ANa#sp2 **3-Sia-T** |
| 167. | NNa3Ab3GNb#spl **Neu5Acα3Type-1** |
| 168. | NNa3Ab3GNb#sp2 **Neu5Acα3Type-1** |
| 169. | NNa3Ab4[6OSO3]GNb#sp2**3'Sia[6Su]LacNAc** |
| 170. | NNa3Ab4[Fa3][6OSO3]GNb#sp2 **6Su-SLeX** |
| 171. | NNa3Ab4[Fa3]GNb#sp1 **SLeX** |
| 172. | NNa3Ab4[Fa3]GNb#sp2 **SLeX** |
| 173. | NNa3Ab4[Fa3]GNb3Ab#sp2 **SleX penta** |
| 174. | NNa3Ab4[Fa3]GNb3Ab4GNb#sp1 **SLeXLacNAc** |
| 175. | NNa3Ab4Gb#sp1 **3'Sialyllactose** |
| 176. | NNa3Ab4Gb#sp2 **3'Sialyllactose** |
| 177. | NNa3Ab4GNb#sp1 **3'SialyllacNAc** |
| 178. | NNa3Ab4GNb#sp2 **3'SialyllacNAc** |
| 179. | NNa3Ab4GNb3Ab4GNb#sp1 **3'SialylDiLacNAc** |
| 180. | NNa3Ab4GNb3Ab4GNb3Ab4GNb#sp1 **3'Sialyl-tri-LacNAc** |
| 181. | NNa3ANa#sp2 **Siaα3GalNAc** |
| 182. | NNa6Ab#sp2 **Siaα6Gal** |
| 183. | NNa6Ab4[6OSO3]]GNb#sp2 **6'Sial[6Su]LacNAc** |
| 184. | NNa6Ab4Gb#sp1 **6'Sia-lactose** |
| 185. | NNa6Ab4Gb#sp2 **6'Sia-lactose** |
| 186. | NNa6Ab4GNb#sp1 **6'Sia-LacNAc** |
| 187. | NNa6Ab4GNb#sp2 **6'Sia-LacNAc** |
| 188. | NNa6Ab4GNb3Ab4[Fa3]GNb3Ab4[Fa3]GNb#sp1 **6Sia-LacNAc-LeX-LeX** |
| 189. | NNa6Ab4GNb3Ab4GNb#sp1 **6SiaLacNAc-LacNAc** |
| 190. | NNa6ANa#sp2 **6SiaβGalNAc** |
| 191. | NNa8NNa3[ANb4]Ab4Gb#sp1 **GD2** |
| 192. | NNa8NNa3Ab4Gb#sp1 **GD3** |
| 193. | NNa8NNa8NNa3[ANb4]Ab4Gb#sp1 **GT2** |
| 194. | NNa8NNa8NNa3Ab4Gb#sp1 **GT3** |
| 195. | NNAa3[NNa6]ANa#sp2 **(Sia)2-Tn** |
| 196. | NNb#sp2 **Sia**β |
| 197. | NNb6Ab4GNb#sp2 **6'SiaβLacNAc** |
| 198. | NNb6ANa#sp2 **βSTn** |
| 199. | OS-11#sp2 **6'sialLacNAc-biantenary glycan** |
| 200. | Ra#sp2 **Rhamnose** |

Many of the abbreviations employed in the table are defined herein or at the website lectinity.com. The website at glycominds.com explains many of the linear abbreviations. In particular, the following abbreviations were used:
Sp1=OCH2CH2NH2;
Sp2=Sp3=OCH2CH2CH2NH2
A=Gal; AN=GalNAc; G=Glc; GN=GlcNAc;
F=Fucose; NN; Neu5Ac (sialic acid);
NJ=Neu5Gc (N-glycolylsialic acid); a=α; b=β;
Su=sulfo; T= Galβ3GalNAc (T-antigen);
Tn=GalNAc (Tn-antigen); KDN=5-OH-Sia

The glycans of the invention can have linkers, labels, linking moieties and/or other moieties attached to them. These linkers, labels, linking moieties and/or other moieties can be used to attach the glycans to a solid support, detect particular glycans in an assay, purify or otherwise manipulate the glycans. For example, the glycans of the invention can have amino moieties provided by attached alkylamine groups, amino acids, peptides, or proteins. In some embodiments, the glycans have alkylamine moieties such as -OCH₂cH₂NH₂ (called Sp1) or -OCH₂CH₂CH₂NH₂ (called Sp2 or Sp3) that have useful as linking moieties (the amine) and act as spacers or linkers.

### Arrays

Unique libraries of different glycans are attached to defined regions on the solid support of the array surface by any available procedure. In general, the arrays are made by obtaining a library of glycan molecules, attaching linking moieties to the glycans in the library, obtaining a solid support that has a surface derivatized to react with the specific linking moieties present on the glycans of the library and attaching the glycan molecules to the solid support by forming a covalent linkage between the linking moieties and the derivatized surface of the solid support.

The derivatization reagent can be attached to the solid substrate via carbon-carbon bonds using, or example, substrates having (poly)trifluorochloroethylene surfaces, or more preferably, by siloxane bonds (using, for example, glass or silicon oxide as the solid substrate). Siloxane bonds with the surface of the substrate are formed in one embodiment via reactions of derivatization reagents bearing trichlorosilyl or trialkoxysilyl groups.

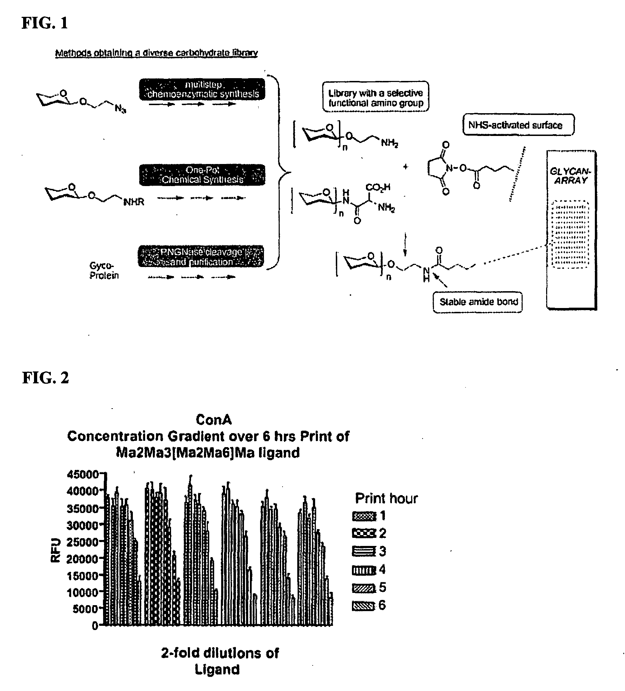

For example, a glycan library can be employed that has been modified to contain primary amino groups. For example, the glycans of the invention can have amino moieties provided by attached alkylamine groups, amino acids, peptides, or proteins. In some embodiments the glycans can have alkylamine groups such as the -OCH₂CH₂NH₂ (called Sp1) or -OCH₂CH₂CH₂NH₂ (called Sp2 or Sp3) groups attached that provide the primary amino group. The primary amino groups on the glycans can react with an N-hydroxy succinimide (NHS)-derivatized surface of the solid support. Such NHS-derivatized solid supports are commercially available. For example, NHS-activated glass slides are available from Accelr8 Technology Corporation, Denver, CO. After attachment of all the desired glycans, slides can further be incubated with ethanolamine buffer to deactivate remaining NHS functional groups on the solid support. The array can be used without any further modification of the surface. No blocking procedures to prevent unspecific binding are typically needed. FIG. 1 provides a schematic diagram of such a method for making arrays of glycan molecules.

Each type of glycan is contacted or printed onto to the solid support at a defined glycan probe location. A microarray gene printer can be used for applying the various glycans to defined glycan probe locations. For example, about 0.1 nL to about 10 nL, or about 0.5 nL of glycan solution can be applied per defined glycan probe location. Various concentrations of the glycan solutions can be contacted or printed onto the solid support. For example, a glycan solution of about 0.1 to about 1000 µM glycan or about 1.0 to about 500 µM glycan or about 10 to about 100 µM glycan can be employed. In general, it may be advisable to apply each concentration to a replicate of several (for example, three to six) defined glycan probe locations. Such replicates provide internal controls that confirm whether or not a binding reaction between a glycan and a test molecule is a real binding interaction.

### Analytical Methods

In another embodiment, the invention provides methods for screening test samples to identify whether the test sample can bind to a glycan. In further embodiments, the invention provides methods for identifying which glycan can bind to a test sample or a test molecule. The cleavable linkers of the invention are particularly well-suited for such screening and structural analysis procedures.

Any sample containing a molecule that is suspected of binding to a glycan can be tested. Thus, antibodies, bacterial proteins, cellular receptors, cell type specific antigens, enzymes, nucleic acids, viral proteins, and the like can be tested for binding to glycans. Moreover, the specific glycan structural features or types of glycans to which these molecules or substances bind can be identified.

The nucleic acids tested include DNA, mRNA, tRNA and ribosomal RNA as well as structural RNAs from any species.

Glycan identified by the methods of the invention can have utility for a multitude of purposes including as antigens, vaccines, enzyme inhibitors, ligands for receptors, inhibitors of receptors, and markers for the molecules to which they bind.

As illustrated herein viral, animal and human lectins as well as monoclonal antibody preparations were successfully tested for binding to glycans, and the specific glycan to which the lectin or antibody bound was identified.

Detection of binding can be direct, for example, by detection of a label directly attached to the test molecule. Alternatively, detection can be indirect, for example, by detecting a labeled secondary antibody or other labeled molecule that can bind to the test molecule. The bound label can be observed using any available detection method. For example, an array scanner can be employed to detect fluorescently labeled molecules that are bound to array. In experiments illustrated herein a ScanArray 5000 (GSI Lumonics, Watertown, MA) confocal scanner was used. The data from such an array scanner can be analyzed by methods available in the art, for example, by using ImaGene image analysis software (BioDiscovery Inc., E1 Segundo, CA).

### Useful Glycans Identified with the Invention

The invention also contemplates glycans identified by use of the cleavable linkers, arrays and methods of the invention. These glycans include antigenic glycans recognized by antibodies. For example, many neutralizing antibodies that recognize glycan epitopes on infectious agents and cancer cells can neutralize the infectivity and/or pathogenicity of those infectious agents and cancer cells. The arrays and methods of the invention can be used to precisely define the structure of such glycan epitopes. Because they bind to neutralizing antibodies with known beneficial properties those glycan epitopes can serve as immunogens in animals and can be formulated into immunogenic compositions useful for treating and preventing diseases, including infections and cancer.

Useful glycans of the invention also include non-antigenic glycans useful for blocking binding to an antibody, receptor or one biomolecule in a complex of biomolecules.

For example, the cell-surface glycosphingolipid Globo H is a member of a family of antigenic carbohydrates that are highly expressed on a range of cancer cell lines. Kannagi et al.(1983) J. Biol. Chem. 258, 8934-8942; Zhang et al. (1997) Chem. Biol. 4, 97-104; Dube, D. H. & Bertozzi, C. R. (2005) Nature Rev. Drug Discov. 4, 477-488. The Globo H epitope is targeted by the monoclonal antibody MBr1. Menard, et al. (1983) Cancer Res. 43, 1295-1300; Canevari, et al.(1983) Cancer Res. 43, 1301-1305; Bremer, et al.(1984) J. Biol. Chem. 259, 4773-4777. The epitopes responsible for binding to the MBr1 antibody have been identified and characterized using the cleavable arrays and methods of the invention. The Globo H antigen structures found to bind the monoclonal antibody MBr1 with greatest affinity were glycans **203a, 203b, 204a** and **204b**. Thus, any one of the following glycans, or a combination thereof, are useful glycans of the invention: 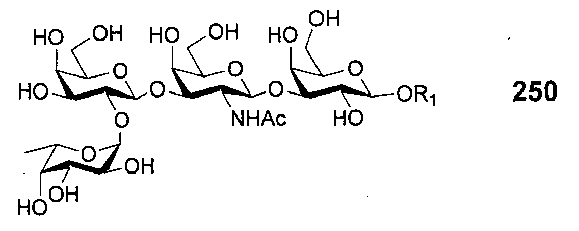 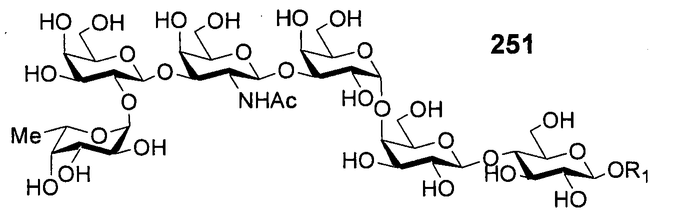 wherein: R₁ is hydrogen, a glycan or a linker. In some embodiments, the linker is or can be attached to a solid support.

Another example of a useful glycan of the invention is a mannose-containing glycan that can bind to anti-HIV 2G12 antibodies. According to the invention, such a mannose-containing glycan includes Manα1-2Man on a first (α1-3) arm of a glycan or on a (α1-6) third arm of a glycan, or a combination thereof. In some embodiments, the mannose-containing glycan may not have a second arm from a (α1-3) branch. In other embodiments, the mannose-containing glycan may have a second arm from a (α1-3) branch. In some embodiments, the mannose-containing glycans has any one of the following oligomannose glycans, or a combination thereof:

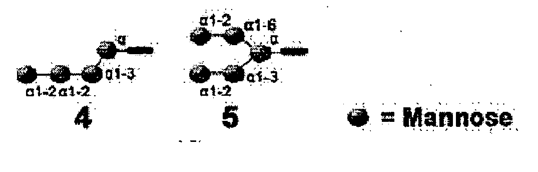

### Methods of Treating Disease

The invention also provides glycan compositions that can be used as immunogens for treating and preventing disease. Thus, for example, the compositions of the invention can be used to treat diseases such as cancer, bacterial infection, viral infection, inflammation, transplant rejection, autoimmune diseases and the like. In some embodiments, the glycans selected for inclusion in a composition of the invention are antigenic and can give rise to an immune response against a bacterial species, a viral species, cancer cell type and the like. In other embodiments, the glycans selected for inclusion in a composition of the invention are generally antigenic. However, in some embodiments, the glycans may bind or compete for binding sites on antibodies, receptors, and the like that contribute to the prognosis of a disease. Hence, for example, a non-antigenic glycan may be administered in order to prevent binding by a virus.

Such compositions include one or more glycans that are typically recognized by circulating antibodies associated with a disease, an infection or an immune condition. For example, to treat or prevent breast cancer, compositions are prepared that contain glycans that are typically recognized by circulating antibodies of subjects with metastatic breast cancer. Examples of glycans that can be included in compositions for treating and preventing breast cancer therefore include useful glycans identified with the cleavable linkers, arrays and methods of the invention.

In some embodiments, the type and amount of glycan is effective to provoke an anticancer cell immune response in a subject. In other embodiments, the type and amount of glycan is effective to provoke an anti-viral immune response in a subject.

The compositions of the invention may be administered directly into the subject, into an affected organ or systemically, or applied ex vivo to cells derived from the subject or from a cell line which is subsequently administered to the subject, or used in vitro to select a subpopulation from immune cells derived from the subject, which are then re-administered to the subject. The composition can be administered with an adjuvant or with immune-stimulating cytokines, such as interleukin-2. An example of an immune-stimulating adjuvant is Detox. The glycans may also be conjugated to a suitable carrier such as keyhole limpet hemocyanin (KLH) or mannan (see WO 95/18145 and Longenecker et al (1993) Ann. NY Acad. Sci. 690, 276-291). The glycans can be administered to the subject orally, intramuscularly or intradermally or subcutaneously.

In some embodiments, the compositions of the invention are administered in a manner that produces a humoral response. Thus, production of antibodies directed against the glycan(s) is one measure of whether a successful immune response has been achieved.

In other embodiments, the compositions of the invention are administered in a manner that produces a cellular immune response, resulting in tumor cell killing by NK cells or cytotoxic T cells (CTLs). Strategies of administration that activate T helper cells are particularly useful. As described above, it may also be useful to stimulate a humoral response. It may be useful to co-administer certain cytokines to promote such a response, for example interleukin-2, interleukin-12, interleukin-6, or interleukin-10.

It may also be useful to target the immune compositions to specific cell populations, for example, antigen presenting cells, either by the site of injection, by use delivery systems, or by selective purification of such a cell population from the subject and ex vivo administration of the glycan(s) to such antigen presenting cells. For example, dendritic cells may be sorted as described in Zhou et al (1995) Blood 86, 3295-3301; Roth et al (1996) Scand. J. Immunology 43, 646-651.

A further aspect of the invention therefore provides a vaccine effective against a disease comprising an effective amount of glycans that are bound by circulating antibodies of subjects with the disease.

### Dosages, Formulations and Routes of Administration

The compositions of the invention are administered to treat or prevent disease. In some embodiments, the compositions of the invention are administered so as to achieve an immune response against the glycans in the composition. In some embodiments, the compositions of the invention are administered so as to achieve a reduction in at least one symptom associated with a disease such as cancer, bacterial infection, viral infection, inflammation, transplant rejection, autoimmune diseases and the like.

To achieve the desired effect(s), the glycan or a combination thereof, may be administered as single or divided dosages, for example, of at least about 0.01 mg/kg to about 500 to 750 mg/kg, of at least about 0.01 mg/kg to about 300 to 500 mg/kg, at least about 0.1 mg/kg to about 100 to 300 mg/kg or at least about 1 mg/kg to about 50 to 100 mg/kg of body weight, although other dosages may provide beneficial results. The amount administered will vary depending on various factors including, but not limited to, what types of glycans are administered, the route of administration, the progression or lack of progression of the disease, the weight, the physical condition, the health, the age of the patient, whether prevention or treatment is to be achieved, and if the glycan is chemically modified. Such factors can be readily determined by the clinician employing animal models or other test systems that are available in the art.

Administration of the therapeutic agents (glycans) in accordance with the present invention may be in a single dose, in multiple doses, in a continuous or intermittent manner, depending, for example, upon the recipient's physiological condition, whether the purpose of the administration is therapeutic or prophylactic, and other factors known to skilled practitioners. The administration of the glycans or combinations thereof may be essentially continuous over a pre-selected period of time or may be in a series of spaced doses. Both local and systemic administration is contemplated.

To prepare the composition, the glycans are synthesized or otherwise obtained, and purified as necessary or desired. These therapeutic agents can then be lyophilized or stabilized, their concentrations can be adjusted to an appropriate amount, and the therapeutic agents can optionally be combined with other agents. The absolute weight of a given glycan, binding entity, antibody or combination thereof that is included in a unit dose can vary widely. For example, about 0.01 to about 2 g, or about 0.1 to about 500 mg, of at least one glycan, binding entity, or antibody specific for a particular glycan can be administered. Alternatively, the unit dosage can vary from about 0.01 g to about 50 g, from about 0.01 g to about 35 g, from about 0.1 g to about 25 g, from about 0.5 g to about 12 g, from about 0.5 g to about 8 g, from about 0.5 g to about 4 g, or from about 0.5 g to about 2 g.

Daily doses of the glycan(s), binding entities, antibodies or combinations thereof can vary as well. Such daily doses can range, for example, from about 0.1 g/day to about 50 g/day, from about 0.1 g/day to about 25 g/day, from about 0.1 g/day to about 12 g/day, from about 0.5 g/day to about 8 g/day, from about 0.5 g/day to about 4 g/day, and from about 0.5 g/day to about 2 g/day.

Thus, one or more suitable unit dosage forms comprising the therapeutic agents of the invention can be administered by a variety of routes including oral, parenteral (including subcutaneous, intravenous, intramuscular and intraperitoneal), rectal, dermal, transdermal, intrathoracic, intrapulmonary and intranasal (respiratory) routes. The therapeutic agents may also be formulated for sustained release (for example, using microencapsulation, see WO 94/ 07529, and U.S. Patent No.4,962,091). The formulations may, where appropriate, be conveniently presented in discrete unit dosage forms and may be prepared by any of the methods well known to the pharmaceutical arts. Such methods may include the step of mixing the therapeutic agent with liquid carriers, solid matrices, semi-solid carriers, finely divided solid carriers or combinations thereof, and then, if necessary, introducing or shaping the product into the desired delivery system.

When the therapeutic agents of the invention are prepared for oral administration, they are generally combined with a pharmaceutically acceptable carrier, diluent or excipient to form a pharmaceutical formulation, or unit dosage form. For oral administration, the therapeutic agents may be present as a powder, a granular formulation, a solution, a suspension, an emulsion or in a natural or synthetic polymer or resin for ingestion of the active ingredients from a chewing gum. The therapeutic agents may also be presented as a bolus, electuary or paste. Orally administered therapeutic agents of the invention can also be formulated for sustained release. For example, the therapeutic agents can be coated, micro-encapsulated, or otherwise placed within a sustained delivery device. The total active ingredients in such formulations comprise from 0.1 to 99.9% by weight of the formulation.

By "pharmaceutically acceptable" it is meant a carrier, diluent, excipient, and/or salt that is compatible with the other ingredients of the formulation, and not deleterious to the recipient thereof.

Pharmaceutical formulations containing the therapeutic agents of the invention can be prepared by procedures known in the art using well-known and readily available ingredients. For example, the therapeutic agent can be formulated with common excipients, diluents, or carriers, and formed into tablets, capsules, solutions, suspensions, powders, aerosols and the like. Examples of excipients, diluents, and carriers that are suitable for such formulations include buffers, as well as fillers and extenders such as starch, cellulose, sugars, mannitol, and silicic derivatives. Binding agents can also be included such as carboxymethyl cellulose, hydroxymethylcellulose, hydroxypropyl methylcellulose and other cellulose derivatives, alginates, gelatin, and polyvinyl-pyrrolidone. Moisturizing agents can be included such as glycerol, disintegrating agents such as calcium carbonate and sodium bicarbonate. Agents for retarding dissolution can also be included such as paraffin. Resorption accelerators such as quaternary ammonium compounds can also be included. Surface active agents such as cetyl alcohol and glycerol monostearate can be included. Adsorptive carriers such as kaolin and bentonite can be added. Lubricants such as talc, calcium and magnesium stearate, and solid polyethylene glycols can also be included. Preservatives may also be added. The compositions of the invention can also contain thickening agents such as cellulose and/or cellulose derivatives. They may also contain gums such as xanthan, guar or carbo gum or gum arabic, or alternatively polyethylene glycols, bentones and montmorillonites, and the like.

For example, tablets or caplets containing the therapeutic agents of the invention can include buffering agents such as calcium carbonate, magnesium oxide and magnesium carbonate. Caplets and tablets can also include inactive ingredients such as cellulose, pre-gelatinized starch, silicon dioxide, hydroxy propyl methyl cellulose, magnesium stearate, microcrystalline cellulose, starch, talc, titanium dioxide, benzoic acid, citric acid, corn starch, mineral oil, polypropylene glycol, sodium phosphate, zinc stearate, and the like. Hard or soft gelatin capsules containing at least one therapeutic agent of the invention can contain inactive ingredients such as gelatin, microcrystalline cellulose, sodium lauryl sulfate, starch, talc, and titanium dioxide, and the like, as well as liquid vehicles such as polyethylene glycols (PEGs) and vegetable oil. Moreover, enteric-coated caplets or tablets containing one or more of the therapeutic agents of the invention are designed to resist disintegration in the stomach and dissolve in the more neutral to alkaline environment of the duodenum.

The therapeutic agents of the invention can also be formulated as elixirs or solutions for convenient oral administration or as solutions appropriate for parenteral administration, for instance by intramuscular, subcutaneous, intraperitoneal or intravenous routes. The pharmaceutical formulations of the therapeutic agents of the invention can also take the form of an aqueous or anhydrous solution or dispersion, or alternatively the form of an emulsion or suspension or salve.

Thus, the therapeutic agents may be formulated for parenteral administration (e.g., by injection, for example, bolus injection or continuous infusion) and may be presented in unit dose form in ampoules, pre-filled syringes, small volume infusion containers or in multi-dose containers. As noted above, preservatives can be added to help maintain the shelve life of the dosage form. The active agents and other ingredients may form suspensions, solutions, or emulsions in oily or aqueous vehicles, and may contain formulatory agents such as suspending, stabilizing and/or dispersing agents. Alternatively, the therapeutic agents and other ingredients may be in powder form, obtained by aseptic isolation of sterile solid or by lyophilization from solution, for constitution with a suitable vehicle, e.g., sterile, pyrogen-free water, before use.

These formulations can contain pharmaceutically acceptable carriers, vehicles and adjuvants that are well known in the art. It is possible, for example, to prepare solutions using one or more organic solvent(s) that is/are acceptable from the physiological standpoint, chosen, in addition to water, from solvents such as acetone, ethanol, isopropyl alcohol, glycol ethers such as the products sold under the name "Dowanol," polyglycols and polyethylene glycols, C₁-C₄ alkyl esters of short-chain acids, ethyl or isopropyl lactate, fatty acid triglycerides such as the products marketed under the name "Miglyol," isopropyl myristate, animal, mineral and vegetable oils and polysiloxanes.

It is possible to add, if necessary, an adjuvant chosen from antioxidants, surfactants, other preservatives, film-forming, keratolytic or comedolytic agents, perfumes, flavorings and colorings. Antioxidants such as t-butylhydroquinone, butylated hydroxyariisole, butylated hydroxytoluene and α-tocopherol and its derivatives can be added.

Additionally, the therapeutic agents are well suited to formulation as sustained release dosage forms and the like. The formulations can be so constituted that they release the active agent, for example, in a particular part of the vascular system or respiratory tract, possibly over a period of time. Coatings, envelopes, and protective matrices may be made, for example, from polymeric substances, such as polylactide-glycolates, liposomes, microemulsions, microparticles, nanoparticles, or waxes. These coatings, envelopes, and protective matrices are useful to coat indwelling devices, e.g., stents, catheters, peritoneal dialysis tubing, draining devices and the like.

For topical administration, the therapeutic agents may be formulated as is known in the art for direct application to a target area. Forms chiefly conditioned for topical application take the form, for example, of creams, milks, gels, dispersion or microemulsions, lotions thickened to a greater or lesser extent, impregnated pads, ointments or sticks, aerosol formulations (e.g., sprays or foams), soaps, detergents, lotions or cakes of soap. Other conventional forms for this purpose include wound dressings, coated bandages or other polymer coverings, ointments, creams, lotions, pastes, jellies, sprays, and aerosols. Thus, the therapeutic agents of the invention can be delivered via patches or bandages for dermal administration. Alternatively, the therapeutic agents can be formulated to be part of an adhesive polymer, such as polyacrylate or acrylate/vinyl acetate copolymer. For long-term applications it might be desirable to use microporous and/or breathable backing laminates, so hydration or maceration of the skin can be minimized. The backing layer can be any appropriate thickness that will provide the desired protective and support functions. A suitable thickness will generally be from about 10 to about 200 microns.

Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents. The active ingredients can also be delivered via iontophoresis, e.g., as disclosed in U.S. Patent Nos. 4,140,122; 4,383,529; or 4,051,842. The percent by weight of a therapeutic agent of the invention present in a topical formulation will depend on various factors, but generally will be from 0.01% to 95% of the total weight of the formulation, and typically 0.1-85% by weight.

Drops, such as eye drops or nose drops, may be formulated with one or more of the therapeutic agents in an aqueous or non-aqueous base also comprising one or more dispersing agents, solubilizing agents or suspending agents. Liquid sprays are conveniently delivered from pressurized packs. Drops can be delivered via a simple eye dropper-capped bottle, or via a plastic bottle adapted to deliver liquid contents dropwise, via a specially shaped closure.

The therapeutic agent may further be formulated for topical administration in the mouth or throat. For example, the active ingredients may be formulated as a lozenge further comprising a flavored base, usually sucrose and acacia or tragacanth; pastilles comprising the composition in an inert base such as gelatin and glycerin or sucrose and acacia; and mouthwashes comprising the composition of the present invention in a suitable liquid carrier.

The pharmaceutical formulations of the present invention may include, as optional ingredients, pharmaceutically acceptable carriers, diluents, solubilizing or emulsifying agents, and salts of the type that are available in the art. Examples of such substances include normal saline solutions such as physiologically buffered saline solutions and water. Specific non-limiting examples of the carriers and/or diluents that are useful in the pharmaceutical formulations of the present invention include water and physiologically acceptable buffered saline solutions such as phosphate buffered saline solutions pH 7.0-8.0.

The active ingredients of the invention can also be administered to the respiratory tract. Thus, the present invention also provides aerosol pharmaceutical formulations and dosage forms for use in the methods of the invention.

In general, such dosage forms comprise an amount of at least one of the agents of the invention effective to treat or prevent the clinical symptoms of a disease. Diseases contemplated by the invention include, for example, cancer, bacterial infection, viral infection, inflammation, transplant rejection, autoimmune diseases and the like. Any statistically significant attenuation of one or more symptoms of a disease is considered to be a treatment of the disease.

Alternatively, for administration by inhalation or insufflation, the composition may take the form of a dry powder, for example, a powder mix of the therapeutic agent and a suitable powder base such as lactose or starch. The powder composition may be presented in unit dosage form in, for example, capsules or cartridges, or, e.g., gelatin or blister packs from which the powder may be administered with the aid of an inhalator, insufflator, or a metered-dose inhaler (see, for example, the pressurized metered dose inhaler (MDI) and the dry powder inhaler disclosed in Newman, S. P. in Aerosols and the Lung, Clarke, S. W. and Davia, D. eds., pp. 197-224, Butterworths, London, England, 1984).

Therapeutic agents of the present invention can also be administered in an aqueous solution when administered in an aerosol or inhaled form. Thus, other aerosol pharmaceutical formulations may comprise, for example, a physiologically acceptable buffered saline solution containing between about 0.1 mg/ml and about 100 mg/ml of one or more of the therapeutic agents of the present invention specific for the indication or disease to be treated. Dry aerosol in the form of finely divided solid therapeutic agent that are not dissolved or suspended in a liquid are also useful in the practice of the present invention. Therapeutic agents of the present invention may be formulated as dusting powders and comprise finely divided particles having an average particle size of between about 1 and 5 µm, alternatively between 2 and 3 µm. Finely divided particles may be prepared by pulverization and screen filtration using techniques well known in the art. The particles may be administered by inhaling a predetermined quantity of the finely divided material, which can be in the form of a powder. It will be appreciated that the unit content of active ingredient or ingredients contained in an individual aerosol dose of each dosage form need not in itself constitute an effective amount for treating the particular immune response, vascular condition or disease since the necessary effective amount can be reached by administration of a plurality of dosage units. Moreover, the effective amount may be achieved using less than the dose in the dosage form, either individually, or in a series of administrations.

For administration to the upper (nasal) or lower respiratory tract by inhalation, the therapeutic agents of the invention are conveniently delivered from a nebulizer or a pressurized pack or other convenient means of delivering an aerosol spray. Pressurized packs may comprise a suitable propellant such as dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. Nebulizers include, but are not limited to, those described in U.S. Patent Nos. 4,624,251; 3,703,173; 3,561,444; and 4,635,627. Aerosol delivery systems of the type disclosed herein are available from numerous commercial sources including Fisons Corporation (Bedford, Mass.), Schering Corp. (Kenilworth, NJ) and American Pharmoseal Co., (Valencia, CA). For intra-nasal administration, the therapeutic agent may also be administered via nose drops, a liquid spray, such as via a plastic bottle atomizer or metered-dose inhaler. Typical of atomizers are the Mistometer (Wintrop) and the Medihaler (Riker).

Furthermore, the active ingredients may also be used in combination with other therapeutic agents, for example, pain relievers, anti-inflammatory agents, anti-viral agents, anti-cancer agents and the like, whether for the conditions described or some other condition.

### Kits

The present invention further pertains to a packaged pharmaceutical composition such as a kit or other container for detecting, controlling, preventing or treating a disease. The kits of the invention can be designed for detecting, controlling, preventing or treating diseases such as cancer, bacterial infection, viral infection, inflammation, transplant rejection, autoimmune diseases and the like. In one embodiment, the kit or container holds an array or library of glycans for detecting disease and instructions for using the array or library of glycans for detecting the disease. The array includes at least one glycan that is bound by antibodies present in serum samples of persons with the disease. The array can include cleavable linkers of the invention.

In another embodiment, the kit or container holds a therapeutically effective amount of a pharmaceutical composition for treating, preventing or controlling a disease and instructions for using the pharmaceutical composition for control of the disease. The pharmaceutical composition includes at least one glycan of the present invention, in a therapeutically effective amount such that the disease is controlled, prevented or treated.

The kits of the invention can also comprise containers with tools useful for administering the compositions of the invention. Such tools include syringes, swabs, catheters, antiseptic solutions and the like.

The following examples are for illustration of certain aspects of the invention and is not intended to be limiting thereof.

### EXAMPLE 1: Enzymatic Synthesis of Glycans

The inventors have previously cloned and characterized the bacterial *N*. *meningitidis* enzymes β4GalT-GalE and β3GlcNAcT. Blixt, O.;Brown, J.;Schur, M.;Wakarchuk, W. and Paulson, J. C., J. Org. Chem. 2001, 66, 2442-2448; Blixt, O.;van Die, I.;Norberg, T. and van den Eijnden, D. H., Glycobiol. 1999, 9, 1061-1071. β4GalT-GalE is a fusion protein constructed from β4GalT and the uridine-5'-diphospho-galactose-4'-epimerase (GalE) for *in situ* conversion of inexpensive UDP-glucose to UDP-galactose providing a cost efficient strategy.

Both enzymes, β4GalT-GalE and β3GlcNAcT, were over expressed in *E*. *coli* AD202 in a large-scale fermentor (100 L). Bacteria were cultured in 2YT medium and induced with *iso*-propyl-thiogalactopyranoside (IPTG) to ultimately produce 8-10 g of bacterial cell paste / L cell media. The enzymes were then released from the cells by a microfluidizer and were solubilized in Tris buffer (25 mM, pH 7.5) containing manganese chloride (10 mM) and Triton X (0.25%) to reach enzymatic activities of about 50 U/L and 115 U/L of cell culture β4GalT-GalE and β3GlcNAcT, respectively.

Specificity studies of the β3GlcNAcT (Table 4) revealed that lactose (**4**) is the better acceptor substrate (100%) while the enzyme shows just about 7-8% activity with N-acetyllactosamine (**6**). The structures of these disaccharides are provided below.

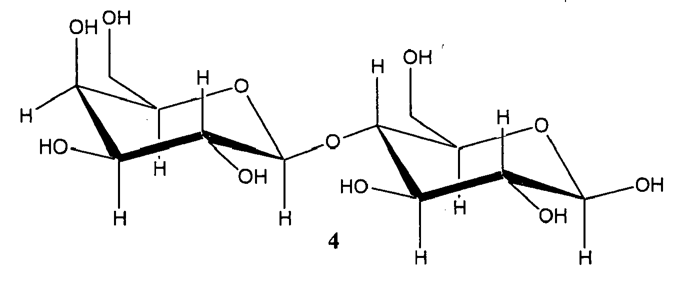

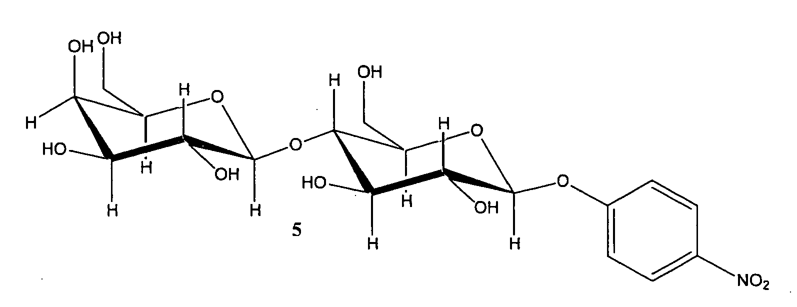

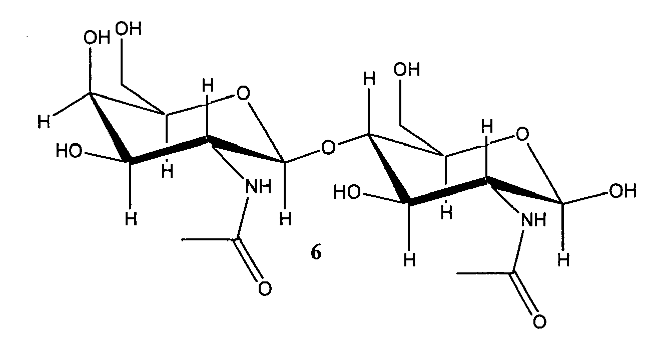

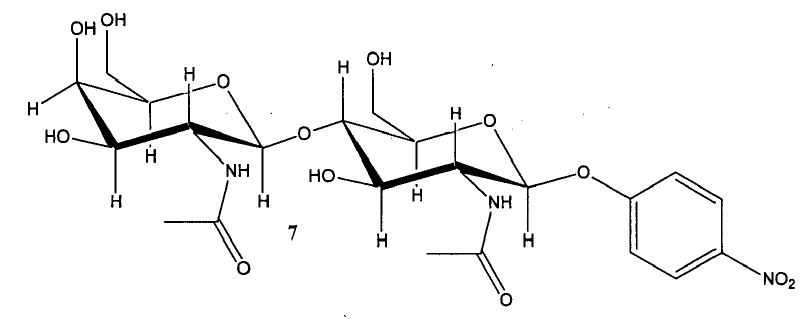

Adding the hydrophobic para-nitrophenyl ring as an aglycon to the reducing end of the acceptors enhanced the activity of the enzyme up to 10 fold (compare **4** with **5** and **6** with **7**). The increase in the enzyme activity by adding a hydrophobic aglycon to the acceptor sugar, though to the lesser extent, has also been shown for β4GalT (compare **12** with **13,14**)**.** The relaxed substrate specificity of these enzymes makes them very useful for preparative synthesis of various carbohydrate structures, including poly-*N*-acetyllactosamines.

**Table 4. Selected β4GalT-GalE and β3GlcNAcT Specificity Data**

| *Acceptor* | | *Relative enzyme activity (%)* |
|---|---|---|
| | | β(1-3)GlcNAcT-activity^{#} |
| **1** | Gal | 5 |
| **2** | Galα-OpNP | 102 |
| **3** | Galβ-OpNP | 16 |
| **4** | Galβ(1-4)Glc | **100** |
| **5** | Galβ(1-4)Glcβ-OpNP | 945 |
| **6** | Galβ(1-4)GlcNAc | 7 |
| **7** | Galβ(1-4)GlcNAcβ-OpNP | 74 |
| **8** | Galβ(1-3)GlcNAc | 5 |
| | | β(1-4)GalT-GalE-activity* |
| **9** | Glc | 80 |
| **10** | Glcβ-OpNP | 60 |
| **11** | GlcNH₂ | 30 |
| **12** | GlcNAc | **100** |
| **13** | GlcNAcβ-OpNP | 120 |
| **14** | GlcNAcβ-Osp₁ | 360 |
| **15** | GlcNAllocβ-sp₂ | 550 |

| | | |
|---|---|---|
| Abbreviations: pNP, para-nitrophenyl; sp₁, 2-azidoethyl; sp₂, 5-azido-3-oxapentyl, Alloc, allyloxycarbonyl | | |

Poly-*N*-acetyllactosamine is a unique carbohydrate structure composed of *N*-acetyllactosamine repeats that provides the backbone structure for additional modifications, such as sialylation and/or fucosylation. These extended oligosaccharides have been shown to be involved in various biological functions by interacting as a specific ligand to selectins or galectins. Ujita, M.;McAuliffe, J.;Hindsgaul, O.;Sasaki, K.;Fukuda, M. N. and Fukuda, M., J Biol. Chem. 1999, 274, 16717-16726; Appelmelk, B. J.;Shiberu, B.;Trinks, C.;Tapsi, N.;Zheng, P. Y.;Verboom, T.;Maaskant, J.;Hokke, C. H.;Schiphorst, W. E. C. M.;Blanchard, D.;SimoonsSmit, I. M.;vandenEijnden, D. H. and Vandenbroucke Grauls, C. M. J. E., Infect. Immun. 1998, 66, 70-76; Leppaenen, A.;Penttilae, L.;Renkonen, O.;McEver, R. P. and Cummings, R. D., J Biol. Chem. 2002, 277, 39749-39759; Renkonen, O., Cell. Mol Life Sci. 2000, 57, 1423-1439; Baldus, S. E.;Zirbes, T. K.;Weingarten, M.;Fromm, S.;Glossmann, J.;Hanisch, F. G.;Monig, S. , P.;Schroder, W.;Flucke, U.;Thiele, J.;Holscher, A. H. and Dienes, H. P., Tumor Biology. 2000, 21, 25 8-266; Cho, M. and Cummings, R. D., TIGG.. 1997, 9, 47-56,171-178.

Based on the specificity data in Table 4, enzymatic synthesis of galactosides and polylactosamines can be performed in multi-gram quantities. This method employed various fucosyltransferases (FUTs). Several fucosyltransferases (FUTs) have been characterized in terms of substrate specificities and biological functions in different laboratories. Murray, B. W.;Takayama, S.;Schultz, J. and Wong, C. H., Biochem. 1996, 35, 11183-11195; Weston, B. W.;Nair, R. P.;Larsen, R. D. and Lowe, J. B., J. Biol. Chem. 1992, 267, 4152-4160; Kimura, H.;Shinya, N.;Nishihara, S.;Kaneko, M.;Irimura, T. and Narimatsu, H., Biochem. Biophys. Res. Comm. 1997, 237, 131-137; Chandrasekaran, E. V.;Jain, R. K.;Larsen, R. D.;Wlasichuk, K. and Matta, K. L., Biochem. 1996, 35, 8914-8924*;* Devries, T.;Vandeneijnden, D. H.;Schultz, J. and Oneill, R., FEBS Lett. 1993, 330, 243-248*;* Devries, T. and van den Eijnden, D. H., Biochem. 1994, 33, 9937-9944

The available specificity data in combination with large scale production of recombinant FUTs made it possible to synthesize various precious fucosides in multigram quantities. Scheme I illustrates the general procedure employed for elongating the poly-LacNAc backbone and selected fucosylated structures using different FUTs and GDP-fucose.

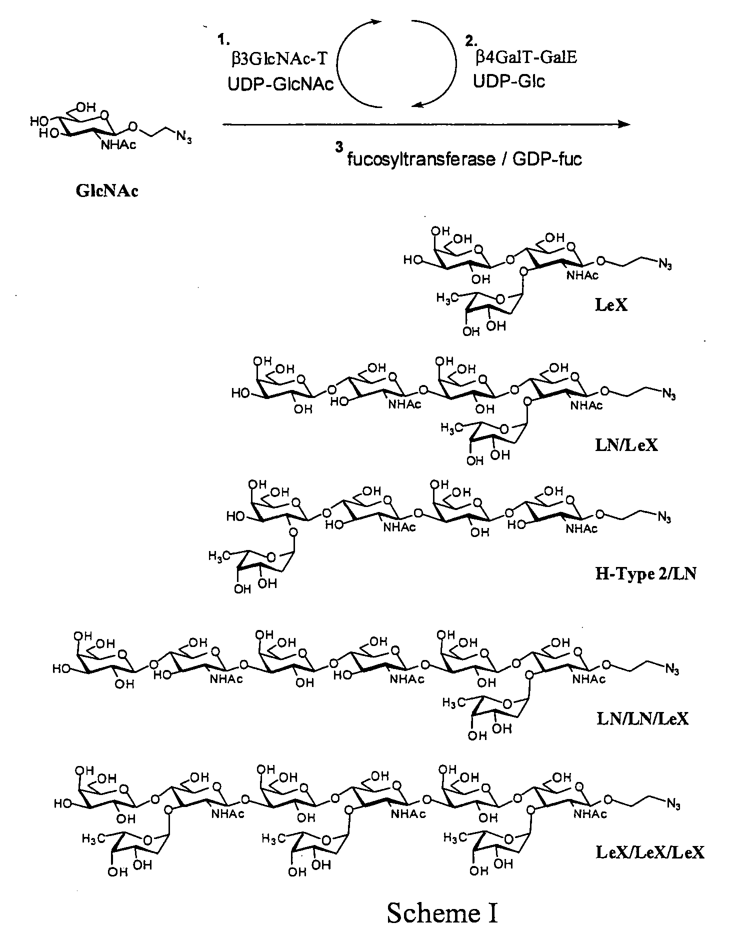

A systematic gram-scale synthesis of different fucosylated lactosamine derivatives was initiated using the Scheme I and the following recombinant fucosyltransferases, FUT-II, FUT-III, FUT-IV, FUT-V, and FUT-VI. All the above fucosyltransferases, except for FUT-V, were produced in the insect cell expression system and either partially purified on a GDP-sepharose affinity column or concentrated in a Tangential Flow Filtrator (TFF-MWCO 10k) as a crude enzyme mixture. The FUT-V enzyme was expressed in *A. niger* as described in Murray, B. W.;Takayama, S.;Schultz, J. and Wong, C. H., Biochem. 1996, 35, 11183-11195.

The yields for different stages of production of the fucosylated lactosamine derivatives were 75-90% for LeX (2 enzymatic steps), 45-50% for dimeric LacNAc structures (4 enzymatic steps) and 30-35% for trimeric lacNAc structures (6 enzymatic steps).

### EXAMPLE 2: Synthesis of sialic-acid-containing oligosaccharides

Sialic acid is a generic designation used for 2-keto-3-deoxy-nonulosonic acids. The most commonly occurring derivatives of this series of monosaccharides are those derived from *N*-acetylneuraminic acid (Neu5Ac), *N-*glycolylneuraminic acid (Neu5Gc) and the non-aminated 3-deoxy-D-*glycero*-D-*galacto*-2-nonulosonic acid (KDN). Sialic-acid-containing oligosaccharides are an important category of carbohydrates that are involved in different biological regulations and functions. Sialic acids are shown to be involved in adsorption of toxins/viruses, and diverse cellular communications through interactions with carbohydrate binding proteins (CBPs). Selectins and Siglecs (sialic acid-binding immunoglobulin-superfamily lectiris) are among those well-characterized CBPs that function biologically through sialic acid interactions.

Synthesis of oligosaccharides containing sialic acids is not trivial. Unfortunately, the chemical approaches have several hampering factors in common. For example, stereo selective glycosylation with sialic acid generally gives an isomeric product, and as a result, purification problems and lower yields. Its complicated nature, also require extensive protecting group manipulations and careful design of both acceptor and donor substrates and substantial amounts of efforts are needed to prepare these building blocks.

For a fast and efficient way to sialylate carbohydrate structures, the method of choice is through catalysis by sialyltransferases. Enzymatic sialylation generating Neu5 Ac-containing oligosaccharides is way to generate sialylate carbohydrates for both analytical and preparative purposes. Koeller, K. M. and Wong, C.-H., Nature 2001, 409, 232-240; Gilbert, M.;Bayer, R.;Cunningham, A.-M.;DeFrees, S.;Gao, Y.;Watson, D. C.;Young, N. M. and Wakarchuk, W. W., Nature Biotechnol. 1998, 16, 769-772; Ichikawa, Y.;Look, G. C. and Wong, C. H., Anal. Biochem. 1992, 202, 215-238. However, efficient methods for preparation of oligosaccharides having the Neu5Gc or KDN structures have not previously been explored to the same extent because of the scarcity of these sialoside derivatives.

A simple way to obtain different sialoside derivatives was devised using a modification of a method, originally developed by Wong and co-workers. Crocker, P. R., Curr. Opin. Struct. Biol. 2002, 12, 609-615. This method employed recombinant sialyltransferases along with a commercial Neu5Ac aldolase, ST3-CMP-Neu5Ac synthetase. Gilbert, M.;Bayer, R.;Cunningham, A.-M.;DeFrees, S.;Gao, Y.;Watson, D. C.;Young, N. M. and Wakarchuk, W. W., Nature Biotechnol. 1998, 16, 769-772.

The preferred route to generate Neu5Ac-oligosaccharides was to use a one-pot procedure described in Scheme II (**B** and **C**).

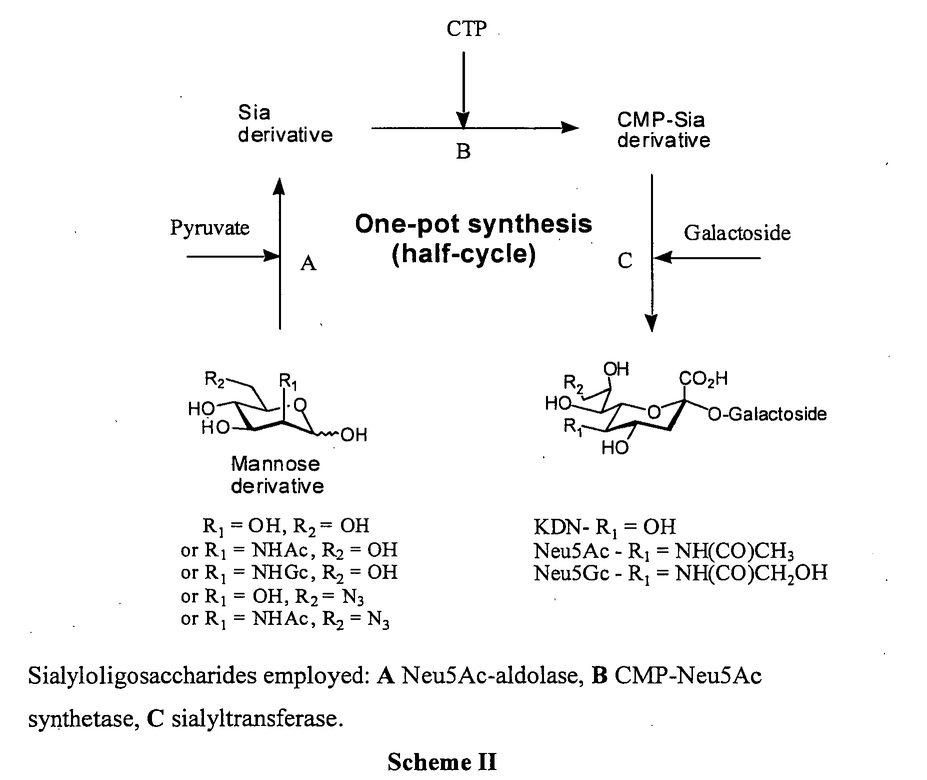

Briefly, ST3-CMP-Neu5Ac synthetase catalyzed the formation of CMP-Neu5Ac quantitatively from 1 equivalent of Neu5Ac and 1 equivalent of CTP. After removal of the fusion protein by membrane filtration (MW cut-off 10k) a selected galactoside and a recombinant sialyltransferase as described in Table 5 was introduced to produce the desired Neu5Ac-sialoside.

**Table 5: Recombinant Sialyltransferases Produced for Synthesis**

| *Sialyltransferase* | *Source of Production* | *Produced Activity** |
|---|---|---|
| hST6Gal-I | Baculovirus (19) | 20 |
| pST3Gal-I | Baculovirus (45) | 20 |
| rST3Gal-III | *A. Niger* ^{#} | 50 |
| chST6Gal-I | Baculovirus (46) | 10 |
| ST3Gal-Fusion | *E. coli* (42) | 6000 |
| ST8 (Cst-II) | *E. coli* (70) | 140 |

| | | |
|---|---|---|
| *Units /L cell culture | | |

This synthetic scheme produced multi-gram quantities of product typically with a yield of 70-90% recovery of sialylated products.

To synthesize Neu5Gc and KDN derivatives the one-pot system would include another enzymatic reaction in addition to routes **B** and **C** (Scheme II). In this respect, mannose derivatives, pyruvate (3 eqv.) and commercial microorganism Neu5Ac aldolase (Toyobo) were introduced into the one-pot half-cycle (Scheme II, A). The enzymes in Table 5 were able to generate various N- and O-linked oligosaccharides with α(2-3)-, α(2-6)- or α(2-8)-linked sialic acid derivatives of Neu5Gc, KDN and some of the 9-azido-9deoxy-Neu5Ac-analogs in acceptable yields (45-90%). O-linked sialyl-oligosaccharides are another class of desired compounds for the biomedical community. These structures are frequently found in various cancer tissues and lymphoma and are highly expressed in many types of human malignancies including colon, breast, pancreas, ovary, stomach, and lung adenocarcinomas. Dabelsteen, E., J. Pathol. 1996, 179, 358-369; Itzkowitz, S. H.;Yuan, M.;Montgomery, C. K.;Kjeldsen, T.;Takahashi, H. K. and Bigbee, W. L., Cancer Res. 1989, 49, 197-204.

The inventors have previously reported the cloning, expression, and characterization of chicken ST6GalNAc-I and its use in preparative synthesis of the *O*-linked sialoside antigens, STn-, α(2-6)SiaT-, α(2-3)SiaT- and Di-SiaT-antigen. Blixt, O.;Allin, K.;Pereira, L.;Datta, A. and Paulson, J. C., J. Am. Chem. Soc. 2002, 124, 5739-5746. Briefly, the recombinant enzyme was expressed in insect cells and purified by CDP-sepharose affinity chromatography to generate approximately 10 U/L of cell culture. The enzymatic activity was evaluated on a set of small acceptor molecules. (Table 6), and it was found that an absolute requirement for enzymatic activity is that the anomeric position on GalNAc is α-linked to threonine.

**Table 6. chST6GalNAc-I Activity of α-D-Galacto Derivatives**

| | | | | | | | |
|---|---|---|---|---|---|---|---|
| 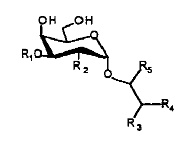 | | | | | | | |

| *Compound* | *R₁* | *R₂* | *R₃* | *R₄* | *R₅* | *cpm* | *nmol*/*mg x min⁻¹* |
|---|---|---|---|---|---|---|---|
| **D-GalNAc** | H | NHAc | | | | 0 | 0.00 |
| **1** | H | NHAc | N₃ | H | H | 65 | 0.06 |
| **2** | H | NHAc | NHAc | H | H | 121 | 0.11 |
| **3c** | H | NHAc | NHAc | COOCH₃ | CH₃ | 9133 | 8.60 |
| **4** | H | N₃ | NHAc | COOCH₃ | CH₃ | 3043 | 2.90 |
| **5** | H | NH₂ | NHAc | COOCH₃ | CH₃ | 1421 | 1.30 |
| **6** | H | NHAc | NHF_{moc} | COOCH₃ | CH₃ | 13277 | 12.50* |
| **7c** | Galβ1,3 | NHAc | NHAc | COOCH₃ | CH₃ | 12760 | 12.00 |

| | | | | | | | |
|---|---|---|---|---|---|---|---|
| NOTE : *Product was isolated by using Sep-Pak (C18)cartridges as described in Palcic, M. M ; Heerze, L.D. ; Pierce, M. and Hindsgaul, O., *Glycoconj. J.* **1988**, *5*, 49-63. | | | | | | | |

Thus, *O*-linked sialosides terminating with a protected threonine could successfully be synthesized on gram-scale reactions using Scheme III. To be able to attach these compounds to other functional groups, the *N*-acetyl protecting group on threonine could be substituted with a removable 9-fluorenyl (F-moc) derivative before enzymatic extension with chST6GalNAc-I. Blixt, O.;Collins, B. E.;Van Den Nieuwenhof, I. M.;Crocker, P. R. and Paulson, J. C., (2003 J. Biol. Chem. 15: 278). As seen in Table 6, the enzyme was not sensitive to bulky groups at this position (compound **6**).

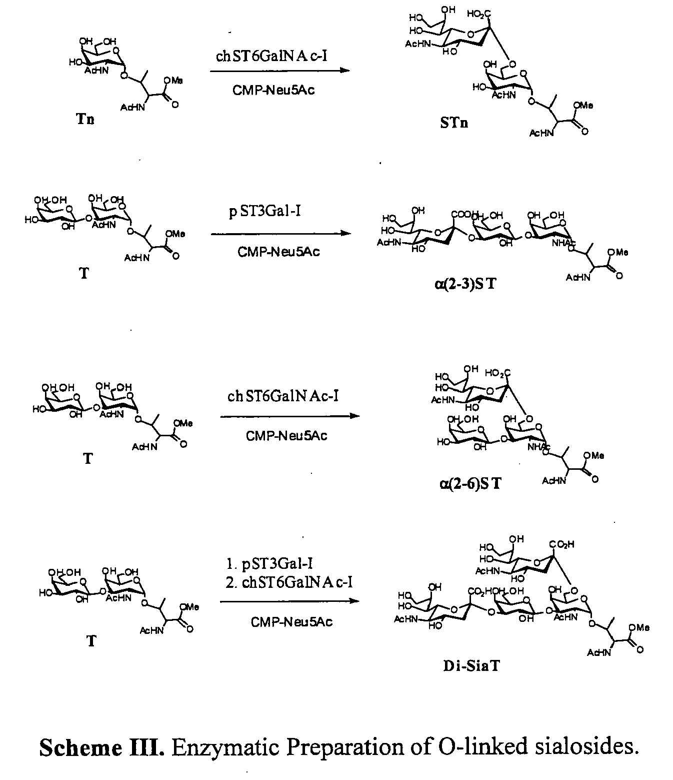

### EXAMPLE 3: Synthesis of Ganglioside Mimics

Gangliosides are glycolipids that comprise a structurally diverse set of sialylated molecules. They are attached and enriched in nervous tissues and they have been found to act as receptors for growth factors, toxins and viruses and to facilitate the attachment of human melanoma and neuroblastoma cells. Kiso, M., Nippon Nogei Kagaku Kaishi. 2002, 76, 1158-1167; Gagnon, M. and Saragovi, H. U., Expert Opinion on Therapeutic Patents. 2002, 12, 1215-1223; Svennerholm, L., Adv. Gen. 2001, 44, 33-41; Schnaar, R. L., Carbohydr. Chem. Biol. 2000, 4, 1013-1027; Ravindranath, M. H.;Gonzales, A. M.;Nishimoto, K.;Tam, W.-Y.;Soh, D. and Morton, D. L., Ind. J Exp. Biol. 2000, 38, 301-312; Rampersaud, A. A.;Oblinger, J. L.;Ponnappan, R. K.;Burry, R. W. and Yates, A. J., Biochem. Soc. Trans.. 1999, 27, 415-422; Nohara, K., Seikagaku. 1999, 71, 337-341.

Despite the importance of these sialylated ganglioside structures, methods for their efficient preparation have been limiting. The introduction of sialic acid to a glycolipid core structure have shown to be a daunting task, needed complicated engineering with well executed synthetic strategies.

Recently, several glycosyltransferase genes from *Campylobacter jejuni* (OH4384) have been identified to be involved in producing various ganglioside-related lipoligosaccharides (LOS) expressed by this pathogenic bacteria. Gilbert, M.;Brisson, J.-R.;Karwaski, M.-F.;Michniewicz, J.;Cunningham, A.-M.;Wu, Y.;Young, N. M. and Wakarchuk, W. W., J. Biol. Chem. 2000, 275, 3896-3906. Among these genes, cst-II, coding for a bifunctional α(2-3/8) sialyltransferase, has been demonstrated to catalyze transfers of Neu5Ac α(2-3) and α(2-8) to lactose and sialyllactose, respectively. Another gene, cgtA, coding for a β(1-4)-*N*-acetylgalactosaminyltransferase (β4Ga1NAcT) that is reported to transfer Ga1NAc β(1-4) to Neu5Acα(2-3)lactose acceptors generating the GM2 (Neu5Acα(2-3)[GalNAcβ(1-4)]Galβ(1-4)Glc-) epitope.

The gene products of the two glycosyltransferase genes (cst-II and cgtA) were successfully over expressed in large scale (100 L *E. coli* fermentation) and used in the preparative synthesis of various ganglioside mimics. For synthetic purposes an extensive specificity study of these enzymes was also conducted using neutral and sialylated structures to further specify the synthetic utility of these enzymes.

For a cost-efficient synthesis of GalNAc-containing oligosaccharides, expensive uridine-5'-diphosphate-*N*-acetylgalactosamine (UDP-GalNAc) was produced *in situ* from inexpensive UDP-GlcNAc by the UDP-GlcNAc-4'-epimerase (GalNAc-E). GalNAc-E was cloned from rat liver into the *E. coli* expression vector (pCWori) and expressed in *E. coli* AD202 cells. Briefly, a lactose derivative was elongated with sialic acid repeats using α(2-8)-sialyltransferase and crude CMP-Neu5Ac. Several products (GM3, GD3, GT3) were isolated from this mixture. Increasing CDP-Neu5Ac from 2.5 to 4 equivalents favors the formation of GT3, and minor amounts of GD3 were isolated. Typical yields range from 40-50% of the major compound and 15-20% for the minor compound. Isolated compounds were further furbished with the action of GM2-synthetase (CgtA) and GalE to give the corresponding GM2, GD2, and GT2 structures in quantitative yields (Scheme IV).

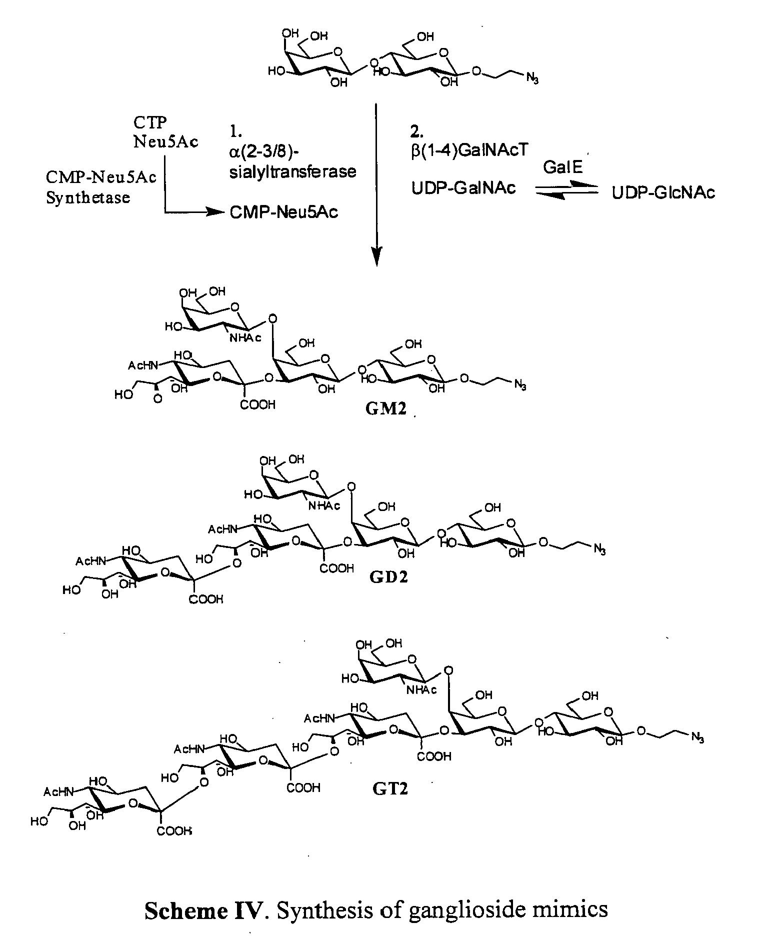

Therefore, methodologies were developed for generating diverse series of glycans, such as poly-*N*-acetyllactosamine and its corresponding fucosylated and/or sialylated compounds, various sialoside derivatives of *N-* and *O*-linked glycans, and ganglioside mimic structures. Furthermore, a simple route to produce the scarce sialic acid derivatives was described. This work demonstrates that chemoenzymatic synthesis of complicated carbohydrate structures can reach a facile and practical level by employing a functional toolbox of different glycosyltransferases. Detailed information of the specificity of these enzymes is needed for developing a library of glycan compounds with an extensive structural assortment. The invention provides such a library of carbohydrates and methods for using the library in high throughput studies of carbohydrate-protein, as well as, carbohydrate-carbohydrate interactions.

### EXAMPLE 4: Isolating Glycans from Natural Sources

The Example illustrates how certain type of mannose-containing glycans can be isolate from bovine pancreatic ribonuclease B.

**Pronase Digestion of Bovine Pancreatic Ribonuclease B:** Bovine pancreatic ribonuclease B (Sigma Lot 060K7650) was dissolved in buffer (0.1 M Tris+1mM MgCl₂+1mM CaCl₂ pH 8.0) and pronase (Calbiochem Lot B 50874) was added to give a ratio by weight of five parts glycoprotein to one part pronase. It was incubated at 60°c for 3 hours. Mannose-containing glycans in the digested sample were affinity purified using a freshly prepared ConA in buffer (0.1M Tris, 1mM MgCl₂, 1mM CaCl₂, pH 8.0), washed and eluted with 200mls 0.1M methyl-α-D-mannopyranoside (Calbiochem Lot B37526). The Con A eluted sample was purified on Carbograph solid-phase extraction column (Alltech 1000mg, 15ml) and eluted with 30% acetonitrile +0.06%TFA. It was dried and reconstituted in 1ml water. Mass analysis was done by MALDI and glycan quantification by phenol sulfuric acid assay.

The pronase digested ribonuclease b was diluted with 5mls 0.1M Tris pH 8.0 loaded onto 15mls Con A column in 0.1M Tris, 1mM MgCl₂, 1mM CaCl₂, pH 8.0, washed and eluted with 50mls 0.1M methyl-α-D mannopyranoside. It was then purified on Carbograph solid-phase extraction column (Alltech 1000mg, 15ml) eluted with 80% acetonitrile, containing 0.1%TFA,dried and reconstituted in 2ml water. Mass analysis and glycan quantification were performed using a Voyager Elite MALDI-TOF (Perseptive BioSystems) in negative mode.

**Separation of Fractions on Dionex:** Pronase digested ribonuclease b was injected on the DIONEX using a PA-100 column and eluted with the following gradient: Solution A= 0.1M NaOH, B=0.5M NaOAc in 0.1M NaOH; 0% B for 3mins, then a linear gradient from 0%B to 6.7%B in 34mins. The individual peak fractions were collected and purified on Carbograph solid-phase columns (Alltech 150mg, 4ml) by eluting with 80% acetonitrile containing 0.1% TFA. They were dried and reconstituted in water. Final Mass analysis and glycan quantification were performed.

### EXAMPLE 5: Generating Glycan Arrays

In this Example, arrays were generated using glycans that had -OCH₂CH₂NH₂ (called Sp1) or -OCH₂CH₂CH₂NH₂ (called Sp2 or Sp3) groups attached. These Sp1, Sp2 and Sp3 moieties provide primary amino groups for attachment to a derivatized solid support. The solid support employed had an N-hydroxy succinimide (NHS)-derivatized surface and was obtained from Accelr8 Technology Corporation, Denver, CO. After attachment of all the desired glycans, slides were incubated with ethanolamine buffer to deactivate remaining NHS functional groups on the solid support. The array was used without any further modification of the surface. No blocking procedures to prevent unspecific binding were needed.

Each type of glycan was printed onto to the solid support at a defined glycan probe location using a microarray gene printer available at Scripps Institute. About 0.5 nL of glycan solution was applied per defined glycan probe location. Various concentrations of the glycan solutions were printed onto the solid support ranging from 10 to about 100 µM glycan can be employed. Six replicates of each glycan concentration were printed onto defined glycan probe locations. Such replicates provide internal controls that confirm whether or not a binding reaction between a glycan and a test molecule is a real binding interaction. This procedure is further outlined in FIG. 1.

### EXAMPLE 6: Illustrative Binding Studies and Optimization of the Glycan Array

Covalent attachment of glycan structures was verified by detection of binding of the lectin Concanavalin A to a mannose-containing glycan. Thus, a mannose oligosaccharide (Ma2Ma3 [Ma2Ma6]Ma) was printed at various concentrations ranging from 4 µM to 500 µM and printed at six different time points over a period of 6 hrs while the slide was exposed to air at 40% humidity. A replicate of eight was used for each concentration. Glycan ligands not recognized by the ConA-FITC labeled lectin as were used as negative controls. FIG. 2 shows that a concentration of >60 µM glycan provided maximal lectin binding signal.

Similar data were obtained in analogous studies with 32 other ligands printed at five different concentrations (6-100 µM) for detection of other lectins. Several glycan specific plant lectins, human lectins and monoclonal antibodies were evaluated at various concentrations (2-300 µg/mL, 50 µL/slide) using methods similar to those described in the Examples provided herein. Detection of binding was via a fluorescent dye conjugated to the binding protein or through a labeled secondary antibody that bound to the binding protein. Fluorescence intensity is observed using a ScanArray 5000 (GSI Lumonics, Watertown, MA) confocal scanner and data analyses is carried out done using ImaGene image analysis software (BioDiscovery Inc., E1 Segundo, CA).

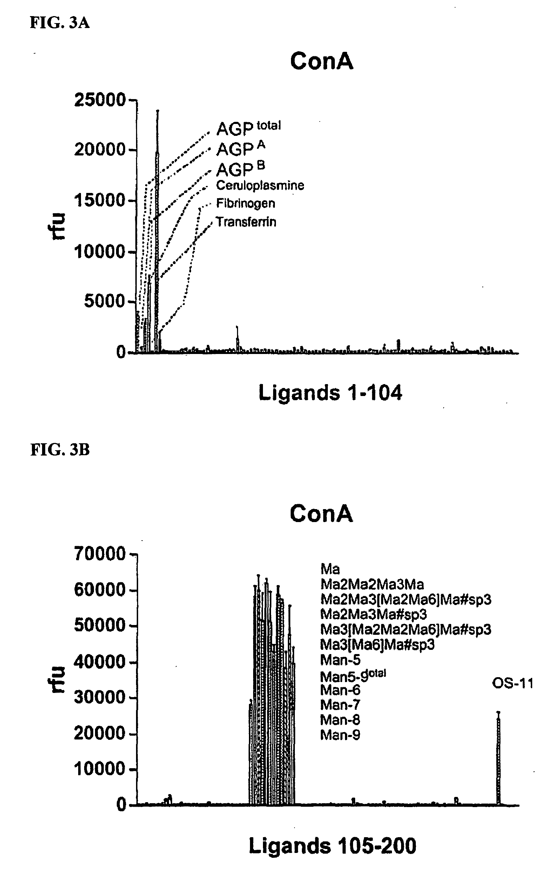

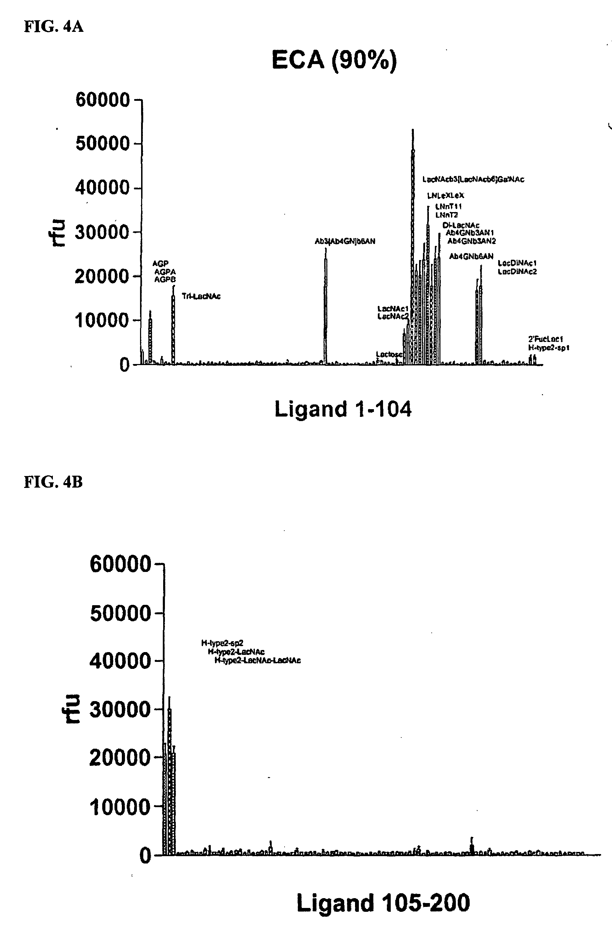

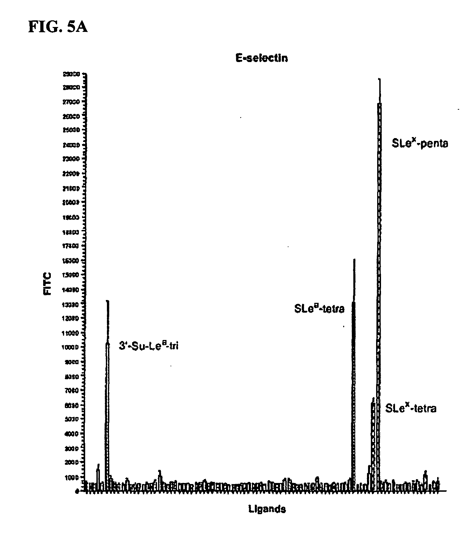

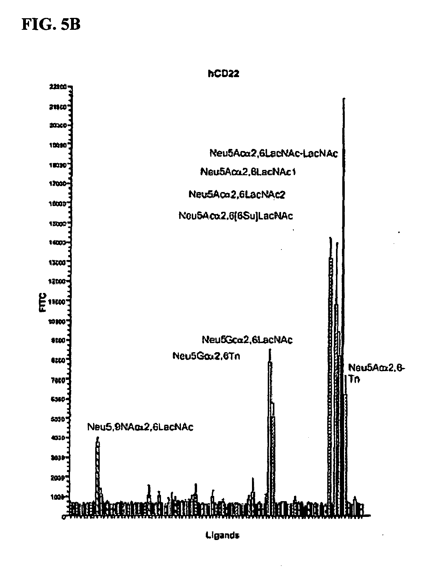

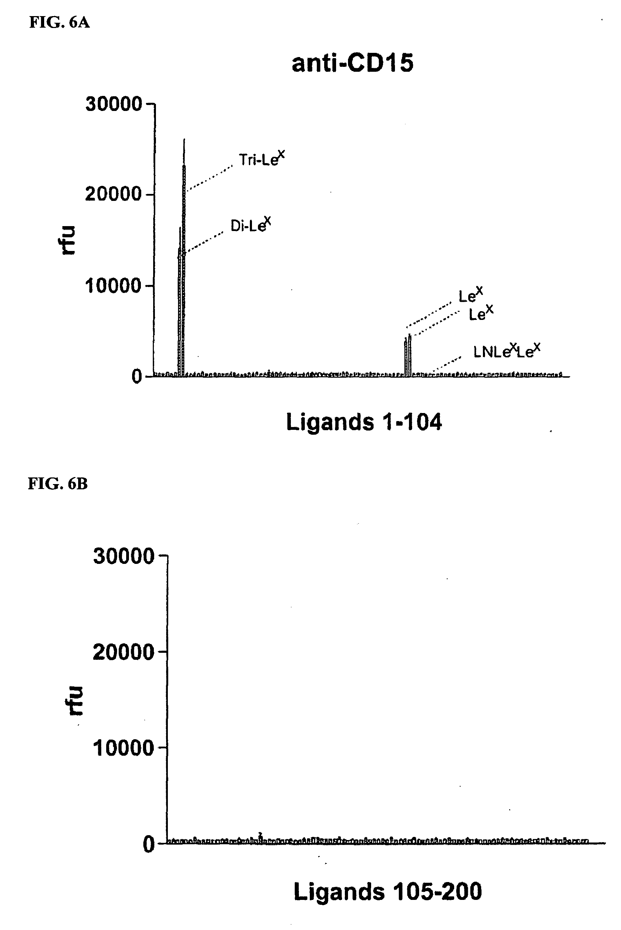

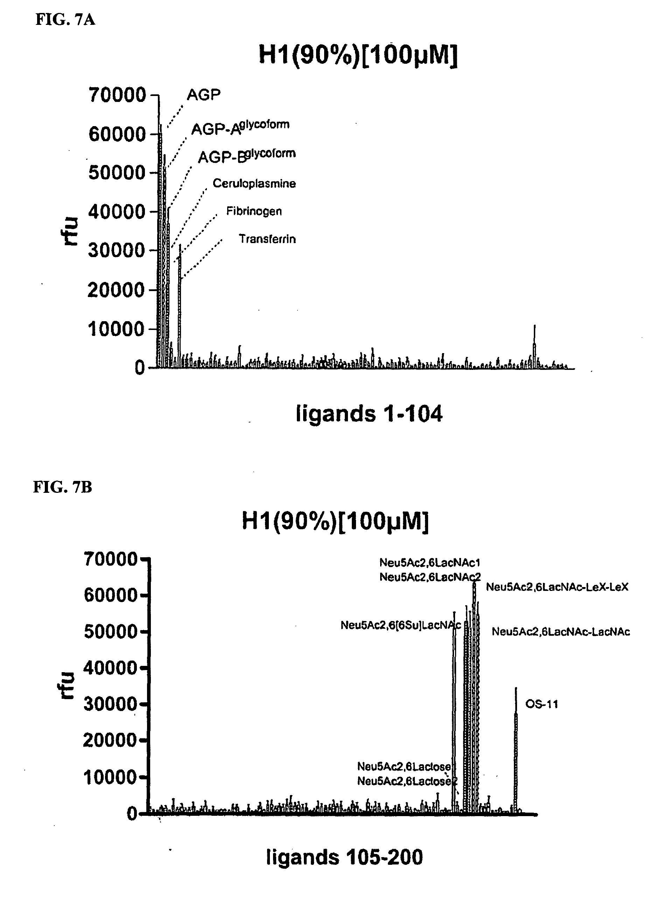

In particular, the following test molecules were examined for binding to the glycan arrays of the invention. FIGs. 3 and 4 provide the results for fluorescently labeled plant lectins ConA (FIG. 3) and ECA (FIG. 4). Similar data were obtained for SNA, LTA and UEA-I (data not shown). FIG. 5 provides the results of binding human lectins human C-type lectin, E selectin and Siglec-2, CD22 to the glycan arrays using fluorescently labeled secondary antibodies to detect a Fc moiety attached to the human lectins. FIG. 6 illustrates that certain fluorescently labeled antibodies bind specifically to selected glycans, for example, the human anti-glycan CD 15 antibodies. FIG. 7 shows that hemaglutinin H1 (1918) of the influenza virus binds to selected glycans as detected with two subsequently added fluorescent labeled secondary antibodies.

These experiments also confirmed that a printing time of up to 6 hrs at 30-50% relative humidity does not significantly reduce the lectin binding signal caused by hydrolytic de-activation of the NHS-surface, which can be important for longer print runs and thus the expansion of the array.

A strong and stable covalently linked library enabled the slides to be intact while exposed to extensive washing procedures before and after incubation of the analyte. Bound lectins could also be removed by competing ligands in solution or in combinations with salt, acid, base or detergent solutions applied on the surface. The ConA lectin was repeatedly stripped off with a sequence of ManαOMe (100mM). HOAc (1M), NaOH (0.3M) and NaCl (1M), and re-applied to the same slide up to 6 times without ally decrease of signal or any significant increase in background signal (data not shown).

### EXAMPLE 7: Generating Cleavable Linkers on a Glycan Array

This Example illustrates synthesis of cleavable linkers that permit cleavage and analysis of the types of glycans on the array. When an antibody other binding entity binds to the glycan array the exact structure(s) of the bound glycan(s) can be determined by cleavage of the glycan from the array and structural analysis.

Cleavable linkers were prepared as described below. Reagents were obtained from commercial suppliers and used without further purification. All glassware and syringes were dried in an oven overnight, allowed to cool and stored under a positive pressure of argon before use. Dichloromethane was dried over CaH₂. Anhydrous methanol was obtained from Aldrich. Methanol employed for the formation of triazoles was degassed before use. Compounds were purified by flash chromatography on silica gel. TLC was run on SiO₂ 60F₂₅₄ (Merck) and detected with UV, H₂SO₄ and KMnO₄ reagents. ¹H and ¹³CNMR spectra were measured at 400 and 500 MHz (Bruker). The melting points are uncorrected. CovaLink-Nunc brand amine-functionalized microtiter plates were purchased from Nunc and the Amine-Trap NHS microtiter plates were purchased from NoAb Biodiscoveries. Fluorescein labeled *Lotus tetragonolobus* and *Erythrina cristagalli* lectins were purchased from Vector Labs. Fluorescein-conjugated Goat Anti-Human IgG antibody was purchased from Jackson ImmunoResearch. All remaining materials for biological assays were purchased from Sigma. A Fusion Universal Microplate Analyzer from Packard BioScience Company was utilized for absorbance and fluorescence measurements and a Hitachi M-8000 Mass Spectrometer was used for SSI and ESI measurements.

### Disulfide Linkers

### Propynoic acid [2-(2-amino-ethyldisulfanyl)-ethyl]-amide, trifluoroacetic acid salt

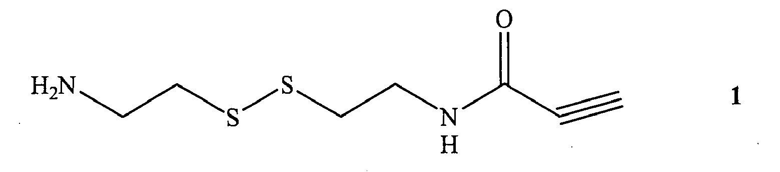

To a stirred solution of dicyclohexylcarboimide (DCC) (3.8 mmol) in 100 mL of anhydrous dichloromethane, under argon, at 0 °C, was added propynoic acid (3.2 mmol). After 10 min, *N-tert*-Butyloxycarbonylcystamine (Jacobson, 1995 #21) (3.2 mmol) dissolved in 50 mL of anhydrous dichloromethane was added dropwise and the resulting mixture stirred for 1 h at 0 °C and for 1 h at room temperature. The mixture was then filtered, and the solution evaporated under reduced pressure. The crude product was purified by flash chromatography on silica gel using as eluent AcOEt/n-hexane (1:1). This compound (1.64 mmol) was dissolved in 5 mL of dichloromethane and cooled at 0 °C. TFA (5 mL) was then added and the solution stirred for 15 min at 0°C. After evaporation, to remove trace of TFA, the crude product was redissolved twice in 10 mL of water and evaporated again. The amine was obtained, without further purifications, as trifluoroacetate salt in high purity. ¹H NMR (CD₃OD) δ 3.14 (s, 1H), 3.12 (t, 2H, *J* = 6.60 Hz), 2.86 (t, 2H, *J* = 6.60 Hz), 2.54 (t, 2H, *J* = 6.60 Hz), 2.43 (t, 2H, *J* = 6.60 Hz). ¹³C NMR (CD₃OD) δ 154.87, 77.91, 76.21, 39.63, 39.27, 37.56, 35.21. HR-MALDI-FTMS: calcd for C₇H₁₃N₂OS₂ [M + H]⁺, 205.0464; found, 205.0468.

### Propynoic acid (2-{2-[3-(4-isothiocyanato-phenyl)-thioureido]-ethyldisulfanyl}-ethyl)-amide (2)

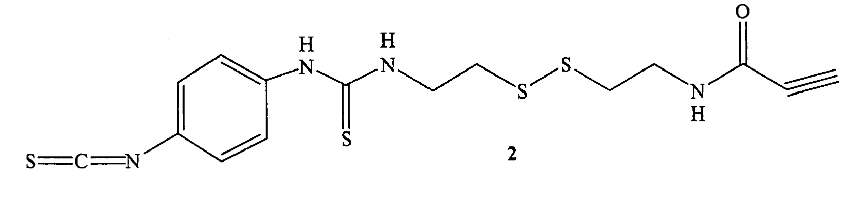

1,4-Phenylene diisothiocyanate (1.38 mmol) was dissolved together with diisopropylethylamine (DIEA) (0.34 mmol) in 2 mL of anhydrous DMF. To this stirred solution was added propynoic acid [2-(2-amino-ethyldisulfanyl)-ethyl]-amide, trifluoroacetic acid salt (**1**)(0.34 mmol) dissolved in 2 mL of anhydrous DMF, over a period of 30 min. The reaction was stirred for additional 30 min at room temperature and the solvent was distilled off under high vacuum (bath temperature <40 °C). The crude product was directly purified by column chromatography on Aluminum Oxide 90 (active neutral) using as solvent *n-*hexane/AcOEt (1:1). Fractions were evaporated at a temperature <30 °C. The isothiocyanate derivative **2** was obtained in 45% yield (60mg). This compound was moisture sensitive and unstable at room temperature. Store in freezer (T° <-30 °C) over Drierite®. ¹H NMR (CDCl₃) δ 8.76 (s, 1NH), 7.95 (s, 2NH), 7.43 (d, 2H, *J* = 8.80 Hz), 7.15 (d, 2H, *J* = 8.80 Hz), 3.92 (q, 2H, *J* = 5.87 Hz), 3.58 (q, 2H, *J* = 6.60 Hz), 2.96 (t, 2H, *J* = 5.87 Hz), 2.86 (s, 1H), 2.75 (t, 2H, *J* = 6.97 Hz). ¹³C NMR (CDCl₃) δ 180.85, 152.80, 137.01, 135.42, 127.95, 126.38, 124.79, (CDCl₃ signals overlap one alkyne-carbon), 74.43, 42.77, 38.98, 37.75, 36.15, 31.44. HR-MALDI-FTMS: calcd for C₁₅H₁₇N₄OS₄ [M + H]⁺, 397.0282; found, 397.0282.

### Azide- and Amine-Containing Glycans

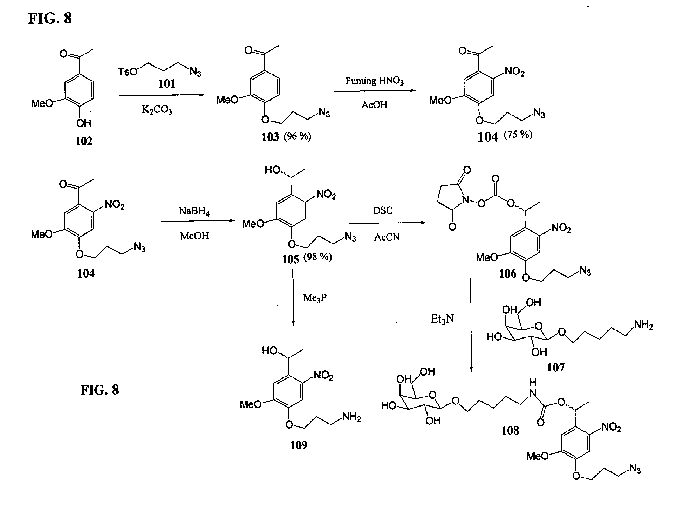

Saccharides containing the azide or amine were synthesized as reported by Fazio et al. Tetrahedron Lett. 45: 2689-92 (2004); Fazio et al., J. Am. Chem. Soc. 124: 14397-14402 (2002); Lee et al. Angew. Chem. Int. Ed. 43: 1000-1003 (2004); Burkhart et al. Angew. Chem. Int. Ed. 40: 1274-77 (2001). The synthetic procedures employed are described below and shown in FIGs. 8-9.

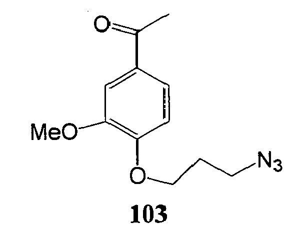

*Synthesis of compound 103:* As shown in FIG. 8, compound **101** (1.5 g, 5.88 mmol) was added to a solution of acetovanillone **102** (0.9 g, 5.41 mmol), potassium carbonate (1.1 g, 7.96 mmol) in DMF (20 mL) at room temperature under Ar. The reaction mixture was warmed to 75 °C and stirred for 12 h. The solvent was removed under reduced pressure and the residue was separated by column chromatography (SiO2/ hexane:EA = 3 : 1) to afford 1.30 g (0.52 mmol, 96%) of compound **103**.

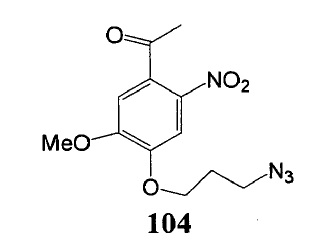

***Synthesis of compound 104:*** Fuming nitric acid (0.96 mL) was added to a solution of compound **3** (0.78 g, 3.12 mmol) in acetic acid (9.60 mL); during the addition, the reaction mixture was cooled by ice-water bath. The reaction mixture was stirred at 70 °C for 18 h and the poured into ice-water. The yellow precipitate was filtered and was purified by column chromatography (SiO₂/hexane : EA = 2 : 1) to afford 0.69 g (2.34 mmol, 75 %) compound **104**.

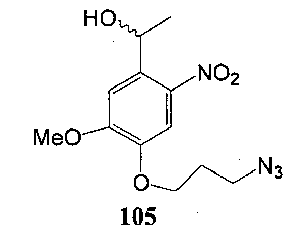

***Synthesis of compound 105:*** Sodium borohydrate (0.18g, 4.90 mmol) was added to a solution of compound **104** (1.2 g, 4.08 mmol) in methanol (15 mL); during the addition , the reaction mixture was cooled by ice-water bath. The reaction mixture was stirred at room temperature for 1 h. The solvent was removed under reduced pressure and the residue was separated by column chromatography (SiO₂/ hexane:EA = 2 : 1) to afford 1.18 g (4.0 mmol, 98 %) of compound **105.**

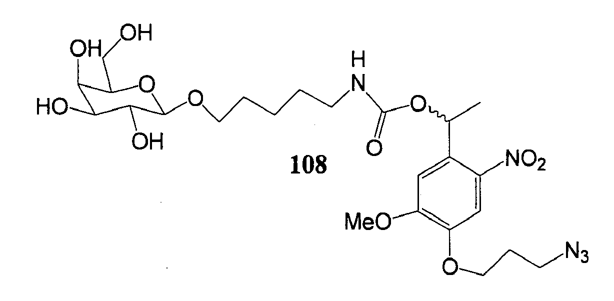

***Synthesis of compound** 108:* Compound **105** (68.5 mg, 0.23 mmol) was dissolved in 3.0 mL of dry acetonitrile . To this solution N, N'-disuccinimidyl carbonate (90 mg, 0.45 mmol) was added, followed by triethylamine (0.18 mL). After stirring at room temperature for 5 hr, solvents were evaporated to dryness. The residue was washed consecutively with 0.1 N NaHCO₃, water and EA, and then dried to give crude compound **106**. Amino linkage mannose compound **107** (61.5 mg, 0.23 mmol) was added to a solution of crude compound 106 in DMF and followed by triethylamine (0.18 mL). The solution was stirred at room temperature for 5 hr and the solvent was removed under reduced pressure and the residue was separated by column chromatography (SiO₂/ CHCl₃/MeOH = 1 : 3) to afford 97.2 mg (0.16 mmol, 72 %) of compound **108.**

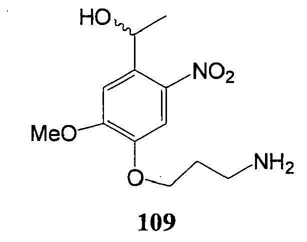

***Synthesis of compound 109:*** A solution of 356.2 mg (1.2 mmol) of azide compound **5** in 8.0 mL of tetrahydrofuran was treated with 2.0 mL (2.0 mmol) of 1 M solution of trimethylphosphine in toluene. The reaction was stirred for 1 h, and then 2.0 mL of water was added, and stirring was continue for 2 h. the reaction mixture was concentrated, and the residue was purified by column chromatography (SiO2/ CHCl3/MeOH = 1 : 3) to afford 291.7 mg (1.08 mmol, 90 %) of compound **109.**

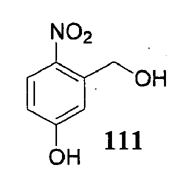

***Synthesis of compound 111:*** Sodium borohydrate (1.4 g, 37.84 mmol) was added to a solution of compound **110** (4.07 g, 24.35 mmol) in methanol (30 mL); during the addition, the reaction mixture was cooled by ice-water bath. The reaction mixture was stirred at room temperature for 1 h. The solvent was removed under reduced pressure and the residue was recrystallized in MeOH to give 4.0 g (23.62 mmol, 97 %) compound **111**.

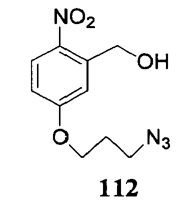

***Synthesis of compound 112:*** Compound **101** (3.0 g, 11.76mmol) was added to a solution of compound **111** (1.8 g, 10.69 mmol), potassium carbonate (2.2 g, 15.92 mmol) in DMF (40 mL) at room temperature under Ar. The reaction mixture was warmed to 60 °C and stirred for 12 h. The solvent was removed under reduced pressure and the residue was separated by column chromatography (SiO2/ hexane:EA = 1 : 1) to afford 2.4 g (9.56 mmol, 95 %) of compound **112.**

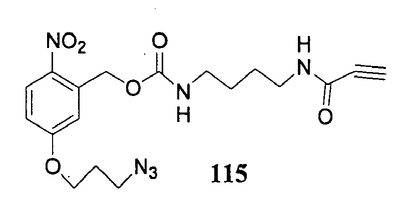

***Synthesis of compound 115**:* Compound **112** (58.0 mg, 0.23 mmol) was dissolved in 3.0 mL of dry acetonitrile. To this solution N, N'-disuccinimidyl carbonate (90.0 mg, 0.45 mmol) was added, followed by triethylamine (0.18 mL). After stirring at room temperature for 5 hr, solvents were evaporated to dryness. The residue was washed consecutively with 0.1 N NaHCO₃, water and EA, and then dried to give the crude compound **113**. Acetylene compound **114** (32.2 mg, 0.23 mmol) was added to a solution of compound **106** in DMF and followed by triethylamine (0.18 mL). The solution was stirred at room temperature for 5 hr and the solvent was removed under reduced pressure and the residue was separated by column chromatography (SiO₂/ CHCl₃/MeOH = 1 : 3) to afford 67.32 mg (0.16 mmol, 70 %) of compound **115.**

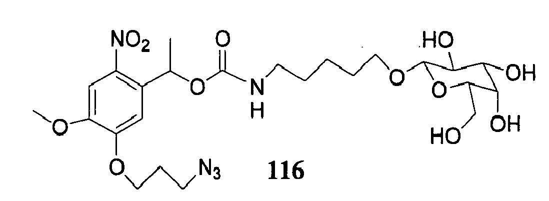

***Synthesis of compound 116:*** Compound **112** (58.0 mg, 0.23 mmol) was dissolved in 3.0 mL of dry acetonitrile . To this solution N, N'-disuccinimidyl carbonate (90 mg, 0.45 mmol) was added, followed by triethylamine (0.18 mL). After stirring at room temperature for 5 hr, solvents were evaporated to dryness. The residue was washed consecutively with 0.1 N NaHCO₃, water and EA, and then dried to give crude compound **106**. Amino linkage mannose compound **107** (61.5 mg, 0.23 mmol) was added to a solution of crude compound **106** in DMF and followed by triethylamine (0.18 mL). The solution was stirred at room temperature for 5 hr and the solvent was removed under reduced pressure and the residue was separated by column chromatography (SiO₂/ CHCl₃/MeOH = 1 : 3) to afford 102.6 mg (0.17 mmol, 76 %) of compound **116**.

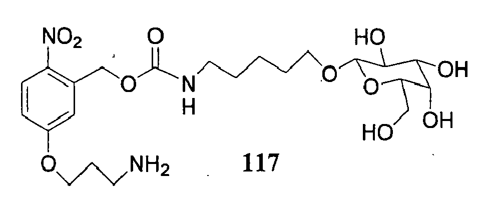

***Synthesis of compound 117:*** A solution of 70.5 mg (0.12 mmol) of azide compound 116 in 2.0 mL of tetrahydrofuran was treated with 0.2 mL (0.2 mmol) of 1 M solution of trimethylphosphine in toluene. The reaction was stirred for 1 h, and then 0.2 mL of water was added, and stirring was continue for 2 h. the reaction mixture was concentrated, and the residue was purified by column chromatography (SiO₂/ CHCl₃/MeOH/NH₄OH = 1 : 3 : 0.3) to afford 60.6 mg (1.08 mmol, 90 %) of compound **117.**

### Covalent attachment of Alkyne-Containing Linker Precursor to Surface

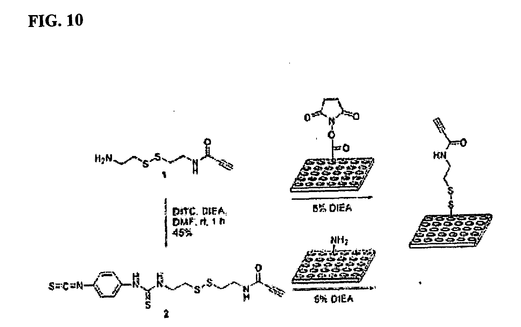

**Thioisocyanate Capture:** Amine-coated microtiter plate wells were treated each with of a 1 mM solution of linker **2** in 5% DIEA/DMSO (100 µL) for 8 h at room temperature. After this time, the solution was removed, and wells were washed with MeOH (2 × 200 µL). This reaction is shown in FIG. 10.

**Amine Capture**: NHS-coated microtiter plate wells were treated with linker **1** (1 mg/ mL 5% DIEA/MeOH; 200 µL) for 8 h at 4°C. After this time, the solution was removed and wells were washed with QH2O (3 × 200 µL). This reaction is shown in FIG. 10.

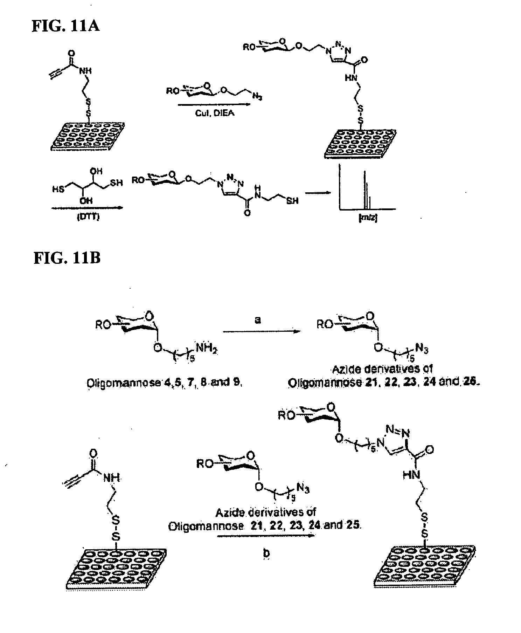

**Triazole Formation and Cleavage**: Successively to the wells were added the azide-containing saccharide in 5% DIEA/MeOH (200 µL) and CuI (cat.). The plate was covered and shaken for 12-14 h at 4 °C. The solution was then removed and the plate was washed with QH₂O (3 × 200 µL). Dithiothreotol (50 mM in H₂O) was then added to wells and the plate was incubated for 24 h at 4°C. The plate was then directly subjected to mass spectral analysis. This reaction is shown in FIG. 11.

### EXAMPLE 8: Arrays with Cleavable Linkers are Useful

### in a Variety of Screening Reactions

An array with a cleavable linker was synthesized as described in the foregoing Example and then used successfully in screening assays to determine which molecules bind to distinct glycans.

### Screening Assays

***Lotus tetragonolobus* Lectin Binding:** After washing with QH₂O, wells were blocked with 10 mM HEPES buffer, pH 7.5/150 mM NaCl buffer (buffer A; 200 µL) containing 0.1% Tween-20 over 1 h at 4 °C. The buffer was then removed and fluorescein-labeled *Lotus tetragonolobus* lectin (20 µg/mL buffer A; 200 µL) was incubated in the well over 1 h in the dark at 4°C. Wells were then washed with QH₂O five times (200 µL) and fluorescence was measured with an excitation wavelength of 485 nm and emission wavelength of 535 nm.

***Erythrina cristagalli* Lectin Binding:** After washing with QH₂O, wells were blocked with 10 mM HEPES buffer, pH 7.5/150 mM NaCl buffer (buffer A; 200 µL) containing 0.1% Tween-20 over 1 h at 4 °C. The buffer was then removed and fluorescein-labeled *E. cristagalli* (5 µg/mL buffer A; 200 µL) was incubated in the well over 1 h in the dark at 4 °C. Wells were then washed with QH₂O five times (200 µL) and fluorescence was measured with an excitation wavelength of 485 nm and emission wavelength of 535 nm.

### Results

To characterize the biological applicability of this display method, lectin-binding studies were performed. Two lectins (sugar-recognizing protein) were used to study the bound carbohydrates: *Lotus tetragonolobus* lectin (LTL), which recognizes R-1-fucose, and *Erythrina cristagalli* (EC), which recognizes galactose. Both lectins were assayed successfully with the simple monosaccharides Fucose-O(CH₂)₂-N₃ and Galactose-O(CH₂)₂-N₃.

### EXAMPLE 9: Identification and Characterization of a Breast Cancer Antigen

This Example describes analysis of the antigenic epitopes recognized by a monoclonal MBr1 antibody that binds to breast cancer cells present in 85% of breast cancer patients.

Globo H analogs **201-204** were synthesized and attached onto a microarray platform.

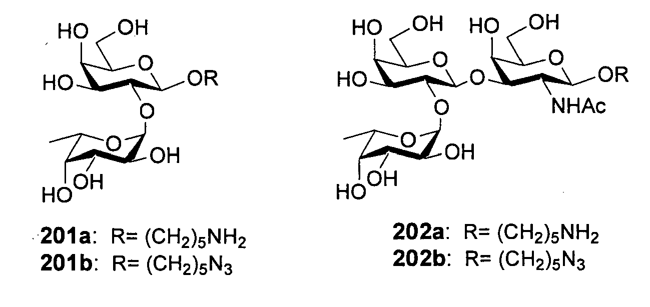

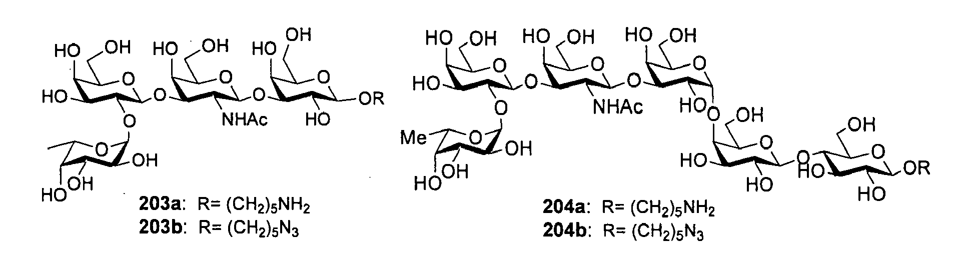

Amino-functionalized derivatives (**201-204a**) and the corresponding azido analogs (**201-204b**) were prepared in order to analyze the sugars using two different immobilization methods. In addition to the microarray analysis, a fluorescence-tagged Globo H derivative was made for analytical sequencing to provide structural confirmation. This method acts as a complement to traditional NMR-based studies for the determination of the structure of biological ligands. The combination of these microarray and sequencing tools permitted thorough characterization of the important carbohydrate epitope of Globo H and its interaction with the corresponding monoclonal antibody binding partner, MBr1.

### Materials and Methods

**General.** All chemicals were purchased and used without further purification. Dichloromethane (CH₂Cl₂) was distilled over calcium hydride. Diethyl Ether (Et₂O) was distilled over sodium. Molecular sieves (MS, AW-300) used in glycosylations were crushed and activated before use. Reactions were monitored with analytical TLC on silica gel 60 F254 plates and visualized under UV (254 nm) and/or by staining with acidic cerium ammonium molybdate. Flash column chromatography was performed on silica gel (35-75 µm) or LiChroprep RP18. ¹H-NMR spectra were recorded on a Bruker DRX-500 (500MHz) or DRX-600 (600MHZ) spectrometer at 20°C. Chemical shifts (in ppm) were determined relative to either tetramethylsilane in deuterated chloroform (δ=0 ppm) or acetone in deuterated water (δ=2.05 ppm). Coupling constants in Hz were measured from one-dimensional spectra. ¹³C Attached Proton Test (¹³C-APT) NMR spectra were obtained by using the same Bruker NMR spectrometer (125 or 150 MHz) and were calibrated with CDCl₃ (δ=77 ppm). Coupling constants (*J*) are reported in Hz. Splitting patterns are described by using the following abbreviations: s, singlet; brs, broad singlet; d, doublet; t, triplet; q, quartet; m, multiplet. ¹H NMR spectra are reported in this order: chemical shift; multiplicity; number(s) of proton; coupling constant(s).

### One-pot synthesis of protected Globo H (208)

Globo H (**208**) was synthesized as follows, and deprotected to form glycans **204a** and **204b.**

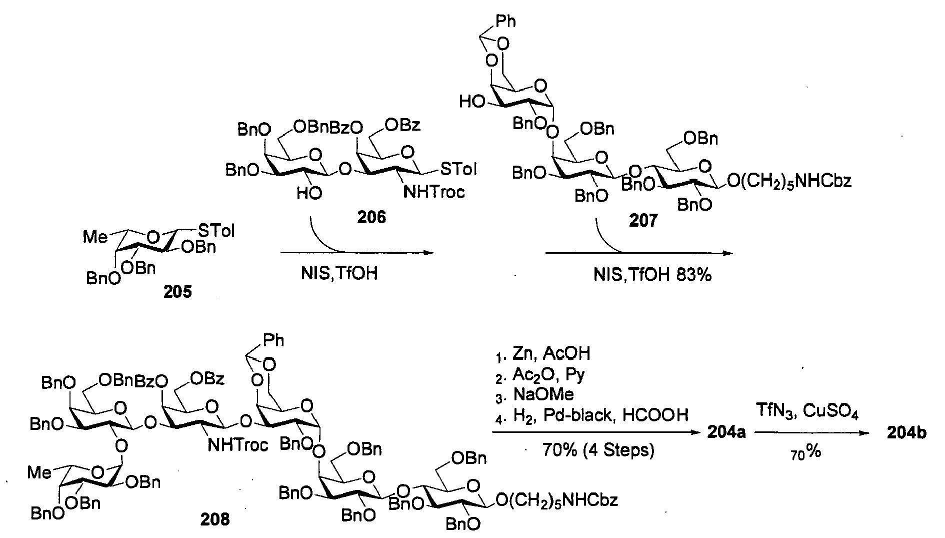

Fucosyl donor **205** (118 mg, 1.2 equiv), disaccharide building block **206** (200 mg, 1 equiv), and MS were stirred in CH₂Cl₂ (7 ml) for one hour at room temperature. The reaction was cooled to -50°C, and NIS (49 mg, 1.2 equiv) was added, followed by TfOH (1M solution in ether, 0.054 ml, 0.3 equiv). The mixture was stirred for two hours at -40 to -50 °C and the reaction was followed by TLC until complete. Trisaccharide **207** (263 mg, 1.0 equiv) was dissolved in CH₂Cl₂ (1.5 ml) and added to the reaction mixture. NIS (49 mg, 1.2 equiv) was then added, followed by TfOH (1M solution in ether, 0.015 ml, 0.16 equiv). The reaction was stirred at -30 °C for two hours and then diluted with CH₂Cl₂ and quenched with a few drops of triethylamine. Next, the reaction mixture was washed with sat. aq. NaHCO₃ and sat. aq. Na₂S₂O₃ and then dried over Na₂SO₄. Purification by column chromatography (1:1:0.1 to 1:1:0.4 Hex:CH₂Cl₂:EtOAc) provided **208** (429 mg, 0.151 mmole, 83%) as a white foam. ¹H-NMR (500MHz, CDCl₃) d 7.96 (dt, 4H, J=1.45, 9.50Hz), 7.48-7.37 (m, 6H), 7.36-7.13 (m, 67H), 7.08-6.97 (m, 8H), 6.09 (d, 1H, J=6.2Hz), 5.62 (d, 1H, J=2.6Hz), 5.53 (d, 1H, J=2.2Hz), 5.24 (s, 1H), 5.13-5.06 (m, 5H), 5.00 (d, 1H, J=1.5Hz), 4.97-4.90 (m, 2H), 4.88-4.66 (m, 7H), 4.64-4.55 (m, 4H), 4.54-4.28 (m, 15H), 4.27-4.22 (m, 3H), 4.19-3.96 (m, 9H), 3.95-3.54 (m, 13H), 3.51-3.45 (m, 2H), 3.41-3.22 (m, 10H), 3.14-3.07 (m, 2H), 1.66-1.57 (m, 2H), 1.52-1.43 (m, 2H), 1.41-1.30 (m, 2H), 0.78 (d, 3H, J=5.9Hz). ¹³C-APT NMR (125MHz, CDCl₃) d165.9, 165.1, 156.3, 153.7, 139.3, 139.1, 139.0, 138.72, 138.7, 138.6, 138.3, 138.26, 138.2, 138.1, 138.0, 137.9, 136.6, 129.8, 129.6, 129.59, 129.5, 129.0, 128.5, 128.4, 128.3, 128.2, 128.18, 128.1, 127.9, 127.8, 127.79, 127.77, 127.6, 127.54, 127.5, 127.4, 127.39, 127.23, 127.2, 127.1, 127.0, 126.7, 126.2, 103.9, 103.4, 103.1, 10.9, 100.4, 96.6, 82.8, 81.9, 81.4, 81.0, 79.3, 78.8, 77.5, 77.1, 75.0, 74.9, 74.7, 74.66, 74.0, 73.8, 73.7, 73.6, 73.5, 73.1, 72.9, 72.8, 72.7, 72.4, 72.3, 71.8, 71.0, 70.3, 69.6, 69.0, 68.6, 68.5, 67.4, 66.9, 66.5, 63.0, 62.9, 55.7, 40.9, 29.7, 29.6, 29.3, 23.3, 16.4. Unit MS: C₁₆₄H₁₇₁Cl₃N₂O₃₅Na [M+Na]⁺ calcd: 2856 found: 2856.

### The protected tetrasaccharide (211)

A protected tetrasaccharide (**211**) was formed as follows and deprotected to form glycans **203a** and **203b.**

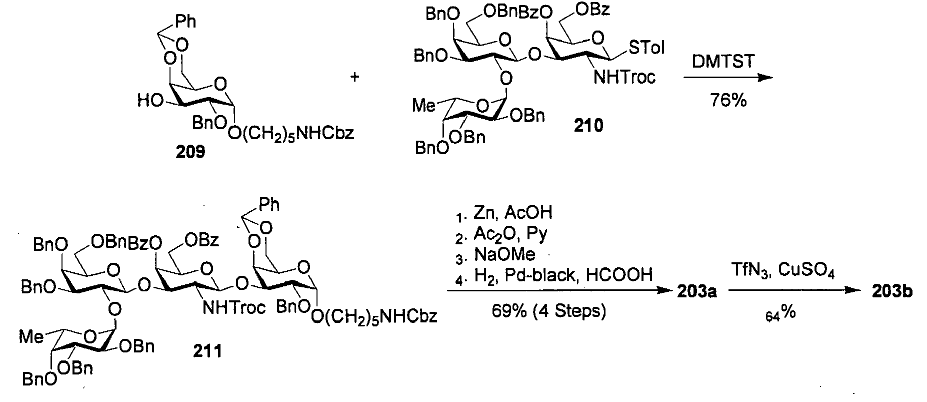

The MS was activated by microwave and was flamed dried under high vacuum over night. To donor **210** (54mg, 1.5 equiv.) and acceptor **209** (13.7mg, 1equiv.) in anhydrous CH₂Cl₂ were added molecular sieves and the reaction was stirred at rt for one hour. The reaction mixture was cooled to 0°C and then freshly synthesized DMTST (6 equiv.) was added. The reaction was stirred at 0°C for two hours and was then quenched with triethylamine. The reaction mixture was diluted with CH₂Cl₂ and was filtered though a celite pad. The organic layer was washed with saturated NaHCO₃ and brine, and then dried over anhydrous Na₂SO₄. The solvent was removed under reduced pressure, and the residue was purified by flash column chromatography on silica gel (Hex:EtOAc =3:1 to 1:1) to give the product (36.3mg, 76%). ¹H-NNM (500MHz, CDCl₃) δ8.08-7.95 (m, 4H), 7.59-6.95 (m, 51H), 5.58 (d, 1H, J=2.6Hz), 5.36 (s, 1H), 5.09 (s, 2H), 4.88-4.70 (m, 6H), 4.60-4.25 (m, 16H), 4.10-3.87 (m, 9H), 3.83-3.76 (m, 2H), 3.65 (d, 1H, J=11.75Hz), 3.61-3.51 (m, 3H), 3.50-3.38 (m, 3H), 3.36-3.10 (m, 2H), 3.21-3.13 (m, 2H), 1.60-1.47 (m, 4H), 1.39-1.31 (m, 2H), 0.89 (s, 3H). ¹³C-APT NMR (150MHz, CDCl₃) δ165.9, 165.3, 156.3, 154.0, 138.9, 138.7, 138.24, 138.2, 137.93, 137.9, 136.6, 133.15, 133.1, 129.9, 129.8, 129.6, 128.5, 128.4, 128.37, 128.32, 128.3, 128.2, 128.11, 128.1, 128.0, 127.9, 127.85, 127.73. 127.7, 127.6, 127.56, 127.4, 127.37, 127.2, 127.1, 127.0717, 127.0, 126.7, 126.3, 126.2, 123.8, 101.7, 100.6, 97.9, 96.6, 95.7, 82.8, 78.9, 77.5, 77.4, 74.9, 74.7, 74.6, 74.1, 74.0, 73.6, 73.4, 73.0, 72.9, 72.86, 72.8, 72.4, 72.2, 71.6, 70.4, 69.0, 68.4, 67.9, 67.3, 66.5, 63.2, 62.6, 55.7, 40.9, 29.7, 29.6, 28.8, 23.3, 16.4. HRMS: C₁₁₀H₁₁₅Cl₃N₂O₂₅Na [M+Na]⁺ calcd: 1991.6746, found: 1991.6776.

### The protected trisaccharide (212)

A protected tetrasaccharide (**212**) was formed as follows and deprotected to form glycans **202a** and **202b.**

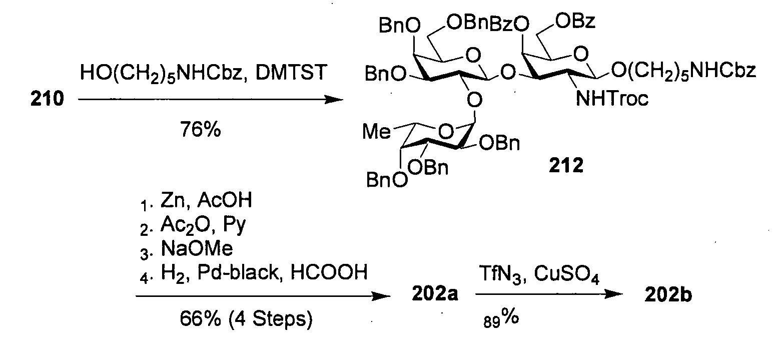

The MS was activated by microwave and was flamed dried under high vacuum over night. To donor **210** (109.6mg, 1 equiv.) and the acceptor (20.6mg, 1.2 equiv.) in anhydrous CH₂Cl₂ were added molecular sieves and the reaction was stirred at rt for one hour. The reaction mixture was cooled to 0°C and then freshly synthesized DMTST (6 equiv.) was added. The reaction was stirred at 0°C for two hours and was then quenched with triethylamine. The reaction mixture was diluted with CH₂Cl₂ and was filtered though a celite pad. The organic layer was washed with saturated NaHCO₃ and brine, and then dried over anhydrous Na₂SO₄. The solvent was removed under reduced pressure, and the residue was purified by flash column chromatography on silica gel (Hex:EtOAc =9:1 to 2:1) to give the product (89.8mg, 76%). ¹H-NMR (600MHz, CDCl₃) δ8.08-7.95 (m, 4H), 7.59-7.01 (m, 41H), 5.58 (d, 1H, J=3.06Hz), 5.08 (s, 2H), 4.87-4.72 (m, 5H), 4.64-4.52 (m, 6H), 4.48-4.32 (m, 9H), 4.13-4.04 (m, 2H), 3.97-3.89 (m, 2H), 3.87-3.72 (m, 4H), 3.64-3.58 (m, 1H), 3.57-3.38 (m, 5H), 3.18-3.09 (m, 2H), 1.61-1.49 (m, 2H), 1.47-1.39 (m, 2H), 1.35-1.28 (m, 2H), 0.99 (s, 3H). ¹³C-APT NMR (125MHz, CDCl₃) δ166.0, 165.2, 156.3, 154.1, 139.0, 138.7, 138.6, 138.3, 138.1, 137.9, 136.6, 133.1, 133.0, 129.96, 129.8, 129.7, 129.5, 128.4, 128.38, 128.2, 128.14, 128.1, 128.07, 128.0, 127.9, 127.8, 127.7, 127.68, 127.6, 127.4, 127.3, 127.2, 127.1, 126.7, 102.8, 101.0, 96.7, 83.1, 79.1, 77.6, 76.6, 74.6, 74.3, 74.1, 73.7, 73.5, 72.9, 72.86, 72.6, 72.2, 71.8, 70.1, 69.8, 68.7, 67.2, 66.5, 63.0, 55.4, 40.9, 29.6, 29.5, 29.0, 23.0, 16.4. HRMS: C₉₀H₉₅Cl₃N₂O₂₀Na [M+Na]⁺ calcd: 1651.5436 found: 1651.5483.

### The protected disaccharide (214)

A protected tetrasaccharide (**214**) was formed as follows and deprotected to form glycans **201a** and **20bb.**

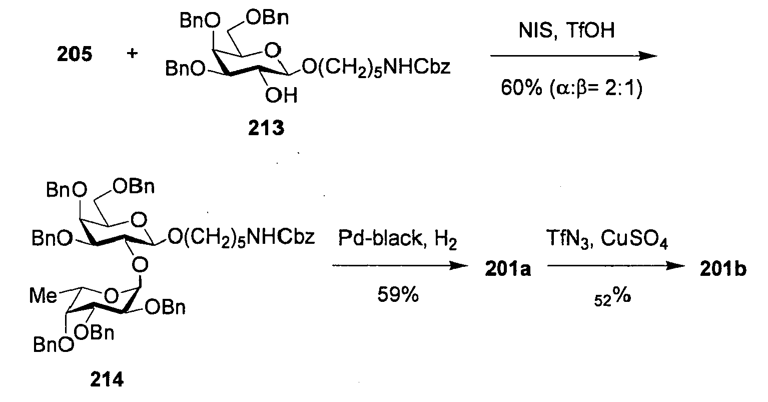

The MS was activated by microwave and was flamed dried under high vacuum over night. To donor **205** (471.5mg, 1.2 equiv.) and acceptor **213** (428.7mg, 1 equiv.) in 6mL 1,4-dioxane:CH₂Cl₂ =1:2 solution was added molecular sieves at rt and the reaction was then stirred for one hour. The reaction mixture was cooled to -40°C and then NIS (1.2 equiv.) and TfOH (0.2 equiv.) were added. The reaction was warmed to -20°C for two hours. The reaction was quenched with saturated sodium bicarbonate and sodium thiosulfate. The reaction mixture was diluted with CH₂Cl₂ and was filtered though a celite pad. The organic layer was washed with saturated NaHCO₃, sodium thiosulfate and brine, and then dried over anhydrous Na₂SO₄. The solvent was removed under reduced pressure, and the residue was purified by flash column chromatography on silica gel (Hex:EtOAc =4:1 to 1:1) to give the product (α isomer 254.0mg, 39%, β isomer 126.1mg, 20%). α isomer: ¹H-NMR (500MHz, CDCl₃) δ 7.51-6.95 (m, 35H), 5.68 (d, 1H, J=3.65Hz), 5.07 (s, 2H), 4.94 (d, 1H, J=11.35Hz), 4.86-4.74 (m, 4H), 4.66-4.37 (m, 9H), 4.21 (dd, 1H, J=9.55Hz, 8.05Hz), 4.03 (dd, 1H, J=9.9Hz, 3.65Hz), 3.98-3.91 (m, 2H), 3.84 (dt, 1H, J=6.6Hz, 8.8Hz), 3.72 (dd, 1H, J=9.9Hz, 2.2Hz), 3.66 (s, 1H), 3.62-3.54 (m, 3H), 3.39 (dt, 1H, J=6.6Hz, 8.8Hz), 3.12 (q, 2H, J=6.6Hz), 1.61-1.37 (m, 4H), 1.32-1.22 (m, 2H), 1.11 (d, 3H, J=6.75Hz). ¹³C-APT NMR (125MHz, CDCl₃) δ156.3, 138.9, 138.7, 138.3, 138.2, 137.8, 136.5, 128.4, 128.4, 128.3, 128.2, 128.1, 128.0, 127.9, 127.87, 127.8, 127.7, 127.5, 127.3, 127.29, 127.2, 127.17, 126.2, 102.0, 97.1, 84.3, 79.5, 77.9, 75.6, 74.6, 74.3, 73.5, 73.2, 72.9, 72.5, 72.2, 71.9, 71.2, 69.2, 68.8, 66.5, 66.1, 40.9, 29.7, 29.3, 23.2, 16.5. HRMS: C₆₇H₇₅NO₁₂Na [M+Na]⁺ calcd: 1108.5181 found: 1108.5171.

### General procedure for deprotection of Globo H (204a), tetrasaccharide (203a), and trisaccharide (202a)

Fully protected oligosaccharide was dissolved in acetic acid. Nanosize activated Zn powder (Aldrich) was added, and the reaction was stirred vigorously for one hour. The reaction was filtered and the solvent removed. The crude residue was then dissolved in pyridine and acetic anhydride and a catalytic amount of DMAP added. After stirring overnight, the reaction was quenched with methanol and the solvent removed. The residue was dissolved in CH₂Cl₂; washed with 2% HCl, sat. aq. NaHCO₃, and brine. After removal of solvent, the crude material was then dissolved in methanol (2 ml) and CH₂Cl₂ (2 ml). NaOMe solution was then added and the reaction stirred for 2 hours. The reaction was neutralized with DOWEX 50WX2-200, filtered, and solvent removed. The material was then dissolved in 5% formic acid in methanol, and Pd black was added. The flask was purged three times with hydrogen, and then stirred under an atmosphere of hydrogen overnight. The reaction was neutralized with NH₄0H, filtered through celite, and concentrated. The product was purified by column chromatography (LiChroprep R18, water to 10% MeOH) to give the product as a white solid.

**Compound 204a:** ¹H-NMR (500 MHz, D₂O) δ 5.22 (d, 1H, J=4.04Hz), 4.87 (d, 1H, J=4.03Hz), 4.59 (d, 1H, J=7.71Hz), 4.52 (d, 1H, J=7.70Hz), 4.49 (d, 1H, J=7.70Hz), 4.46 (d, 1H, J=7.07Hz), 4.37 (t, 1H, J=6.4Hz), 4.24-4.18 (m, 2H), 4.08 (d, 1H, J=1.83 Hz), 4.01 (d, 1H, 3.3Hz), 3.99-3.53 (m, 33H), 3.28 (t, 1H, J=8.5Hz), 2.98 (t, 2H, J=7.52Hz), 2.02 (s, 3H), 1.71-1.60 (m, 4H), 1.47-1.40 (m, 2H), 1.19 (d, 3H, J=6.6Hz,). ¹³C-APT NMR (125 MHz, D₂O) δ 175.9, 105.6, 105.0, 103.7, 103.6, 102.1, 100.9, 80.4, 79.9, 78.8, 78.0, 77.4, 77.1, 76.7, 76.4, 76.3, 76.2, 75.2, 74.6, 73.7, 73.5, 72.5, 71.8, 71.7, 71.14, 71.1, 70.8, 70.7, 70.1, 69.7, 69.5, 68.4, 62.6, 62.59, 62.0, 61.7, 53.3, 41.0, 29.8, 28.1, 23.9, 23.7, 17.0 MALDI-FTMS calculated for C₄₃H₇₆N₂O₃₀ [M+Na]+ 1101.4555, found 1101.4525.

**Compound 203a:** ¹H-NMR (600MHz, D₂O) δ5.08 (d, 1H, J=3.96Hz), 4.73 (d, 1H, J=3.96Hz), 4.46 (d, 1H, J=7.44Hz), 4.39 (d, 1H, J=7.44Hz), 4.08 (q, 1H, J=6.54Hz), 4.03 (d, 1H, J=2.7Hz), 3.95 (s, 1H), 3.84-3.72 (m, 5H), 3.70-3.53 (m, 12H), 3.52-3.46 (m, 3H), 3.39-3.35 (m, 1H), 2.85 (t, 2H, J=7.5Hz), 1.89 (s, 3H), 1.58-1.47 (m, 4H), 1.38-1.28 (m, 2H), 1.06 (d, 3H, J=6.6Hz). ¹³C-APT NMR (150MHz, D₂O) δ 175.02, 104.6, 102.7, 99.9, 99.1, 79.3, 77.0, 76.8, 75.7, 75.3, 74.2, 72.5, 72.2, 71.0, 70.2, 69.7, 69.1, 68.7, 68.4, 68.3, 67.4, 61.8, 61.6, 52.3, 40.0, 28.7, 27.1, 23.0, 22.9, 16.0. HRMS: C₃₁H₅₆N₂O₂₀Na [M+Na]⁺ calcd: 799.3318 found: 799.3323

**Compound 202a:** ¹H-NMR (500MHz, D₂O) δ5.14 (d, 1H, J=4.05Hz), 4.51 (d, 1H, J=7.7Hz), 4.22 (d, 1H, J=8.1Hz), 4.13 (q, 1H, J=6.6Hz), 4.01 (d, 1H, J=2.6Hz), 3.90-3.78 (m, 4H), 3.76-3.59 (m, 8H), 3.58-3.50 (m, 3H), 3.47-3.41 (m, 1H), 2.89 (t, 2H, J=7.5Hz), 1.94 (s, 3H), 1.61-1.52 (m, 2H), 1.51-1.42 (m, 2H), 1.35-1.22 (m, 2H), 1.11 (d, 3H, J=6.6Hz). ¹³C-APT NMR (125MHz, D₂O) δ174.3, 103.4, 102.8, 99.8, 77.4, 76.6, 75.7, 75.5, 74.2, 72.5, 70.7, 70.2, 69.8, 69.2, 68.7, 67.5, 61.7, 61.6, 52.1, 40.0, 28.8, 27.0, 22.9, 22.8, 15.9. HRMS: C₂₅H₄₇N₂O₁₅ [M+H]⁺ calcd: 615.2971 found: 615.2976.

### The procedure for deprotection of the disaccharide (201a)

Fully protected disaccharide **214** 190mg was dissolved in 5% formic acid in methanol (3 ml), and Pd black (150 mg) was added. The flask was purged three times with hydrogen, and then stirred under an atmosphere of hydrogen overnight. The reaction was neutralized with NH₄OH, filtered through celite, and concentrated. The product was purified by column chromatography (LiChroprep R18, water to 10% MeOH) to give a white solid **201a** (42.3mg, 59%). ¹H-NMR (500MHz, D₂O) δ5.12 (d, 1H, J=4.05Hz), 4.37 (d, 1H, J=8.05Hz), 4.20 (q, 1H, J=6.6Hz), 3.87-3.77 (m, 2H), 3.76-3.68 (m, 2H), 3.67-3.52 (m, 6H), 3.46 (dd, 1H, J=8.1Hz, 9.5Hz), 2.88 (t, 2H, J=7.7Hz), 1.62-1.51 (m, 4H), 1.37-1.28 (m, 2H), 1.09 (d, 3H, J=6.6Hz). ¹³C-APT NMR (125MHz, D₂O) δ 102.2, 100.1, 77.5, 74.4, 72.5, 70.7, 70.2, 69.6, 69.0, 67.5, 61.6, 40.0, 29.1, 27.2, 22.9, 16.1. HRMS: C₁₇H₃₃NO₁₀Na [M+Na]⁺ calcd: 434.1997 found: 434.1988.

### General procedure for the diazotransfer reaction

Sodium azide (20 equiv.) was dissolved in a minimum volume of water and cooled to 0°C. An equal volume of dichloromethane was added, and trifluoromethanesulfonic anhydride (10 equiv.) was slowly added to the vigorously stirring solution. The reaction was stirred at 0°C for 2 hours. Saturated sodium bicarbonate was then added to quench the reaction. The mixture was extracted twice with dichloromethane. The combined organic layer was washed once with saturated sodium bicarbonate and the solution was used for the next reaction without further purification.

The substrate and 0.1 equiv. CuSO₄ were dissolved in the same volume of water as the volume of triflyl azide solution to be added. Triethylamine (3 molar equiv.) was added to the mixture. The fresh prepared dichloromethane solution of triflyl azide was added at once with vigorous stirring. The methanol was added to obtain the desired 3:10:3 ratio of water:methanol:dichloromethane. The solution was stirred overnight. The reaction was evaporated to a residue and then purified by column chromatography.

**Compound 204b:** ¹H-NMR (600MHz, D₂O) δ5.09 (d, 1H, J=3.96Hz), 4.75 (d, 1H, J=3.96Hz), 4.48 (d, 1H, J=7.86Hz), 4.40 (d, 1H, J=7.44Hz), 4.37 (d, 1H, J=7.44Hz), 4.34 (d, 1H, J=8.28Hz), 4.26 (t, 1H, J=6.6Hz), 4.14-4.07 (m, 2H), 3.97 (br, s, 1H), 3.89 (d, 1H, J=3.06Hz), 3.87-3.74 (m, 7H), 3.73-3.43 (m, 24H), 3.20 (t, 2H, J=6.96Hz), 3.16 (t, 2H, J=8.34Hz), 1.90 (s, 3H), 1.59-1.45 (m, 4H), 1.36-1.24 (m, 2H), 1.08 (d, 3H, J=6.6Hz). ¹³C-APT NMR (150MHz, D₂O) δ175.1, 104.8, 104.1, 102.82, 102.80, 101.2, 100.1, 79.5, 79.1, 77.9, 77.1, 76.9, 76.3, 75.9, 75.6, 75.4, 75.3, 74.4, 73.8, 72.9, 72.7, 71.6, 70.9, 70.3, 70.0, 69.9, 68.8, 68.6, 67.6, 62.2, 61.8, 61.1, 52.4, 51.9, 50.5, 29.1, 28.5, 23.4, 22.8, 16.1. HRMS: C₄₃H₇₄N₄O₃₀Na [M+Na]⁺ calcd: 1149.4280 found: 1149.4215.

**Compound 203b:** ¹H-NMR (600MHz, D₂O) δ5.09 (d, 1H, J=3.5Hz), 4.74 (d, 1H, J=4.0Hz), 4.47 (d, 1H, J=7.4Hz), 4.40 (d, 1H, J=7.4Hz), 4.09 (q, 1H, J=6.54Hz), 4.05 (d, 1H, J=2.22Hz), 3.96 (s, 1H), 3.87-3.73 (m, 5H), 3.71-3.54 (m, 12H), 3.53-3.47 (m, 3H), 3.41-3.36 (m, 1H), 3.20 (dt, 2H, J=7.02Hz, 6.12Hz), 1.90 (s, 3H), 1.58-1.47 (m, 4H), 1.37-1.28 (m, 2H), 1.08 (d, 3H, J=6.6Hz). ¹³C-APT NMR (150MHz, D₂O) δ175.1, 104.6, 102.7, 100.0, 99.1, 79.4, 77.1, 76.8, 75.8, 75.3, 74.2, 72.9, 72.5, 71.1, 70.20, 70.19, 69.8, 69.2, 68.6, 68.3, 67.5, 61.8, 61.7, 52.4, 51.7, 28.8, 28.5, 23.4, 22.9, 16.0. HRMS: C₃₁H₅₄N₄O₂₀Na [M+Na]⁺ calcd: 825.3223 found: 825.3232.

**Compound 202b:** ¹H-NMR (600MHz, D₂O) δ5.10 (d, 1H J=3.48Hz), 4.47 (d, 1H, J=7.44Hz), 4.18 (d, 1H, J=7.86Hz), 4.10 (q, 1H, J=6.6Hz), 3.97 (d, 1H, J=1.74Hz), 3.87-3.73 (m, 4H), 3.72-3.55 (m, 8H), 3.54-3.47 (m, 3H), 3.42-3.37 (m, 1H), 3.23-3.15 (m, 2H), 1.91 (s, 3H), 1.50-1.39 (m, 4H), 1.28-1.19 (m, 2H), 1.08 (d, 3H, J=6.6Hz). ¹³C-APT NMR (150MHz, D₂O) δ174.3, 103.4, 102.8, 99.8, 77.4, 76.6, 75.7, 75.5, 74.2, 72.5, 70.7, 70.2, 69.8, 69.2, 68.7, 67.5, 61.7, 61.6, 52.1, 40.0, 28.8, 27.0, 22.9, 22.8, 15.9. HRMS: C₂₅H₄₄N₄O₁₅Na [M+Na]⁺ calcd: 663.2695 found: 663.2689.

**Compound 201b:** ¹H-NMR (500MHz, D₂O) δ5.12 (d, 1H, J=3.65Hz), 4.3 (d, 1H, J=8.1Hz), 4.21 (q, 1H, J=6.6Hz), 3.82-3.51 (m, 10H), 3.45 (dd, 1H, J=9.55Hz, 8.05Hz), 3.20 (t, 2H, J=6.75Hz), 1.58-1.45 (m, 4H), 1.35-1.24 (m, 2H), 1.08 (d, 3H, J=6.6Hz). ¹³C-APT NMR (125MHz, D₂O) δ102.1, 100.0, 77.2, 75.6, 74.5, 72.5, 70.9, 70.2, 69.6, 69.0, 67.4, 61.6, 51.7, 29.2, 28.5, 23.3, 16.1. HRMS: C₁₇H₃₁N₃O₁₀Na [M+Na]⁺ calcd: 460.1902 found: 460.1899.

### Microarray analysis of Globo H derivatives of 201-204a:

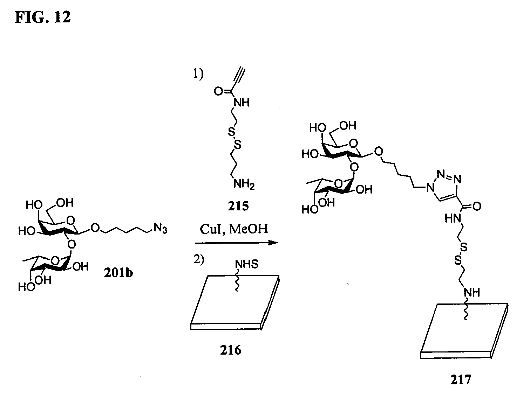

NHS-coated glass slides were spotted with methanolic solutions of sugars **201-204a** with concentrations of 5, 10, 20, 30, 40, 50, 60, 80 and 100 µM from bottom to top with ten duplicates horizontally placed in each grid. This attachment procedure is illustrated in FIG. 12. The slide was washed with PBS buffer for one minute.

Next, 100 µL of a 70 µg/mL solution of MBr1 anti-Globo H monoclonal antibody (IgM) from mouse (Alexis Biochemicals) was formed in 0.05 % tween20 / PBS buffer. This solution was added below the printed grid of sugars and then spread through the application of a coverslip. Incubation in a glass humidifying chamber was performed with shaking for 1 hour. Following this, the slide was washed 5x with 0.05 % tween20 / PBS buffer, 5x with PBS buffer and 5x with water. Next, 200 µL of a 70 µg/mL solution of FITC-tagged goat anti-mouse antibody (Cal Biochem) was formed and added to the slide as before. Humidifying chamber incubation with shaking was performed under foil for 1 hour. Following this, the slide was once again washed 5x with 0.05 % tween20 /PBS buffer, 5x with PBS buffer and 5x with water and then dried with nitrogen. A fluorescence scan was then performed on the slide. The resulting image was analyzed using the program Imagene to locate and quantify the fluorescence of all the spots within the grid. This data was plotted verses the concentration of the solution used for sugar printing to obtain carbohydrate-antibody binding curves.

**Disulfide linker immobilization (of type 217):** The BOC protected derivative of disulfide linker 215 (3.9 mg, 13.5 µmol) was dissolved in 1 mL of dichloromethane and 1 mL trifluoroacetic acid was added. The reaction was allowed to stir for 1 hour and the reaction was then stopped via solvent removal through rotary evaporation. Remaining trifluoroacetic acid was then removed by azeotroping twice with methanol and benzene. A 1 mM solution of the deprotected linker (**215**) was prepared. Samples of the azide-modified sugars were weighed (1 mg for **201b**) and the linker solutions were added to each sugar (2.29 mL for **201b**) such that 1 equivalent of the two starting materials were present. A spatula tip of copper iodide was added and the reactions were stirred overnight. The next day, the methanol was removed and 1 mM stock solutions were formed by adding water. These solutions were spotted on a solid surface and analyzed along side **201-204a**. This reaction is illustrated in FIG. 12.

### Results

Truncated Globo H analogs **201-204** were prepared using the one-pot programmable protocol for oligosaccharide synthesis such that binding to MBr1 could be evaluated using microarray analysis. These analogs contain the saccharide domain of the natural glycolipid with sequentially clipped sugars. Furthermore, pentamine or pentazide linkers were included at the reducing ends for immobilization via covalent linkage to NHS-coated glass slides. The inventors had previously reported the one-pot programmable synthesis of Globo H. Burkhart et al. (2001) Angew. Chem. Int. Ed. 40, 1274-+. A new synthetic strategy was used for this study as described above. Instead of using two one-pot reactions, the entire hexasaccharide was constructed in a single one-pot reaction using a novel [1+2+3] approach. Formation of the most difficult α 1-4-Galactose-Galactose bond in advance improved the yield of the one-pot reaction (83% verses 62% for the previous strategy). The trisaccharide building block is also valuable in the synthesis of all Globo family oligosaccharides.

The synthesis of the tetrasaccharide, trisaccharide, and disaccharide analogs implemented the same building blocks as the full Globo H hexamer. The coupling of trisaccharide **210** to galactose building block **209** or the linker *N*-Cbz-5-hydroxylpentamine gave tetrasaccharide **211** or trisaccharide **212,** respectively, as described above. The coupling of galactose building block **213** and fucose building block **205** gave disaccharide **214** as described above. Deprotection of Globo H hexasaccharide **208**, tetrasaccharide **211**, and trisaccharide **212** began with the removal of the Troc group using activated Zn particles and acetic acid. This was followed by acetylation of the amine group with acetic anhydride and pyridine. The benzoate groups were removed with sodium methoxide in methanol. Final deprotection of the benzyl ether, benzylidene acetal and N-Cbz groups was accomplished using Pd-black in 5% formic acid / methanol under 1 atm hydrogen. This yielded the fully deprotected Globo H hexasaccharide **204a**, tetrasaccharide **203a** and trisaccharide **202a.** Deprotection of disaccharide **214** through treatment with Pd-black in 5% formic acid in methanol as described above gave compound **201b.** The diazotransfer reaction (triflyl azide with copper sulfate catalyst) was used to convert the amine groups of deprotected oligosaccharides **204a, 203a, 202a, 201a** to the corresponding azido-sugars **204, 203, 202** and **201,** respectively.

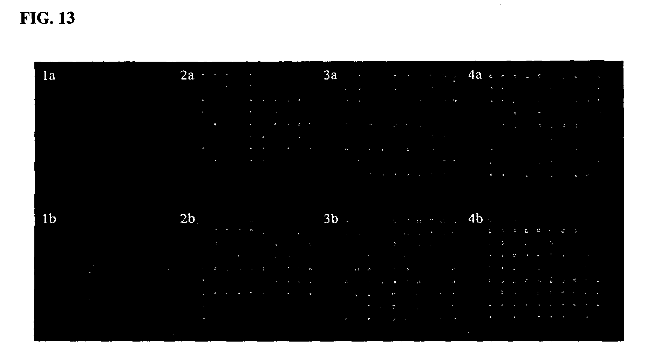

Following their synthesis, the antibody binding abilities of Globo H analogs **201-204** were studied within the microarray platform. Amino-functionalized Globo H analogs **201-204a** were directly immobilized onto NHS-coated glass slides. For azido-analogs **201-204b**, the disulfide linker strategy was implemented for surface attachment. The azides, such as **201b** (FIG. 12), were combined with disulfide linker **215** via the 1,3-dipolarcycloaddition reaction followed by spotting onto the NHS microplate (**216**) for immobilization to **217**. Sugars were spotted in a range of concentrations to allow for antibody binding curve generation. The assay involved initial treatment with monoclonal mouse IgM antibody MBr1, followed by incubation with a fluorescein-tagged secondary antibody, goat anti-mouse IgM, for detection. Scanning the slide for fluorescence yielded images such as the one displayed in FIG. 13, in which the binding of the antibody to printed oligosaccharide spots could be directly observed. The slides contained sugars printed in grids with **201a-204a** in the top row from left to right and **201b-204b** in the bottom row. Initial visual analysis indicated that the shorter oligosaccharides show weaker recruitment of the antibody to the plate surface.

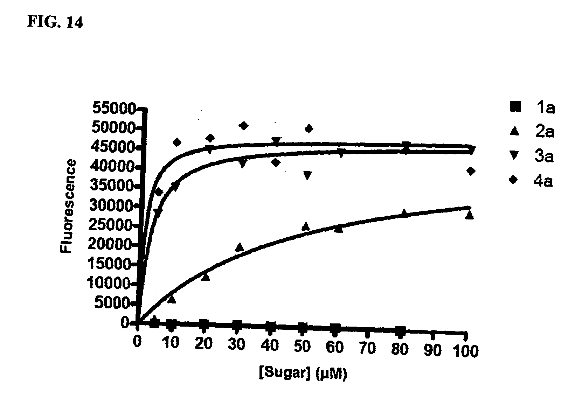

Images such as the one shown in FIG. 13 could then be processed using the program Imagene to encircle and quantify the fluorescence of each spot. The resulting data was plotted verses the concentration of sugar to which each location was subjected during spotting. This yielded binding curves for the different carbohydrate-antibody interactions as indicated in FIG. 14 for **201a-204a.** Amino-derivatized oligosaccharide analogs **201a-204a** yielded higher antibody-recruitment properties than the azide containing moieties (**201b-204b**) immobilized via the disulfide linker. This could be due to poor solubility of linker containing sugars, lack of full conversion in linker attachment or, simply, that the shorter linker is more suited to binding. The assay results showed that antibody binding generally increases with the complexity of the oligosaccharide structure. Disaccharide Globo H derivatives **201a** and **201b** produced no recruitment of antibody to the surface. Trisaccharides **202a** and **202b** bound antibody, but not to the point where they could compete with the full natural hexasaccharides **204a** and **204b**. Tetramers **203a** and **203b**, however, displayed similar binding on the surface to the full natural hexamers, indicating that the following tetrasaccharide core structures are effective for binding the antibody. 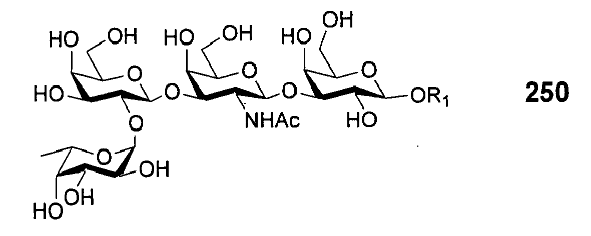 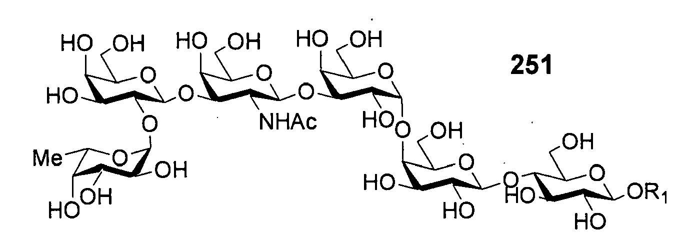 wherein: R₁ is hydrogen, a glycan or a linker. In some embodiments, the linker is or can be attached to a solid support.

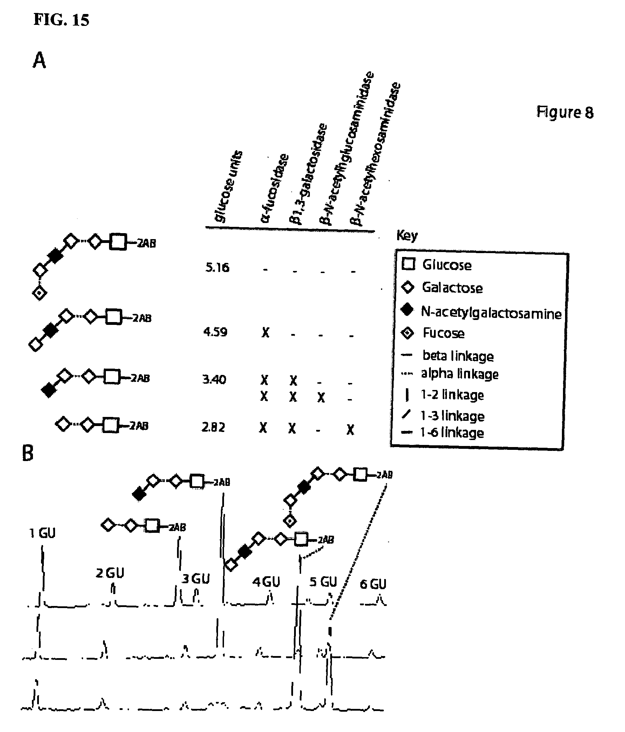

In further characterization of the Globo H oligosaccharide epitope, an analytical sequencing was used for the purpose of structure confirmation. For this purpose, a Globo H derivative containing a fluorescent tag was prepared. This was then subjected to various digestions by the endoglycosidases (FIG. 15A) α-fucosidase (bovine kidney, Sigma), b-1,3-galactosidase (recombinant from E. coli, Calbiochem), b-N-acetylglucosaminidase (recombinant from Streptococcus pseumoniae, Prozyme), and b-N-acetylhexosaminidase (from Jack Bean, Prozyme). The resulting digests were next subjected to HPLC analysis with fluorescence detection (FIG. 15B). The glycan fragments obtained from digestion were as expected and confirm the structure of the Globo H antigen.

Thus, this study illustrates the efficacy of this approach for obtaining structural information pertaining to natural ligands which are involved in important biological processes.

These studies also expand upon the understanding of the oligosaccharide epitope found on the crucial glycosphingolipid Globo H and its interaction with MBr1 antibody. Thus, they should assist in the advancement of anti-cancer vaccine development. One aspect of this pursuit has involved the display of Globo H on a scaffold for multivalent presentation in order to yield an innate immune response in patients. Towards this end, it is beneficial to facilitate the synthesis of the immunogen such that the cost and efficiency of production are decreased and increased, respectively.

Simplified Globo H tetrasaccharide **203** shows similar binding affinity to **204** in multivalent format, while the synthetic route to this compound is shorter. As a result, this derivative shows great promise for the efficient development of an anti-cancer vaccine and for diagnostic methods. The advancement of cancer therapy will require an arsenal of tools for understanding and treating the disease. This has become more vital due to the recent reports of cancer stem cells and the promise and challenges exhibited by this field. The sequencing and microarray techniques presented herein represent effective methods for rapid characterization of processes pertaining to cancer onset at the molecular level.

### EXAMPLE 10: Preliminary Studies Indicate that 2G12 Anti-HIV Antibodies

### Preferentially Bind Man8 Glycans.

This Example describes preliminary experiments indicating that glycans bound by the 2G12 anti-HIV antibody include Man8 glycans. The 2G12 antibody is a broadly neutralizing antibody that was initially observed to bind (Man9GlcNAc2) (see Calarese et al. Science 2003, 300, 2065-2071).

### Materials and Methods:

The natural high mannose type N-glycans used for the analysis were purified from pronase treated bovine ribonuclease B on Dionex. Each preparation was a single molecular weight species as determined by MALDI-MS.

*Construction of a glycan array printed on a glass slide:* The library of carbohydrate structures was obtained through chemical and chemo-enzymatic synthesis as described above, as well as natural sources. This compound library contained a *N*-hydroxy succinimide (NHS)-reactive primary amino group, which was printed on a commercial NHS-activated glass surface (Accelr8 Technology Corporation, Denver, CO) using a microarray gene printer (TSRI).

Each glycan type was printed (≈0.5nL/spot) at various concentrations (10-100 µM) and each concentration in a replicate of six. Slides were further incubated with ethanolamine buffer to deactivate remaining NHS functional groups.

*Cleavable Linker Glycan Array in Microtiter Plates:* Further experiments were performed using glycans immobilized within microtiter wells with the cleavable triazole linkers described in Example 7 as shown in FIG. 11.

*2G12 Binding:* After washing with QH₂O, wells were blocked with 10 mM HEPES buffer, pH 7.5/150 mM NaCl buffer (200 µL) containing 0.1% Tween-20 over 1 h at 4 °C. The buffer was then removed and 2G12 (17 µg/mL PBS buffer; 200 µL) was incubated in the well over 1 h at 4°C. Wells were then washed with PBS buffer (3×; 200 µL) and fluorescein-conjugated Goat Anti-Human IgG antibody (14 µg/mL PBS buffer; 200 µL) was incubated in the well over 1 h in the dark at 4 °C. Wells were then washed with PBS buffer (3×; 200 µL) and fluorescence was measured with an excitation wavelength of 485 nm and emission wavelength of 535 nm.

### Results:

The 2G12 antibody was pre-complexed (for 10 min) with secondary human anti-IgG-FITC (2:1, 20µg/mL) prior to application to the glycan array. After incubation with the 2G12 antibodies, the array was washed by dipping the slide in buffer and water.

Binding was observed in initial experiments to several synthetic mannose-containing oligosaccharides and to isolated and purified Man-8 N-glycans. The glycans to which the 2G12 antibodies bound had any the following glycan structures, or were a combination thereof: 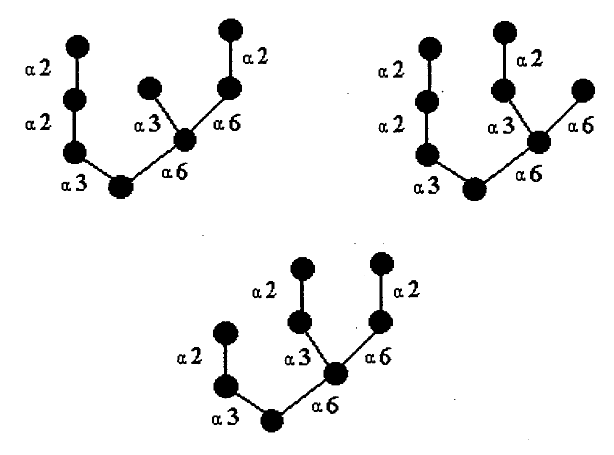 wherein each filled circle (•) represents a mannose residue.

A smaller level of binding was observed between the 2G12 antibodies and Man-9-N-glycans. As shown in Table 7, simpler synthetic glycans bind 2G12 as well as the Man8 glycans. However, the simpler compounds are more likely to elicit an immune response that will generate antibodies to the immunogen, but not the high mannose glycans of the gp120. The natural structure is also less likely to produce an unwanted immune response. Indeed, yeast mannan is a polymer of mannose and is a potent immunogen in humans, representing a major barrier to production of recombinant therapeutic glycoproteins in yeast.

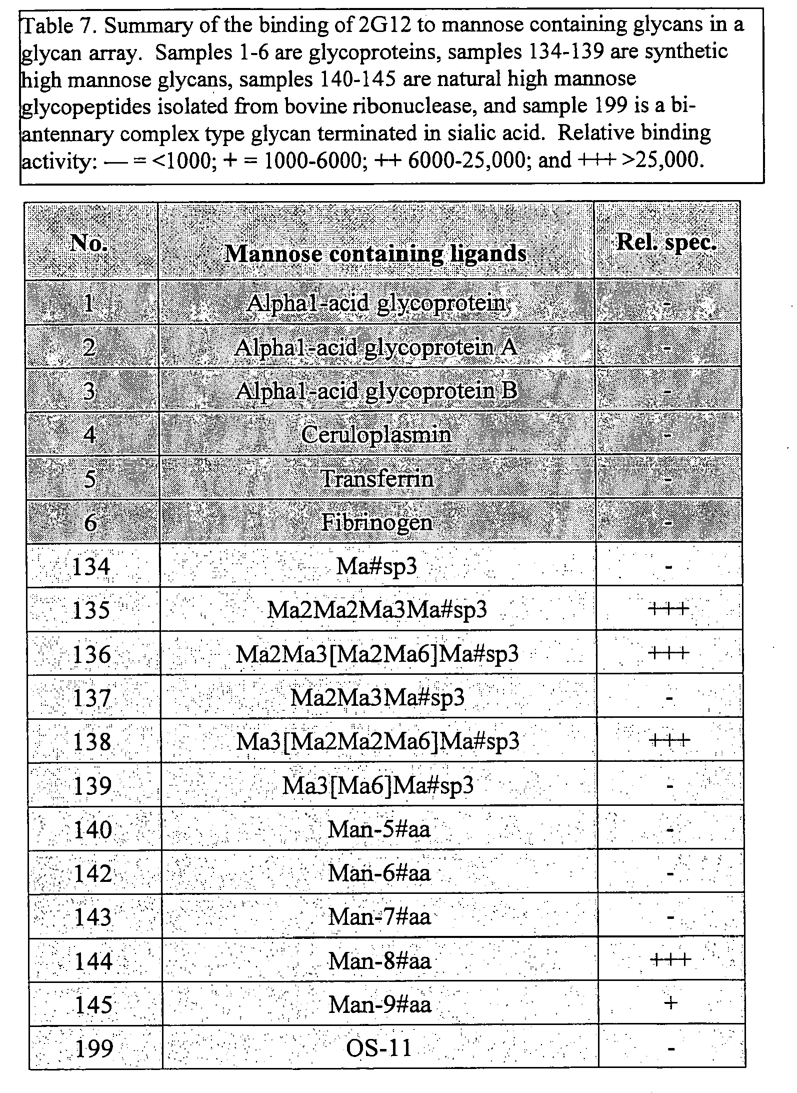

These results indicate that glycans with eight mannose residues are superior antigens for binding the 2G12 anti-HIV neutralizing antibodies.

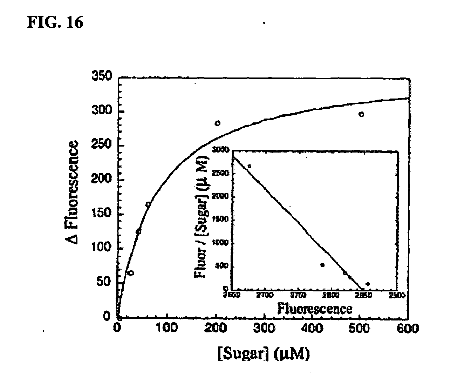

Further studies were performed using a cleavable linker array to clarify the types of mannose-containing glycans bound by the 2G12 anti-HIV antibodies. These cleavable linker studies demonstrated that glycans containing Manα1,2 Manα1,2 Manα1,3Man and/or Manα1,2Manα1,3Manα1,2 Manα1,6Man were the optimal epitope(s) with micromolar affinity to 2G12. The results of a binding study using increasing amounts of labeled Manα1,2Manα1,3Manα1,2 Manα1,6Man glycan with a constant amount of 2G12 antibody are shown in FIG. 16. The *K*_{d} values for binding of structurally related mannose-containing glycans with the 2G12 antibody were observed to be as follows.

Manα1,2Manα1,3Manα1,2 Manα1,6Man: *K*_{d} = 0.1 µM.

Manα1,2 Manα1,2 Manα1,3Man: *K*_{d} = 0.1 1µM.

Manα1,2Manα1,2Manα1,3(Manα1,2Man1,2Man1.6)Man: *K*_{d} = 0.7 µM.

Manα 1,2Manα1 ,2Manα1 1,3(Manα1,2Man1,3 (Manα1.2Manα1 ,2Manα 1.6) )Man: *K*_{d} = 1.0 µM.

These studies identified glycans Manα1,2 Manα1,2 Manα1,3Man and Manα1,2Manα1,3Manα1,2 Manα1,6Man as the optimal epitope(s) with micromolar affinity to 2G12. This result further illustrates the utility of the glycoarray of the invention.

### EXAMPLE 11: Dissection of the Carbohydrate Specificity of 2G12 Antibodies

Novel antigens, oligomannoses **7**,**8** and **9** (shown below and in FIG. 17) were synthesized and the ligand specificity of 2G12 was probed by studying the ability of these oligomannoses to (i) inhibit the binding of 2G12 to gp120 in solution phase ELISA assay (4) and (ii) bind 2G12 in microtiter plate-based or glass-slide assays.

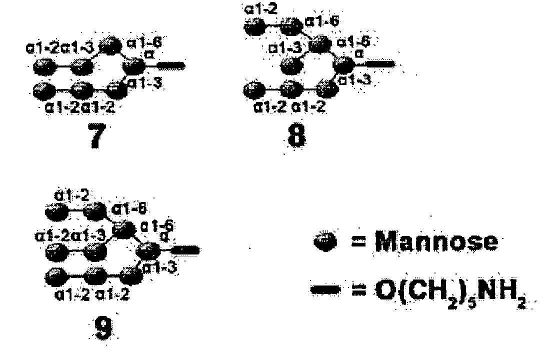

In addition, the crystal structures of Fab 2G12 bound to four of these synthetic oligomannoses (**4**, **5**, **7**, and **8** FIG. 17) was determined. These biochemical, biophysical, and crystallographic results reveal that Fab 2G12 can recognize the terminal Manα1-2Man of both the D1 and D3 arms of Man₉GlcNAc₂. These data confirm that 2G12 is highly specific for terminal Manα1-2Man, but in the context of a broader range of linkages to the third position of the oligomannose moieties than previously thought, which may expedite development of a carbohydrate-based immunogen that could contribute to an HIV-1 vaccine.

### Materials and Methods:

**Oligomannose Synthesis.** All chemicals were purchased from Aldrich and used without further purification. Building blocks **10** and **13** were synthesized as described in Lee et al. (2004) Angew. Chem. Int. Ed. Engl. 43: 1000-1003. Experimental details for the synthesis of the key thioglycoside building blocks (**12, 16** and **19**)**,** the protected Man₇ **14,** Man₈ **17** and Man₉ **20,** the unprotected Man₇ 7, Man₈ **8** and Man₉ **9,** the remaining reaction intermediates **11, 15** and **18,** and all the characterization data for **7-9, 11, 12** and **14-25** are provided as described below.

**Enzyme-linked immunosorbent assay.** Microtiter plate wells (flat bottom, Costar type 3690; Corning Inc.) were coated with 50 ng/well gp120_{JR-CSF} overnight at 4°C. All subsequent steps were performed at room temperature. The wells were then washed four times with PBS/0.05% (vol/vol) Tween20 (Sigma) using a microplate washer (SkanWash 400, Molecular Devices) prior to blocking for 1 hr with 3% (mass/vol) BSA. IgG 2G12, diluted to 0.5 µg/mL (25 ng/well) with 1% (mass/vol) BSA/0.02% (vol/vol) Tween20/PBS (PBS-BT), was then added for 2 hrs to the antigen-coated wells in the presence of serially-diluted oligomannoses starting at 2mM. Unbound antibody was removed by washing four times, as described above. Bound 2G12 was detected with an alkaline phosphatase-conjugated goat anti-human IgG F(ab')₂ antibody (Pierce) diluted 1:1000 in PBS-BT. After 1hr, the wells were washed four times and bound antibody was visualized with *p-*nitrophenol phosphate substrate (Sigma) and monitored at 405 nm.

**Carbohydrate microarray analysis.** Ninety-six well NHS-coated microtiter plates (NoAb Biodiscoveries) were treated with 200 µL of a 1 mM methanolic solution of the amino-functionalized disulfide and alkyne containing linker (scheme V) containing 5 % diisopropylethylamine (DIEA) and incubated overnight at 4°C. The microtiter plate was then washed with 2 x 200 µL methanol and 2 x 200 µL water. Next, 200 µL solutions of azido-functionalized oligomannose derivatives **21-25** at varying concentrations from 0 to 500 µM in 5 % DIEA / methanol were introduced. A sprinkle of copper (I) iodide was added and the contents were allowed to react overnight at 4°C. The next day, the contents were removed and the plates were washed with 2 x 200 µL methanol and 2 x 200 µL water. The plates were then blocked with 0.1 % Tween20 solution in HEPES buffer pH 7.5 at 4 °C for 1 hour and then washed with 3 x 200 µL HEPES buffer. Next, 200 µL of a 1 µg/mL solution of 2G12 antibody in 0.1 % Tween20 / PBS buffer was added to the wells for a 1 hour incubation at 4 °C and then washed with 2 x 200 µL PBS buffer. At this point, 200 µL of FITC-tagged goat anti-human IgG antibody (10 µg/mL in PBS, Cal Biochem) was added for 1 hour at 4 °C, and the wells were then washed with 2 x 200 µL PBS buffer. Detection of the FITC-tagged secondary antibody was performed in 200 µL water using a fluorescence plate reader. The resulting data yielded the oligomannose-2G12 binding isotherms and Scatchard plot analysis was implemented in the determination of dissociation constants. Adams et al. (2004) Chem. Biol. 11: 875-81.

**2G12 purification, crystallization, structure determination, and analysis**. Human monoclonal antibody 2G12 (IgG1, κ) was produced by recombinant expression in Chinese hamster ovary cells. Fab fragments were produced by digestion of the immunoglobulin with papain followed by purification on protein A and protein G columns, and then concentrated to ~30 mg/ml. For each complex (Man₄, Man₅, Man₇, and Man₈), the solid sugar ligand was added to the Fab solution to saturation. For crystallization, 0.6 µl of protein + sugar were mixed with an equal volume of reservoir solution. All crystals were grown by the sitting drop vapor diffusion method with a reservoir volume of 1mL. Fab 2G12 + Man₄ crystals were grown with a reservoir solution of 27% PEG 4000 and 0.05 M sodium acetate, Man₅ co-crystals with 1.6 M Na/K Phosphate, pH 6.8, Man7 co-crystals with 20% PEG 4000 and 0.2 M sodium tartrate, and Man8 co-crystals with 20% PEG 4000 and 0.2 M imidizole malate pH 7.0. All crystals were cryoprotected with 25% glycerol. Data were collected at 100K at the Advanced Light Source (ALS) beamline 8.2.2, and Stanford Synchotron Radiation Laboratory (SSRL) beamlines 9-2 and 11-1. All data were indexed, integrated, and scaled with HKL2000 (25) using all observations > -3.0 σ.

The structures were determined by molecular replacement using the 1.75 Å structure of Fab 2G12 (PDB code: 1 OP3) as the starting model for Phaser. Li & Wang (2004) Org. Biomol. Chem. 2: 483-88; Storoni e al. (2004) Acta Cryst. D60: 432-38. The Matthews' coefficients of the asymmetric unit suggested that the Fab 2G12 + Man4 data contained a single Fab + sugar complex, while the asymmetric unit of the other complexes consisted of two Fab + sugar complexes. The model building was performed using TOM/FRODO (Jones (1985) Methods Enzymol. 115:157-71), and refined with CNS version 1.1 (Brunger et al. (1998) Acta Cryst. D54: 905-21) and REFMAC using TLS refinement (CCPA Acta Cryst. D50: 760-763), using all measured data (with *F* > 0.0 σ). Tight noncrystallographic symmetry (NCS) restraints were applied initially, but released gradually during refinement. An Rfree test set (5%) was maintained throughout the refinement. Data collection and refinement results are summarized in Table 8.

**Table 8**

| | Fab 2G12 + Man4 | Fab 2G12 + Man5 | Fab 2G12 + Man7 | Fab 2G12 + Man8 |
|---|---|---|---|---|
| Space Group | C222 | P2₁2₁2₁ | P2₁2₁2₁ | P2₁2₁2₁ |
| Unit cell dimensions (Å) | *a* = 144.6, | *a* = 44.9, | *a=* 44.8, | *a* = 45.2, |
| | *b* = 148.3, | *b =* 131.8, | *b =* 131.1, | *b =* 165.7, |
| | *c* = 54.6 | *c* = 170.3 | *c* = 170.0 | *c* = 169.6 |
| Resolution *(Å) | 30-2.0 (2.05-2.0) | 50-2.75 (2.86-2.75) | 50-2.33 (2.39-2.33) | 50-2.85 (2.93-2.85) |
| No. of observations | 130,911 | 152,645 | 294,916 | 119,281 |
| No. of unique reflections | 37,831 | 32,006 | 43,875 | 33,657 |
| Completeness (%) | 94.3 (88.0) | 89.3 (82.1) | 99.5 (98.1) | 99.5 (99.9) |
| *R*_{sym} (%) | 8.5 (57.6) | 8.5 (37.6) | 5.3 (41.9) | 10.2 (52.9) |
| Average *Ilσ* | 16.4 (2.4) | 23.5 (4.0) | 45.1 (3.8) | 13.0 (2.4) |
| *R*_{cryst} (%) | 28.1 (33.7) | 22.2 (34.8) | 20.9 (34.7) | 22.0 (44.6) |
| *R*_{free} (%) | 32.6 (40.9) | 28.6 (51.6) | 25.1 (40.8) | 27.7 (48.8) |
| No. of refined atoms Fab/ligand/water | 3206/45/121 | 6468/79/- | 6463/93/77 | 6447/88/- |
| <*B*> values (Å²) | | | | |
| Variable domain ½ | 46.2 | 33.7/48.4 | 47.8/53.3 | 44.4/45.2 |
| Constant domain ½ | 72.7 | 49.3/43.9 | 67.8/56.5 | 82.0/68.5 |
| Ligand | 32.9 | 52.0 | 71.9 | 43.1 |
| Ramachandran plot (%) | | | | |
| Most favored | 90.6 | 80.5 | 88.1 | 83.4 |
| Additionally allowed | 8.6 | 16.9 | 10.8 | 14.7 |
| Generously allowed | 0.0 | 1.9 | 0.7 | 0.8 |
| Disallowed⁺ | 0.8 | 0.7 | 0.4 | 1.1 |
| Rms deviations | | | | |
| Bond lengths (Å)/ Angles (*) | .016/1.8 | .019/2.0 | .017/1.7 | .018/1.9 |
| * Numbers in parentheses are for the highest resolution shell. | | | | |
| + Includes residue L51 of each light chain, which commonly exists in a γ turn in all antibodies, but is flagged by PROCHECK as an outlier. | | | | |
| Other residues designated as disallowed by PROCHECK have a good fit to the corresponding electron density. | | | | |

Diffraction data for Fab 2G12 in complex with Man₄ suffered from severe anisotropy despite the 2.0 Å diffraction limit. Although we report on the measured data observed to 2.0 Å (*I*/σ > 2.0 and a completeness of 88% in the highest resolution shell of 2.05 - 2.00 Å), anisotropic diffraction, which is significant beyond 2.75 Å, leads to modest *R* values. However, the electron density is very well defined and more easily interpretable at this resolution. Refinement of the Fab 2G12 + Man4 structure at a lower resolution of 2.75 Å yields slightly better R_{crys} and R_{free} values of 21.9% and 28.2%, respectively, but with significantly poorer quality electron density maps. Thus, the higher resolution structure is reported.

Potential hydrogen bonds and van der Waal contacts were evaluated using the program CONTACSYM (Sheriff et al. (1987) J. Mol. Biol. 197: 273-96). Buried molecular surface areas were measured using the program MS (Connolly (1993) J. Mol. Graphics 11: 139-141).

### Results and Discussion

### Synthesis of Man₇ 7, Man₈ 8 and Man₉ 9.

The synthetic steps for making Man₇ **7** are shown in Scheme V below and were performed according to literature procedures (Schmidt et al. (1990) Synletters 694-96).

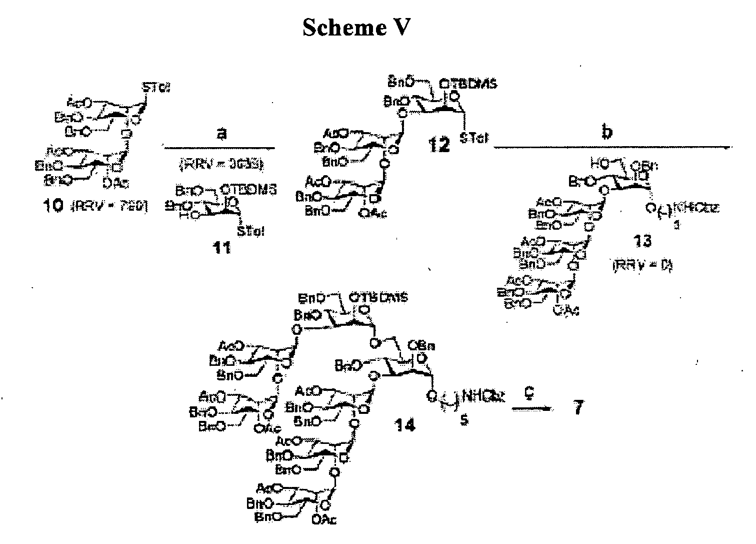

Reagents and conditions employed for Scheme V: step a: (i) NBS, Acetone, 0°C, 30 mins; (ii), CCl₃CN, DBU, CH₂Cl₂, 0°C, 8h; (iii), **11**, TBDMSOTf, Et₂O, -40°C, 4h, 75% over three steps; step b: **13** NIS/TfOH, MS, CH₂Cl₂, -45°C, 2h, 85%; step c: (i) TBAF/AcOH, THF, rt, 2h; (ii) NaOMe, MeOH, rt, 48h; (iii) Pd black, HCOOH/MeOH (20:1 v/v), H2, rt, 24h, 60% over three steps.

Thioglycoside disaccharide building block **10** was converted to its trichloroacetimidate derivative, which was activated with TBDMSOTf for glycosylation with building block **11** to give trisaccharide building block **12** in good yield (75% over 3 steps). Convergent synthesis of Man₇ **7** in good yield (85%) was achieved by glycosylation of tetrasaccharide acceptor **13** with trisaccharide donor **12** using the NIS/TfOH promoting system in anhydrous CH₂Cl₂ at -25°C. Excellent Manα1-6Man selectivity was controlled by the presence of the TBDMS group, at the 2-position of trisaccharide donor **12.** Global deprotection of protected Man₇ **14** was achieved smoothly through desilylation with TBAF/AcOH buffer (Geng et al. (2004) Angew. Chem. Int. Ed. Engl. 43: 2562-65), deacetylation, and hydrogenolysis to afford unprotected Man₇ **7** (60% in 3 steps).

Using a similar strategy, syntheses of Man₈ **8** and Man₉ **9** were performed as depicted in Scheme VI.

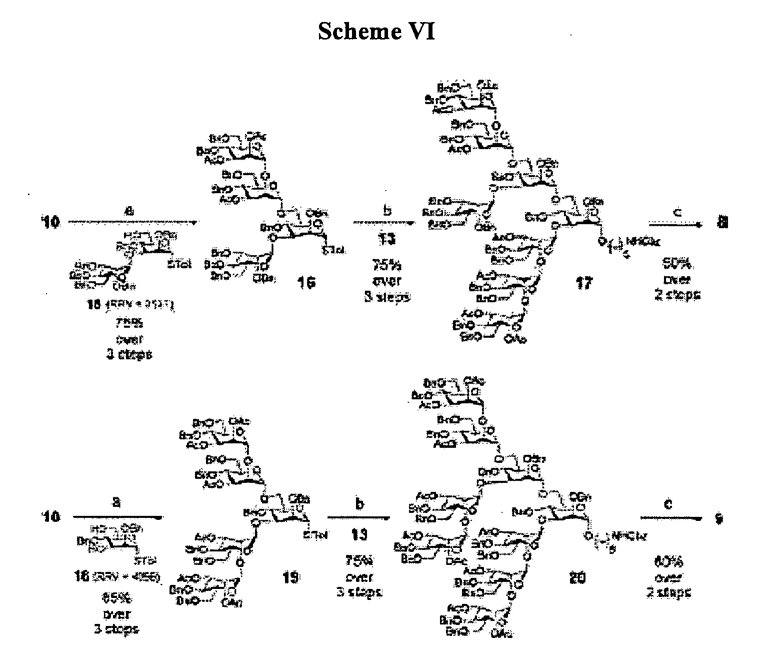

Reagents and conditions employed for Scheme VI: step a: (i) NBS, Acetone, 0°C, 30 mins; (ii), CCl₃CN, DBU, CH₂Cl₂, 0°C, 8h; (iii), **14** or **18**, TBDMSOTf, Et₂O, -60 °C, 4h; step b: (i) NBS, Acetone, 0°C, 30 mins; (ii), CCl₃CN, DBU, CH₂Cl₂, 0°C, 8h; (iii), **13**, TBDMSOTf, Et₂O, -40°C, 4h; c, (i) NaOMe, MeOH, rt, 48h; (ii) Pd black, HCOOH/MeOH (20:1 v/v), H₂, 24h.

Thioglycoside disaccharide building block **10** was converted to its trichloroacetimidate derivative, which was activated with TBDMSOTf in anhydrous Et₂O at -50 °C and glycosylated using building block **15** (1.1 equiv.) or **18** (0.45 equiv.) to give the tetrasaccharide building block **16** (75% over 3 steps) and pentasaccharide building block **19** (65% over 3 steps) in good yield. In the convergent synthesis of Man₈ **8** and Man₉ **9**, control of the Man1α-6Man selectivity was a problem due to the lack of steric bulk or neighboring participating group at the 2-position of thioglycoside tetrasaccharide donor **16** and pentasaccharide donor **19**. Finally, the Manα1-6Man selectivity was controlled by implementing Seeberger's protocol (Ratner et al. (2002) Eur. J. Org. Chem. 5: 826-33). Thioglycoside tetrasaccharide **16** and pentasacharride **19** were converted to the corresponding trichloroimidates, which were coupled to tetrasaccharide **13** using TBDMSOTf in anhydrous Et₂O at-40°C to give protected Man₈ **17** (75% over 3 steps) and Man₉ **20** (75% over 3 steps) in good yield. Global deprotection of protected Man₈ **17** and Man₉ **20** was achieved through deacetylation and hydrogenolysis to afford unprotected Man₈ **8** (60% in 2 steps) and Man₉ **9** (60% in 2 steps).

### Solution Phase ELISA Assay Analysis of Oligomannose 1-8 Inhibition of 2G12

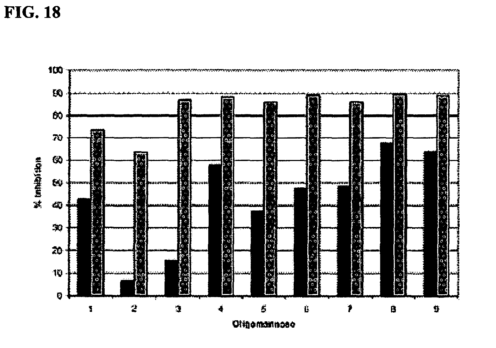

**Binding.** Man₉GlcNAc₂ **1** and deprotected oligomannoses **2-6** and **7-9** (see FIG. 17) were evaluated for their ability to inhibit the interaction between 2G12 and gp120 in a solution phase enzyme-linked immunosorbent assay (ELISA). These results (FIG. 18) confirmed that terminal Manα1-2Man is critical for binding. All of the oligomannoses that contain a Manα1-2Manα1-2Man motif (which corresponds to the D1 arm of Man₉GlcNAc₂ shown in FIG. 17) are capable of inhibiting 2G12 binding at similar levels to the intact Man₉GlcNAc₂ moiety. However, 2G12 does not readily recognize Manα1-2Manα1-3Man, as oligomannose **3** does not inhibit effectively at lower concentrations (15.8% at 0.5 mM). Oligomannose **5**, which is similar to oligomannose **3**, but contains the Manα1-2Manα1-6Man motif, is capable of inhibition (37.7% at 0.5mM). These results suggest that 2G12 recognizes Manα1-2Man in the context of the D1 arm (Manα1-2Manα1-2Man) or the D3 arm (Manα1-2Manα1-6Man), but not the D2 arm (Manα1-2Manα1-3Man).

Overall, many of the oligomannose derivatives can compete for binding of Man₉GlcNAc₂, and, therefore, may serve as building blocks for potential immunogens to elicit 2G12-like antibodies.
**Carbohydrate Microarray Analysis.** Previous studies by the inventors using covalent microtiter plates with a panel of carbohydrate epitopes for interaction with 2G12 involved converting the amine-containing oligomannoses **4**, **5**, **7**, **8** and **9** to the corresponding azide derivatives **21**, **22**, **23**, **24** and **25**. These derivatives were then covalently attached to a microplate-immobilized cleavable linker via the Cu (I)-catalyzed 1,3-dipolar cycloaddition reaction (FIG. 11B). *K*_{d} values for the interaction of 2G12 with oligomannoses **4**, **5**, **7**, **8**, **9** (Table 9) were determined using a microtiter-based assay with detection via a fluorescent secondary antibody (see Bryan et al.(2004) J. Am. Chem. Soc. 126, 8640-41).

**Table 9: The Kd values of oligomannoses 1 and 4, 5, 7, 8, 9 binding to 2G12, as determined by carbohydrate microarray analysis.**

| **Oligomannose** | **4** | **5** | **7** | **8** | **9** |
|---|---|---|---|---|---|
| *K*d (µM) | 0.1 | 0.1 | 0.7 | 1.3 | 1.0 |

These results indicate that oligomannose 4 and oligomannose 5 bind 2G12 antibodies with the greatest affinity. The structures for these oligomannose glycans are provided below.

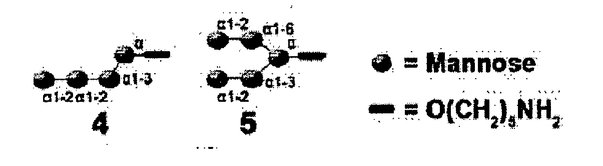

The result of binding analysis on microtiter plate arrays is consistent with that on glass-slide arrays. As expected, the specific spatial orientation of the epitopes on the surface is crucial for binding to the 2G12 antibody. Significant enhancement of the binding affinity of these compounds in microarray studies may be explained by multivalent interactions of the oligomannoses with 2G12 that mimic the cluster of oligomannoses on the surface of gp120. The smaller oligomannose derivatives especially benefit from multivalent display. Thus, this system may be an effective model for studying binding events involving carbohydrates presented on a surface, such as that of a virus.

Overall, the carbohydrate specificity of 2G12 is less restrictive than initial studies may have indicated. Calarese et al. (2003) Science 300, 2065-71. The combined biochemical, biophysical, and crystallographic evidence clearly indicate that 2G12 can bind to the Manα1-2Man at the termini of both the D1 and D3 arms of an oligomannose sugar. In the Man₄, Man₇, and Man₈ crystal structures, 2G12 interacts with the D1 arm, while in the Man₅ and Man₈ crystal structures, the D3 arm also binds in the combining site. Therefore, 2G12 can bind not only the D1 arms from two different N-linked oligomannoses on gp120, but also to both the D1 and D3 arms from different sugars within the oligomannose constellations on gp120. This mode of recognition would enhance binding to a cluster of oligomannose moieties, and relax the constraint of an exact match of the oligomannose moieties with respect to the multivalent binding site of the antibody. Nevertheless, despite this increased potential for multivalent interaction, 2G12 is highly restricted to oligomannose cluster binding on gp120, as no significant binding to "self' proteins has been observed.

The 2G12 antibody can neutralize a broad range of HIV-1 isolates. The results presented here reveal more precisely the carbohydrate specificity of this antibody. This deeper understanding of the 2G12-oligomannose interaction can now be applied to carbohydrate-based immunogen design, as the nature of the mannose building blocks needed to design a multivalent oligomannose presentation for immunization trials has been established.

### References:

Calarese, D. A., Scanlan, C. N., Zwick, M. B., Deechongkit, S., Mimura, Y., Kunert, R., Zhu, P., Wormald, M. R., Stanfield, R. L., Roux, K. H., et al. (2003). Antibody domain exchange is an immunological solution to carbohydrate cluster recognition. Science 300, 2065-2071.
Lee, H. K., Scanlan, C. N., Huang, C. Y., Chang, A. Y., Calarese, D. A., Dwek, R. A., Rudd, P. M., Burton, D. R., Wilson, I. A., and Wong, C. H. (2004). Reactivity-Based One-Pot Synthesis of Oligomannoses: Defining Antigens Recognized by 2G12, a Broadly Neutralizing Anti-HIV-1 Antibody. Angew Chem Int Ed Engl 43, 1000-1003.
Sanders, R. W., Venturi, M., Schiffner, L., Kalyanaraman, R., Katinger, H., Lloyd, K. O., Kwong, P. D., and Moore, J. P. (2002). The mannose-dependent epitope for neutralizing antibody 2G12 on human immunodeficiency virus type 1 glycoprotein gp120. J Virol 76, 7293-7305. Scanlan, C. N., Pantophlet, R., Wormald, M. R., Ollmann Saphire, E., Stanfield, R., Wilson, I. A., Katinger, H., Dwek, R. A., Rudd, P. M., and Burton, D. R. (2002). The broadly neutralizing anti-human immunodeficiency virus type 1 antibody 2G12 recognizes a cluster of alpha1-->2 mannose residues on the outer face of gp120. J Virol 76, 7306-7321.
Tremblay, L. O., and Herscovics, A. (2000). Characterization of a cDNA encoding a novel human Golgi alpha 1, 2-mannosidase (IC) involved in N-glycan biosynthesis. J Biol Chem 275, 31655-31660.
Trkola, A., Purtscher, M., Muster, T., Ballaun, C., Buchacher, A., Sullivan, N., Srinivasan, K., Sodroski, J., Moore, J. P., and Katinger, H. (1996). Human monoclonal antibody 2G12 defines a distinctive neutralization epitope on the gp120 glycoprotein of human immunodeficiency virus type 1. J Virol 70, 1100-1108.
Vallee, F., Karaveg, K., Herscovics, A., Moremen, K. W., and Howell, P. L. (2000). Structural basis for catalysis and inhibition of N-glycan processing class I alpha 1,2-mannosidases. J Biol Chem 275, 41287-41298.

### Summary

Summary embodiments of the invention will now be described in the following paragraphs.
1. An array of molecules comprising a library of molecules attached to an array through a cleavable linker, wherein the cleavable linker has the following structure:

   X-Cv -Z

   wherein:
   Cv is a cleavage site;
   X is a solid surface, a spacer group attached to the solid surface or a spacer group with a reactive group for attachment of the linker to a solid surface; and
   Z is a reactive moiety for attachment of a molecule, a spacer group with a reactive moiety for attachment of a molecule, a spacer group with a molecule, or a molecule attached to the linker via a linking moiety.
2. The array of paragraph 1, wherein the cleavable linker is chemically cleavable, photocleavable, or enzymatically cleavable.
3. The array of paragraph 1, wherein the linker is a photocleavable linker comprising either formula **IVa** or **IVb:** 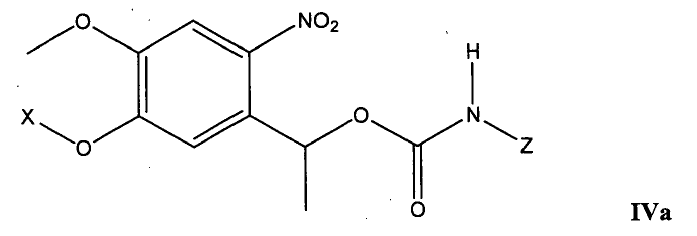 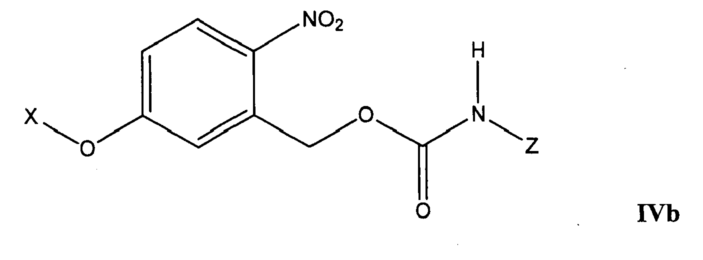
4. The array of paragraph 1, wherein the linker is a disulfide linker that has the following structure:

   X-S-S-Z
5. The array of paragraph 1, wherein the linker is a disulfide linker that has the following structure: 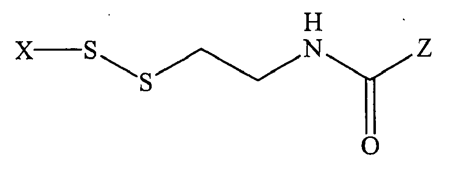
6. The array of paragraph 1, wherein the solid surface is a glass surface or a plastic surface.
7. The array of paragraph 1, wherein the solid surface is a glass slide or a microtiter plate.
8. The array of paragraph 1 wherein the linker is cleaved by reduction of a bond.
9. The array of paragraph 1, wherein the linker is cleaved by light.
10. The array of paragraph 1, wherein the molecules are glycans, nucleic acids or proteins.
11. The array of paragraph 1, wherein the molecules are mannose-containing glycans.
12. The array of paragraph 11, wherein the mannose-containing glycans comprise any one of the following glycans, or a combination thereof: 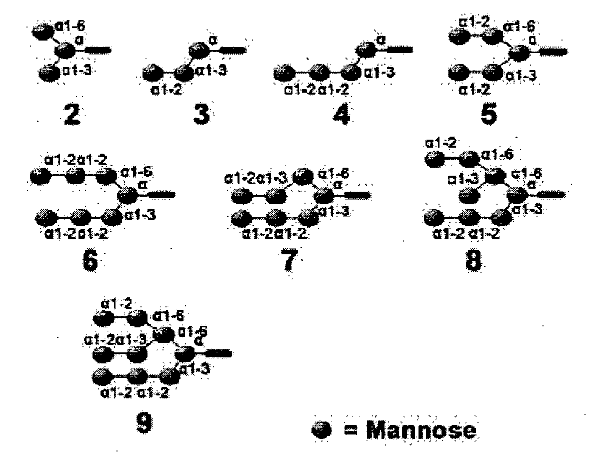
13. The array of paragraph 1, wherein the molecules comprise at least one glycan of the following formula or a combination thereof: 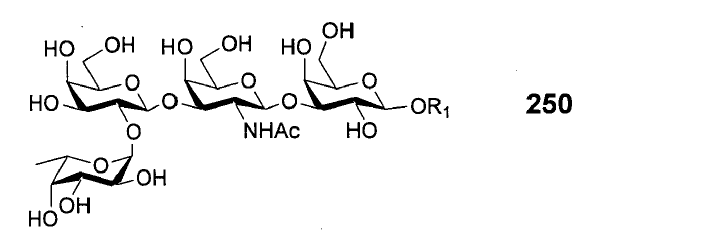 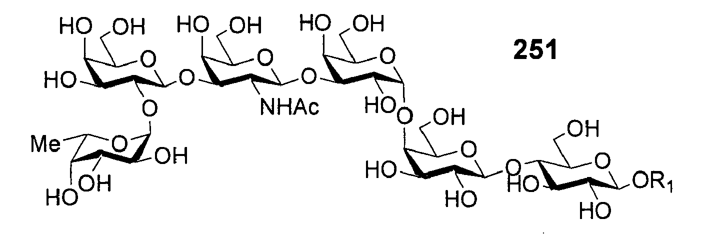 wherein R₁ comprises a linker attached to a solid support.
14. The array of paragraph 1, wherein the array comprises a solid support and a multitude of defined glycan probe locations on the solid support, each glycan probe location defining a region of the solid support that has multiple copies of one type of similar glycan molecules attached thereto.
15. The array of paragraph 14, wherein the multitude of defined glycan probe locations are about 5 to about 200 glycan probe locations.
16. A method of testing whether a molecule in a test sample can bind to the array of molecules of any one of paragraphs 1-15 comprising (a) contacting the array with the test sample; and (b) observing whether a molecule in the test sample binds to a molecule attached to the array.
17. A method of determining which molecular structures bind to a biomolecule in a test sample comprising contacting an array of molecules of any one of paragraphs 1-15 with a test sample, washing the array and cleaving the cleavable linker to permit structural or functional analysis of molecular structures of the molecules attached to an array.
18. The method of paragraph 17, wherein the biomolecule is an antibody, a receptor or a protein complex.
19. A method of detecting breast cancer in a test sample comprising (a) contacting a test sample with glycans comprising glycans **250** or **251**, or a combination thereof: 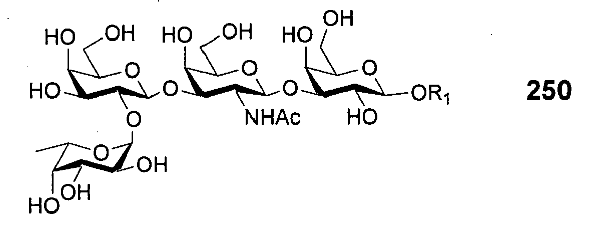 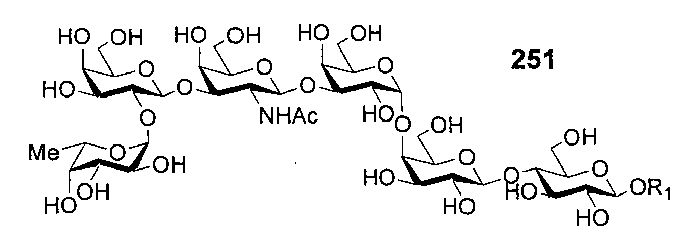 wherein R₁ is hydrogen, a glycan, a linker or a linker attached to a solid support; and
   (b) determining whether antibodies in the test sample bind to molecules comprising **250** or **251**.
20. A method of detecting HIV infection in a subject comprising (a) contacting a test sample from the subject with an array of mannose-containing glycans; and (b) determining whether antibodies in the test sample bind to a glycan comprising Manα1-2Man on a first (α1-3) branch of the glycan or a glycan comprising Manα1-2Man on a third (α1-6) branch of a glycan, or a combination thereof.
21. The method of paragraph 20, wherein the glycans are attached to the array by a cleavable linker.
22. The method of paragraph 21, wherein the mannose-containing glycans include at least one of the following glycans, or a combination thereof: 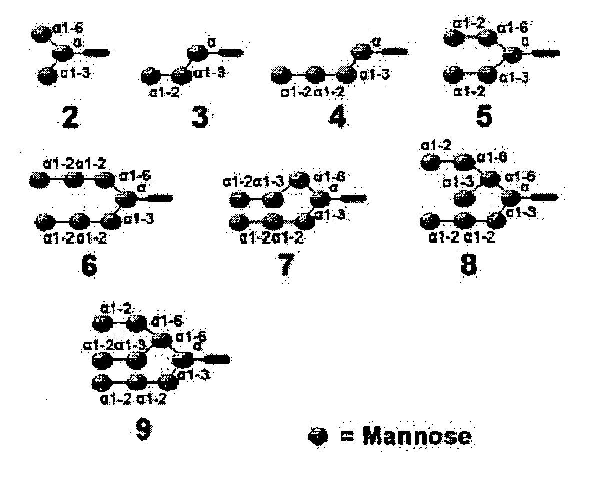
23. An isolated glycan comprising any one of the following glycans, or a combination thereof: 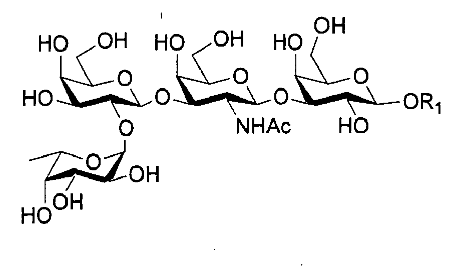 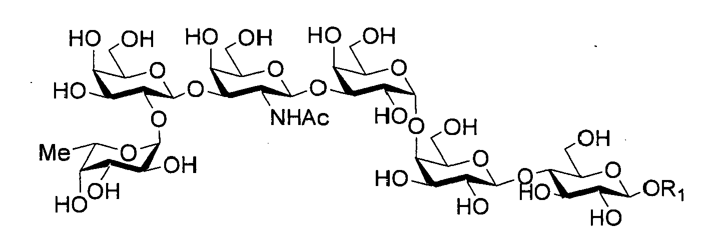 wherein: R₁ is hydrogen, a glycan or a linker that can be attached to a solid support.
24. An isolated glycan comprising Manα1-2Man on a first (α1-3) arm of a glycan or Manα1-2Man on a (α1-6) third arm of a glycan, or a combination thereof.
25. The isolated glycan of paragraph 24, wherein the glycan does not have a second (α1-3) arm.
26. An isolated glycan comprising any one of the following oligomannose glycans, or a combination thereof: 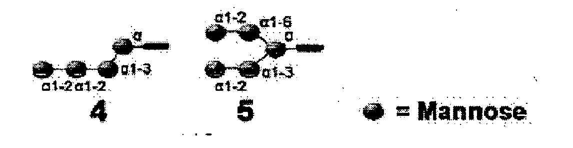
27. A pharmaceutical composition comprising a pharmaceutically acceptable carrier and an effective amount of a glycan comprising any one of the following oligomannose glycans, or a combination thereof: 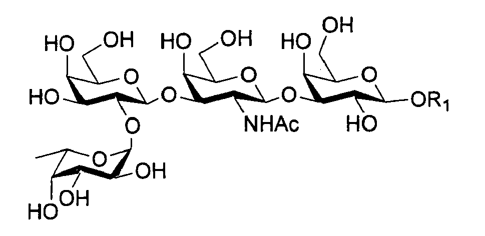 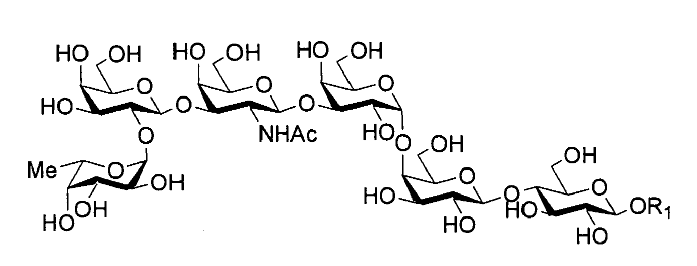 wherein: R₁ is hydrogen, a glycan or a linker.
28. A pharmaceutical composition comprising a pharmaceutically acceptable carrier and an effective amount of a glycan comprising Manα1-2Man on a first (α1-3) arm of a glycan or Manα1-2Man on a (α1-6) third arm of a glycan, or a combination thereof.
29. A pharmaceutical composition comprising a pharmaceutically acceptable carrier and an effective amount of a glycan comprising any one of the following oligomannose glycans, or a combination thereof: 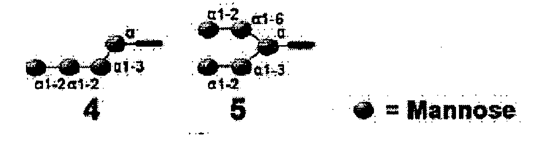
30. A pharmaceutical composition comprising a pharmaceutically acceptable carrier and an effective amount of a glycan comprising any one of the following oligomannose glycans, or a combination thereof: 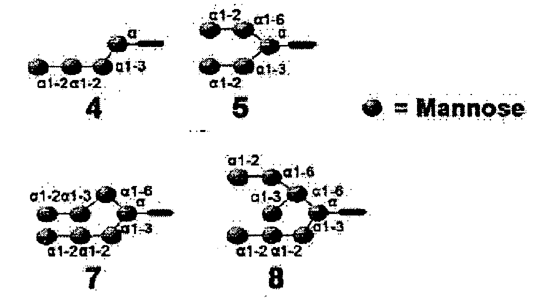
31. The pharmaceutical composition of any one of paragraphs 28-30,
   wherein the glycan is linked to an HIV gp120 peptide.
32. A method of treating or preventing breast cancer in a subject comprising administering a pharmaceutical composition comprising a pharmaceutically acceptable carrier and an effective amount of a glycan comprising any one of the following oligomannose glycans, or a combination thereof: 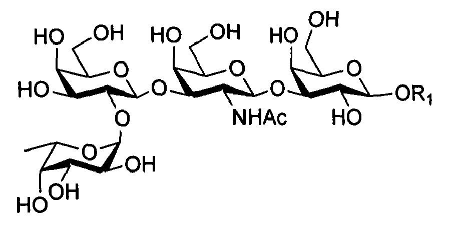 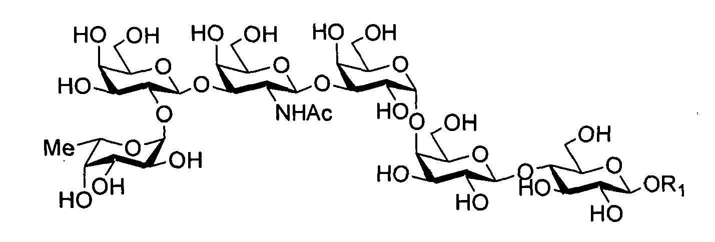 wherein: R₁ is hydrogen, a glycan or a linker.
33. A method for treating or preventing HIV infection in a subject comprising administering to the subject a pharmaceutical composition comprising a pharmaceutically acceptable carrier and an effective amount of a glycan comprising Manα1-2Man on a first (α1-3) arm of a glycan, or Manα1-2Man on a (α1-6) third arm of a glycan, or a combination thereof.
34. A method for treating or preventing HIV infection in a subject comprising administering to the subject a pharmaceutical composition comprising a pharmaceutical composition comprising a pharmaceutically acceptable carrier and an effective amount of a glycan comprising any one of the following oligomannose glycans, or a combination thereof: 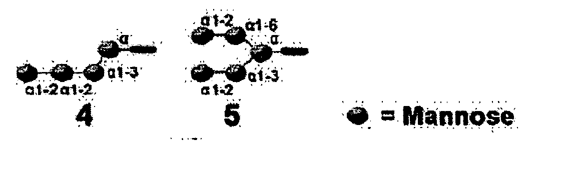
35. The method of paragraph 33, wherein the composition further comprises a glycan comprising any one of the following glycans: 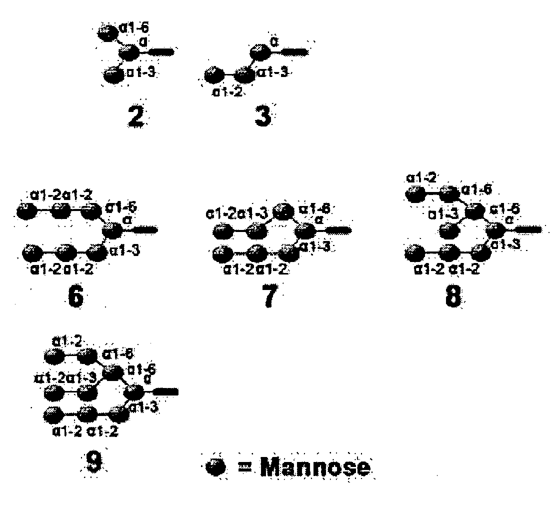
36. The method of any one of paragraphs 33-35, wherein the glycan is linked to an HIV gp120 peptide.

All patents and publications referenced or mentioned herein are indicative of the levels of skill of those skilled in the art to which the invention pertains, and each such referenced patent or publication is hereby incorporated by reference to the same extent as if it had been incorporated by reference in its entirety individually or set forth herein in its entirety. Applicants reserve the right to physically incorporate into this specification any and all materials and information from any such cited patents or publications.

The specific methods and compositions described herein are representative of preferred embodiments and are exemplary and not intended as limitations on the scope of the invention. Other objects, aspects, and embodiments will occur to those skilled in the art upon consideration of this specification, and are encompassed within the spirit of the invention as defined by the scope of the claims. It will be readily apparent to one skilled in the art that varying substitutions and modifications may be made to the invention disclosed herein without departing from the scope and spirit of the invention. The invention illustratively described herein suitably may be practiced in the absence of any element or elements, or limitation or limitations, which is not specifically disclosed herein as essential. The methods and processes illustratively described herein suitably may be practiced in differing orders of steps, and that they are not necessarily restricted to the orders of steps indicated herein or in the claims. As used herein and in the appended claims, the singular forms "a," "an," and "the" include plural reference unless the context clearly dictates otherwise. Thus, for example, a reference to "an antibody" includes a plurality (for example, a solution of antibodies or a series of antibody preparations) of such antibodies, and so forth. Under no circumstances may the patent be interpreted to be limited to the specific examples or embodiments or methods specifically disclosed herein. Under no circumstances may the patent be interpreted to be limited by any statement made by any Examiner or any other official or employee of the Patent and Trademark Office unless such statement is specifically and without qualification or reservation expressly adopted in a responsive writing by Applicants.

The terms and expressions that have been employed are used as terms of description and not of limitation, and there is no intent in the use of such terms and expressions to exclude any equivalent of the features shown and described or portions thereof, but it is recognized that various modifications are possible within the scope of the invention as claimed. Thus, it will be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

## Claims

1. An array of molecules comprising a library of molecules attached to an array through a cleavable linker, wherein the cleavable linker has the following structure:
X-Cv-Z
wherein:
Cv is a cleavage site;
X is a solid surface, a spacer group attached to the solid surface or a spacer group with a reactive group for attachment of the linker to a solid surface; and
Z is a reactive moiety for attachment of a molecule, a spacer group with a reactive moiety for attachment of a molecule, a spacer group with a molecule, or a molecule attached to the linker via a linking moiety.

2. The array of claim 1, wherein the cleavable linker is chemically cleavable, photocleavable, or enzymatically cleavable.

3. The array of claim 1, wherein the linker is a photocleavable linker comprising either formula **IVa** or **IVb**:

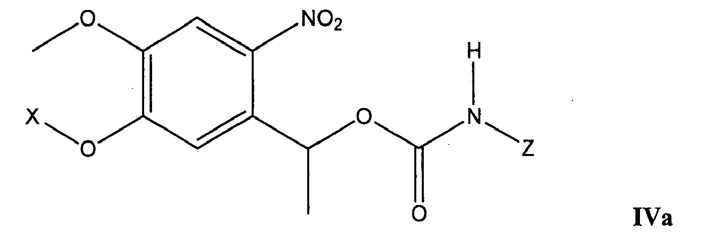

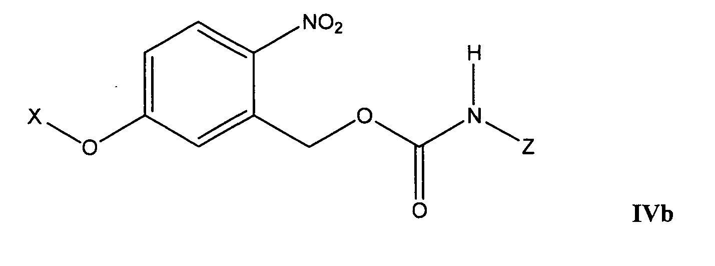

4. The array of claim 1, wherein the linker is a disulfide linker that has the following structure:
X-S-S-Z

5. The array of claim 1, wherein the linker is a disulfide linker that has the following structure:

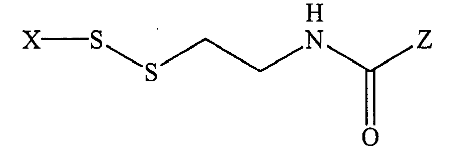

6. The array of claim 1, wherein the solid surface is a glass surface or a plastic surface.

7. The array of claim 1, wherein the solid surface is a glass slide or a microtiter plate.

8. The array of claim 1 wherein the linker is cleaved by reduction of a bond.

9. The array of claim 1, wherein the linker is cleaved by light.

10. The array of claim 1, wherein the molecules are glycans, nucleic acids or proteins.

11. The array of claim 1, wherein the molecules are mannose-containing glycans.

12. The array of claim 11, wherein the mannose-containing glycans comprise any one of the following glycans, or a combination thereof:

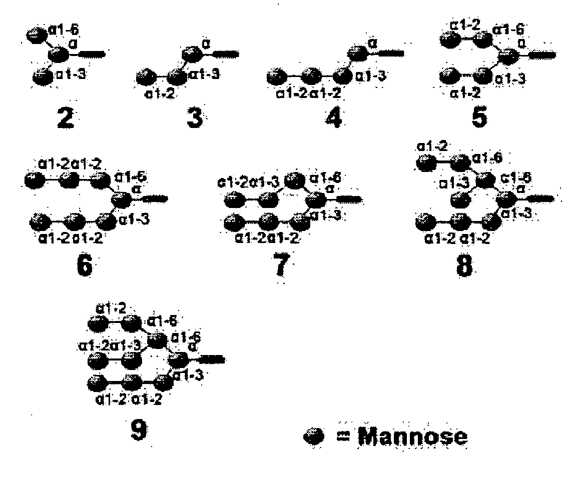

13. The array of claim 1, wherein the molecules comprise at least one glycan of the following formula or a combination thereof: 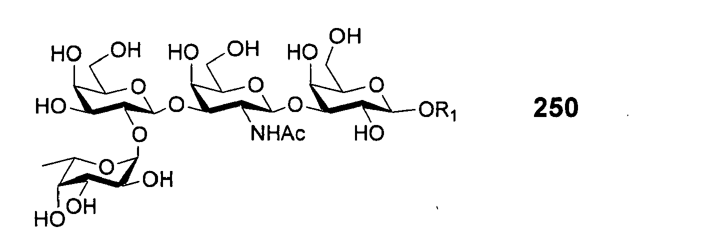 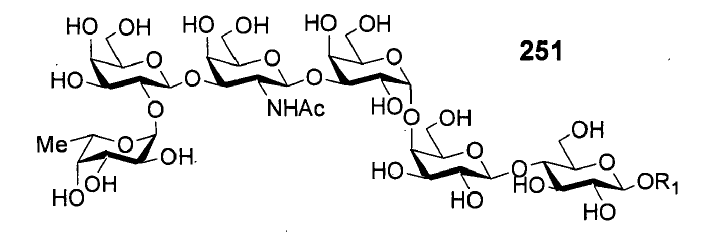 wherein R₁ comprises a linker attached to a solid support.

14. The array of claim 1, wherein the array comprises a solid support and a multitude of defined glycan probe locations on the solid support, each glycan probe location defining a region of the solid support that has multiple copies of one type of similar glycan molecules attached thereto.

15. The array of claim 14, wherein the multitude of defined glycan probe locations are about 5 to about 200 glycan probe locations.

16. A method of testing whether a molecule in a test sample can bind to the array of molecules of any one of claims 1-15 comprising (a) contacting the array with the test sample; and (b) observing whether a molecule in the test sample binds to a molecule attached to the array.

17. A method of determining which molecular structures bind to a biomolecule in a test sample comprising contacting an array of molecules of any one of claims 1-15 with a test sample, washing the array and cleaving the cleavable linker to permit structural or functional analysis of molecular structures of the molecules attached to an array.

18. The method of claim 17, wherein the biomolecule is an antibody, a receptor or a protein complex.

19. A method of detecting breast cancer in a test sample comprising (a) contacting a test sample with glycans comprising glycans **250** or **251**, or a combination thereof: 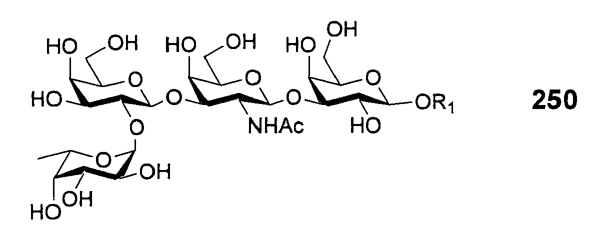 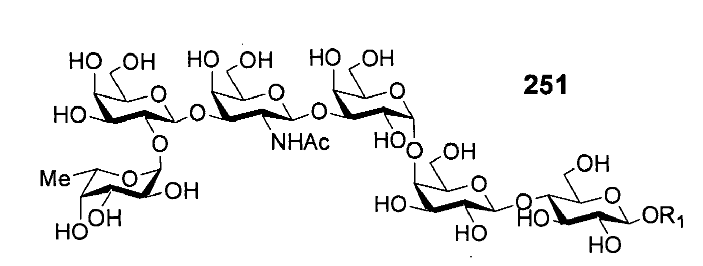 wherein R₁ is hydrogen, a glycan, a linker or a linker attached to a solid support; and
(b) determining whether antibodies in the test sample bind to molecules comprising **250** or **251**.

20. An isolated glycan comprising any one of the following glycans, or a combination thereof: 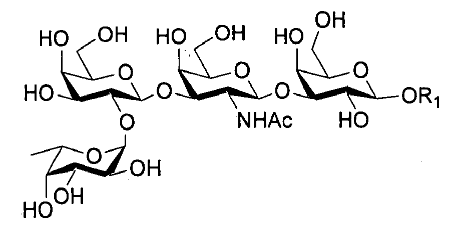 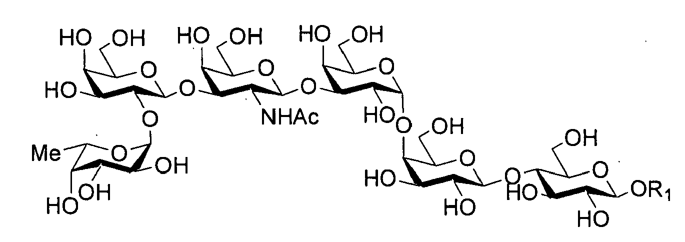 wherein: R₁ is hydrogen, a glycan or a linker that can be attached to a solid support.

21. An isolated glycan comprising Manα1-2Man on a first (α1-3) arm of a glycan or Manα1-2Man on a (α1-6) third arm of a glycan, or a combination thereof.

22. The isolated glycan of claim 21, wherein the glycan does not have a second (α1-3) arm.

23. An isolated glycan comprising any one of the following oligomannose glycans, or a combination thereof:

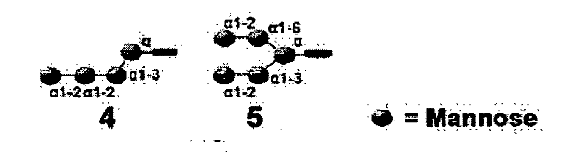

24. A pharmaceutical composition comprising a pharmaceutically acceptable carrier and an effective amount of a glycan comprising any one of the following oligomannose glycans, or a combination thereof: 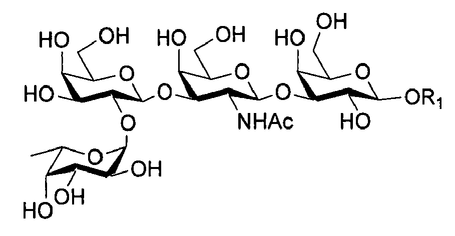 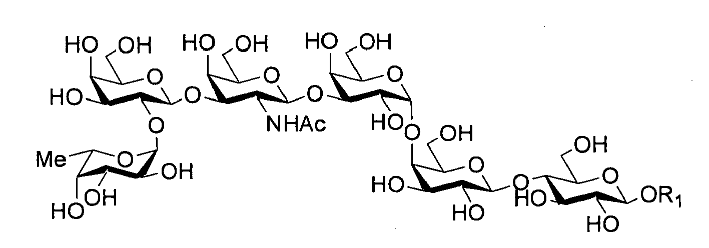 wherein: R₁ is hydrogen, a glycan or a linker.

25. A pharmaceutical composition comprising a pharmaceutically acceptable carrier and an effective amount of a glycan comprising Manα1-2Man on a first (α1-3) arm of a glycan or Manα1-2Man on a (α1-6) third arm of a glycan, or a combination thereof.

26. A pharmaceutical composition comprising a pharmaceutically acceptable carrier and an effective amount of a glycan comprising any one of the following oligomannose glycans, or a combination thereof:

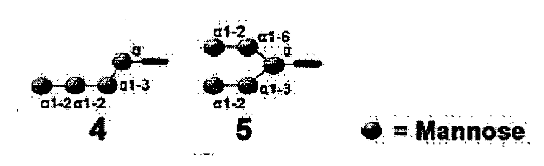

27. A pharmaceutical composition comprising a pharmaceutically acceptable carrier and an effective amount of a glycan comprising any one of the following oligomannose glycans, or a combination thereof:

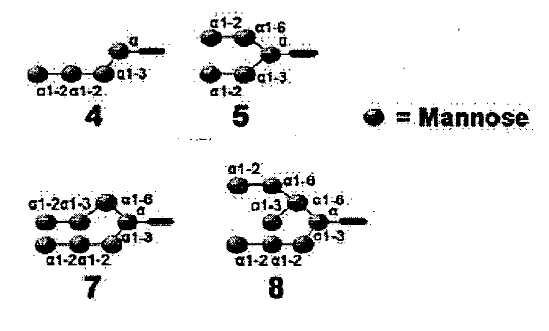

28. A method of treating or preventing breast cancer in a subject comprising administering a pharmaceutical composition comprising a pharmaceutically acceptable carrier and an effective amount of a glycan comprising any one of the following oligomannose glycans, or a combination thereof: 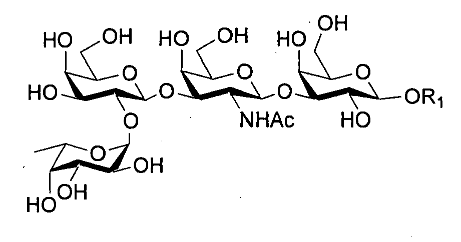 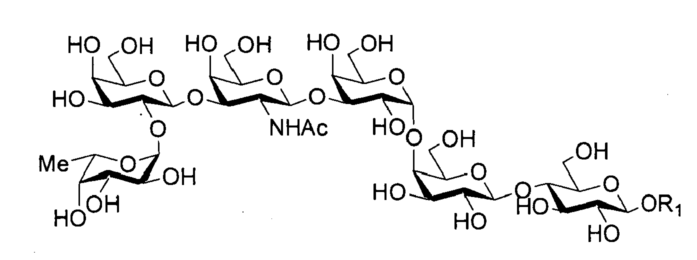 wherein: R₁ is hydrogen, a glycan or a linker.
